# EUROPEAN PATENT APPLICATION

(11) **EP 2 716 685 A1**
(43) Date of publication of application: **09.04.2014**
(21) Application number: 12791948.8
(22) Date of filing: 22.05.2012
(51) Int. Cl.: C08G 77/48, A61K 8/892, A61K 47/34, A61Q 1/00, A61Q 1/12, A61Q 5/00, A61Q 15/00, A61Q 17/04, A61Q 19/00, C08K 5/00, C08L 83/14

(54) **NOVEL LIQUID ORGANO POLYSILOXANE AND USE THEREFOR**

(30) Priority: 30.05.2011 JP 2011121095
(71) Applicant: Dow Corning Toray Co., Ltd., Tokyo 100-0004 (JP)
(72) Inventor: TAMURA Seiki, Ichihara-shi Chiba 299-0108 (JP); SOUDA Tatsuo, Ichihara-shi Chiba 299-0108 (JP); IIMURA Tomohiro, Ichihara-shi Chiba 299-0108 (JP); SAWAYAMA Sayuri, Ichihara-shi Chiba 299-0108 (JP); HORI Seiji, Ichihara-shi Chiba 299-0108 (JP); FURUKAWA Haruhiko, Ichihara-shi Chiba 299-0108 (JP)
(74) Representative: Ede, Eric
(86) International application number: PCT/JP2012/063086
(87) International publication number: WO 2012/165233

(57) **Abstract**

The present invention relates to a liquid organo polysiloxane which: exhibits fluidity at at least 100°C; has a sugar alcohol modified group bonded to a silicon atom; and has a cross-linked structure comprising carbon-silicon bonds in the cross-linked portions. This liquid organo polysiloxane exhibits superior emulsification properties, and imparts a good sensation. In particular, external preparations or cosmetics which contain oil and water and with which the liquid organo polysiloxane has been blended are pleasant to use due to the likes of: the utilization of the effects of water to inhibit an oily feel; a smooth, thick texture when applied; a natural cutaneous sensation from blending well with the skin; a good moisturizing effect and a lack of stickiness.

## Description

### TECHNICAL FIELD

This application claims priority rights based on Japanese Patent Application No. 2011-121095 filed May 30, 2011 in Japan, the content of which is incorporated herein by reference. The present invention relates to a novel liquid organopolysiloxane having a crosslinked structure and uses thereof.

### BACKGROUND ART

As a liquid organopolysiloxane having a hydrophilic group and a crosslinking portion, a product obtained by reacting a particular alkylene oxide derivative, a particular silicone derivative, and a particular isocyanate compound has been reported as being an excellent anti-foaming agent for industrial use (Patent Document 1). However, this material uses isocyanate, which is very hazardous, as a raw material, so there are obstacles in using such a material in cosmetics from the perspectives of safety, refinement cost, and the like.

In addition, as a liquid organopolysiloxane having a hydrophilic group and a crosslinking portion, an organopolysiloxane-polyoxyalkylene in which at least two organopolysiloxane-polyoxyalkylene molecules are crosslinked with a crosslinking compound free of internal hydrolysable bonds has been disclosed and has been reported to be useful as an emulsifier (Patent Document 2). A substantial amount of applied research is being conducted with regard to this material, and there have been reports of technology related to a polar liquid emulsion in a nonpolar liquid (Patent Document 3), technology related to an oil-based vaseline-containing emulsion free of oily stickiness (Patent Document 4), technology related to a hair conditioning composition (Patent Document 5), and technology related to a defoaming composition or a silicone anti-foaming agent composition (Patent Documents 6 and 7).

However, although the material proposed in Patent Document 2 has excellent characteristics for emulsifying/dispersing water in non-silicone oils such as a hydrocarbon oil or an ester oil, the material cannot stably emulsify/disperse water in a system containing a large amount of silicone oil. Further, there is the drawback that when the resulting emulsion is stored for a long period of time, the emulsion develops an abnormal odor.

On the other hand, a polysiloxane-polyether base copolymer containing a T unit and comprising randomly bonded organopolysiloxane units and straight-chain polyether units in which the organopolysiloxane and polyether units are bonded via Si-O-C or Si-C bonds has been disclosed, and it has been reported that such a copolymer has improved characteristics as a lacquer coating additive (Patent Document 8). However, this material is produced in accordance with a method in which a straight-chain alkenyl-containing polyether having a terminal OH group is added via hydrosilylation to an organopolysiloxane having a Si-H group, and the remaining Si-H groups and the terminal OH groups of the polyether portion bonded to the siloxane are then condensed by dehydrogenation in the presence of a strong base. Therefore, the severing of the organopolysiloxane part caused by the strong base is likely to occur, which makes it difficult to achieve constant quality and performance. In addition, since the crosslinked portions contain Si-O-C bonds, there is a problem in that hydrolysis occurs when compounded in a formulation containing water, which leads to a gradual decline in effectiveness.

As hydrolysis-resistant liquid organopolysiloxanes having a hydrophilic group and a crosslinking portion, a polyether-polysiloxane copolymer that has alkylene groups having from 2 to 10 carbon atoms at both terminals and that is crosslinked by a polyether in which the free valence of the group is further bonded to one silicon atom of the copolymer (Patent Document 9); and a branched-chain polyether-polysiloxane copolymer having a constituent expressed by the general formula Y[-CₙH₂ₙ-(R₂SiO)ₘ-Ap-R₂Si-G]ₓ (Patent Document 10) are also known. In addition, a polysiloxane copolymer has been disclosed which can be produced by reacting an organopolysiloxane having at least one Si-H group per molecule with an essentially linear compound that can be added by hydrosilylation and then further reacting with an organic compound having at least two isocyanate groups per molecule (Patent Document 11). Further, a surfactant comprising a crosslinked body of an active hydrogen-containing modified silicone and a crosslinking agent, wherein the viscosity (25°C) of the crosslinked body is from 500 to 100,000,000 mPa·s, has also been reported (Patent Document 12). However, the applicable fields of these materials are primarily limited to anti-foaming agents, and the application to cosmetics has not been reported.

In addition, as technologies related to a liquid organopolysiloxane having a hydrophilic group and a crosslinking portion, organohydrogen silicon compounds comprising at least one silicon-bonded hydrogen atom and at least one cyclosiloxane ring per molecule and applications as a curable composition for paper coating have been reported (Patent Documents 13 to 16). However, in these documents, there is no investigation of the liquid types in which the hydrophilic group is modified, and there is no mention of applications to cosmetics.

As described above, materials used in the field of cosmetics as liquid organopolysiloxanes having a hydrophilic group and a crosslinking portion are limited to only the organopolysiloxane-polyoxyalkylene described in Patent Document 2, and there is a need to solve the various problems described above.

Now, a great deal of research into reducing the odor of polyether-modified polysiloxanes (polyoxyalkylene group-containing organopolysiloxanes) that do not have crosslinking portions has been conducted in the past. The cause of odorization over time of a polyether-modified polysiloxane that was first reported was the aldehyde and acid produced as a result of oxidation degradation (rancidity) over time of the polyether moiety in the polyether-modified polysiloxane composition. Examples of technologies to suppress this oxidation degradation include the methods recited in Patent Documents 17 and 18 in which tocopherol, phytic acid, or a similar antioxidant component is added to the polyether-modified polysiloxane composition.

However, the use of only an anti-oxidizing agent results in the insufficient suppression of the odorization over time of a formulation based on the polyether-modified polysiloxane and, as a result, other causes were investigated. As a result, Patent Document 19 recites that propionaldehyde originating from unreacted propenyl-etherified polyoxyalkylene is a cause of the odor.

The polyether-modified polysiloxane composition is typically synthesized via a hydrosilylation reaction of an organohydrogenpolysiloxane having a silicon-bonded hydrogen group and a polyoxyalkylene having an allyl ether group at a terminal. Patent Document 19 recites that, in the production of the polyether-modified polysiloxane composition, a double bond of the allyl etherified polyoxyalkylene migrates inward due to the influence of a platinum catalyst and a portion of the allyl-etherified polyoxyalkylene becomes a propenyl-etherified polyoxyalkylene and remains in the polyether-modified polysiloxane composition as is without reacting with the organohydrogenpolysiloxane. Patent Document 19 also recites that the propenyl-etherified polyoxyalkylene degrades over time, thus producing ketones and aldehydes which results in the odorization. Moreover, hydrolysis in the presence of an acid is disclosed as a useful deodorization method.

However, while this deodorization method could be thought to be useful if all of the allyl groups of the polyoxyalkylene remaining in the composition were replaced with propenyl groups, in actuality, a significant proportion of the allyl-etherified polyoxyalkylene which is not easily hydrolyzed remains. As a result, the composition cannot be sufficiently deodorized using the deodorization method of Patent Document 19. On the other hand, if a strong acid is used that can hydrolyze the allyl-etherified polyoxyalkylene, the carbon-oxygen bond at the polyoxyalkylene site and/or the silicon-oxygen bond at the polysiloxane site may disconnect, so using such an acid is inappropriate. Additionally, in order to perform the hydrolysis reaction in a quantitative manner, excessive amounts of water and acid are needed. These excessive amounts of water and acid complicate post treatment processes and, therefore, this deodorization method is not preferable.

In order to resolve this problem, methods for suppressing the production of propionaldehyde have been disclosed (Patent Documents 20 to 23). In these methods, a hydrogenation treatment is performed as a deodorization method of the polyether-modified polysiloxane composition in order to alkylate the alkenyl groups (double bonds) included in the alkenyl group-containing polyoxyalkylene (including both propenyl-etherified polyoxyalkylene and allyl-etherified polyoxyalkylene) remaining in the composition. However, even with a polyether-modified polysiloxane composition deodorized using a hydrogenation reaction, in cases where a formulation including water and an alcohol is compounded, it may be difficult to achieve sufficient deodorization over time or under elevated temperature conditions.

A cause of the odorization is acetal and similar aldehyde condensation products that are free of unsaturated bonds that remain in the composition. Thus, for the purpose of completely eliminating the acetal and other aldehyde condensation products, technology in which treatment using the acid aqueous solution and hydrogenation treatment are combined (Patent Document 24); and technologies in which hydrogenation treatment and treatment using a solid acid catalyst are combined (Patent Documents 25 and 26) are disclosed. That is, it is acknowledged that performing at least hydrogenation treatment is preferable in the deodorization of polyether-modified polysiloxanes as a raw material suitable for use in cosmetic products.

On the other hand, sugar alcohol-modified polysiloxanes that do not have crosslinked portions and applications thereof to cosmetics and the like have been reported in Patent Documents 27 to 34. However, there has been no disclosure of a liquid organopolysiloxane having a sugar alcohol derivative group and a crosslinked portion, wherein the crosslinked portion links an organopolysiloxane portion and an organic portion with a carbon-silicon bond.

### BACKGROUND DOCUMENTS

### Patent Documents

Patent Document 1: Japanese Unexamined Patent Application Publication No. S63-248410A
Patent Document 2: US Patent No. 4853474B
Patent Document 3: US Patent No. 5136068B
Patent Document 4: US Patent No. 5387417B
Patent Document 5: European Patent No. 0381318 Specification
Patent Document 6: Japanese Unexamined Patent Application Publication No. H08-000908A
Patent Document 7: Japanese Unexamined Patent Application Publication No. H07-185212A
Patent Document 8: Japanese Unexamined Patent Application Publication No. H07-292119A
Patent Document 9: Japanese Unexamined Patent Application Publication No. 2001-115390A
Patent Document 10: Japanese Unexamined Patent Application Publication No. 2004-174495A
Patent Document 11: Japanese Unexamined Patent Application Publication (Translation of PCT Application) No. 2008-542010A
Patent Document 12: Japanese Unexamined Patent Application Publication No. 2009-262080A
Patent Document 13: Japanese Unexamined Patent Application Publication (Translation of PCT Application) No. 2005-523980A
Patent Document 14: Japanese Unexamined Patent Application Publication (Translation of PCT Application) No. 2005-529989A
Patent Document 15: Japanese Unexamined Patent Application Publication (Translation of PCT Application) No. 2006-511645A
Patent Document 16: Japanese Unexamined Patent Application Publication (Translation of PCT Application) No. 2006-511646A
Patent Document 17: Japanese Examined Patent Application Publication No. S55-041210A
Patent Document 18: Japanese Unexamined Patent Application Publication No. S60-018525A
Patent Document 19: Japanese Unexamined Patent Application Publication No. H02-302438A
Patent Document 20: US Patent No. 5225509B
Patent Document 21: Japanese Unexamined Patent Application Publication No. H07-330907A
Patent Document 22: Japanese Unexamined Patent Application Publication No. H09-165315A
Patent Document 23: Japanese Unexamined Patent Application Publication No. H09-165318A
Patent Document 24: WO2002/055588
Patent Document 25: WO2004/046226
Patent Document 26: Japanese Unexamined Patent Application Publication No. 2005-120293
Patent Document 27: Japanese Unexamined Patent Application Publication No. S62-068820A
Patent Document 28: Japanese Unexamined Patent Application Publication No. S63-139106A
Patent Document 29: Japanese Unexamined Patent Application Publication No. H05-186596A
Patent Document 30: Japanese Unexamined Patent Application Publication No. H07-041417A
Patent Document 31: Japanese Unexamined Patent Application Publication No. 2002-119840A
Patent Document 32: Japanese Unexamined Patent Application Publication No. 2008-274241 A
Patent Document 33: Japanese Unexamined Patent Application Publication No. 2002-179798A
Patent Document 34: Japanese Unexamined Patent Application Publication No. 2003-146991A

### SUMMARY OF INVENTION

### Technical Problem

The present invention was conceived in order to solve the problems described above, and a first object of the present invention is to provide a novel liquid organomodified organopolysiloxane and a production method thereof, wherein the novel liquid organopolysiloxane has affinity with various oil agents, has excellent emulsion characteristics, imparts excellent tactile sensation, and, when compounded in an external use preparation or a cosmetic composition containing water and oil, takes advantage of the effects of water to suppress oiliness and provide a smooth, thick feeling when applied, a natural feeling to the skin with good compatibility with the skin, excellent moisturizing effects, and an excellent sensation during use without any oily stickiness.

A second object of the present invention is to provide raw material for a preparation for external use or a cosmetic such as a tactile sensation improver, a film-forming agent, a binder, a viscosity adjuster, a surfactant, an emulsifier, or a powder dispersing agent and a cosmetic or preparation for external use containing the liquid organomodified organopolysiloxane.

A third object of the present invention is to provide a liquid organomodified organopolysiloxane with a reduced odor, a raw material for an external use preparation or a cosmetic composition containing the same, and an external use preparation or a cosmetic composition containing the raw material.

### Solution To Problem

The present inventors arrived at the present invention as a result of conducting dedicated research in order to achieve the objective described above. Specifically, the first object of the present invention is achieved by a liquid organopolysiloxane having fluidity at a temperature of at least 100°C, having a silicon-bonded sugar alcohol-modified group, and having a crosslinked structure comprising a carbon-silicon bond in the crosslinking portion.

The sugar alcohol-modified group is preferably expressed by the following general formula (4-1): 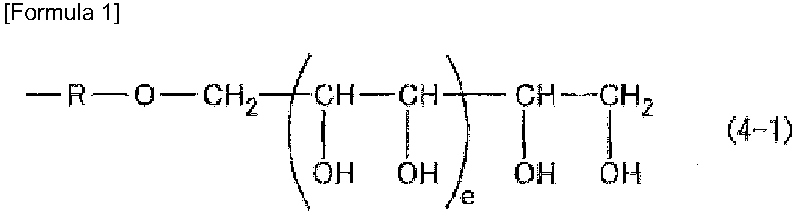 (wherein
R is a divalent organic group, and
e is 1 or 2) or the following general formula (4-2): 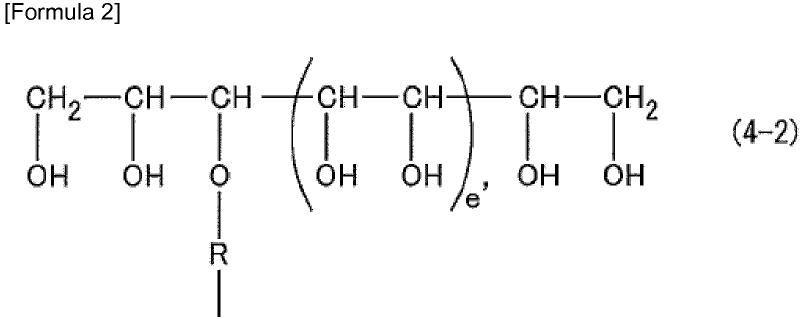
(wherein
R is as defined above, and
e' is 0 or 1).

In the general formula (4-1) or (4-2) described above, the divalent organic group represented by R is preferably a substituted or unsubstituted, straight-chain or branched divalent hydrocarbon group having from 3 to 5 carbon atoms.

The liquid organopolysiloxane described above can be obtained by reacting:
(A) an organohydrogenpolysiloxane;
(B) a sugar alcohol group-containing organic compound having an unsaturated bond; and
(C) at least one type of organic compound selected from the group consisting of (C1) an organic compound having an average number of reactive unsaturated bonds in the molecule that is greater than 1 and (C2) an organic compound having at least one unsaturated bond and at least one epoxy group in the molecule.

The average value of the number of silicon-bonded hydrogen atoms per molecule of the component (A), which reacts with unsaturated bonds or epoxy groups of the component (C) constituting the crosslinking portion, is preferably greater than 1 and less than 2.

The component (A) is preferably expressed by the average composition formula (1):

[Formula 3] R¹ₐH_{b}SiO_{(4-a-b)/2} (1)

(wherein R¹ moieties are each independently monovalent organic groups, 1.0≤a≤3.0, and 0.001≤b≤1.5).

The component (C) is preferably at least one organic compound selected from the following formulas (C1-1) to (C1-5) and (C2-1) to (C2-2):
(C1-1) an α,ω-diene expressed by the general formula (2-1):

   [Formula 4] CH₂=CH(CH₂)ₓCH=CH₂ (2-1)

   (wherein 1≤x≤20);
(C1-2) an α,ω-diyne expressed by the general formula (2-2):

   [Formula 5] CH=C(CH₂)ₓC≡CH (2-2)

   (wherein 1≤x≤20);
(C1-3) an α,ω-ene-yne expressed by the general formula (2-3):

   [Formula 6] CH₂=CH(CH₂)ₓC=CH (2-3)

   (wherein 1≤x≤20);
(C1-4) a bisalkenyl polyether compound expressed by the general formula (2-4):

   [Formula 7] CₘH₂ₘ₋₁O(CₙH₂ₙO)_{y}CₘH₂ₘ₋₁ (2-4)

   (wherein 2≤m≤20, 2≤n≤4, y is the total value of the number of repetitions of the oxyethylene unit, the oxypropylene unit, and the oxybutylene unit, and 1≤y≤180);
(C1-5) an unsaturated group-containing silicone-compound expressed by the average composition formula (2-5):

   [Formula 8] R²ₚR³_{q}SiO_{(4-p-q)/2} (2-5)

   (wherein R² moieties may each be independent from one another but are monovalent organic groups that are different from R³;
   R³ moieties are each independently monovalent unsaturated aliphatic hydrocarbon groups having from 2 to 30 carbon atoms, 1.0≤p≤2.5, and 0.001≤q≤1.5);
(C2-1) an unsaturated epoxy compound expressed by the general formula (2-6): 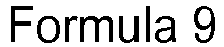 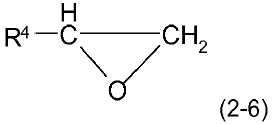 (wherein R⁴ is a substituted or unsubstituted, straight-chain or branched monovalent hydrocarbon group having one unsaturated bond and from 2 to 20 carbon atoms); and (C2-2) an unsaturated group-containing cycloaliphatic epoxy compound expressed by the general formula (2-7): 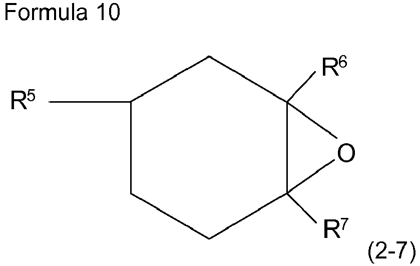 (wherein R⁵ is a substituted or unsubstituted, straight-chain or branched monovalent hydrocarbon group having one unsaturated bond and from 2 to 20 carbon atoms;
   R⁶ is a hydrogen atom or a methyl group; and
   R⁷ is a hydrogen atom or a methyl group).

In the average composition formula (1), the monovalent organic group represented by R¹ is preferably selected from the following (D1) to (D10):
(D1) a substituted or unsubstituted, straight-chain or branched monovalent hydrocarbon group having from 1 to 60 carbon atoms;
(D2) a polyoxyalkylene group expressed by -R⁸O(AO)_{z}R⁹ (wherein AO is an oxyalkylene group having from 2 to 4 carbon atoms; R⁸ is a substituted or unsubstituted, straight-chain or branched divalent hydrocarbon group having from 3 to 5 carbon atoms; R⁹ is a hydrogen atom, a substituted or unsubstituted, straight-chain or branched monovalent hydrocarbon group having from 1 to 24 carbon atoms, or a substituted or unsubstituted, straight-chain or branched acyl group having from 2 to 24 carbon atoms; and z=1 to 100);
(D3) a substituted or unsubstituted, straight-chain or branched alkoxy group having from 1 to 30 carbon atoms;
(D4) a hydroxyl group;
(D5) an ester group expressed by -R¹⁰-COOR¹¹ (wherein R¹⁰ is a substituted or unsubstituted, straight-chain or branched divalent hydrocarbon group having from 2 to 20 carbon atoms, and R¹¹ is a substituted or unsubstituted, straight-chain or branched monovalent hydrocarbon group having from 1 to 30 carbon atoms);
(D6) an ester group expressed by -R¹⁷-OCOR¹⁸ (wherein R¹⁷ substituted or unsubstituted, straight-chain or branched divalent hydrocarbon group having from 2 to 20 carbon atoms, and R¹⁸ is a substituted or unsubstituted, straight-chain or branched monovalent hydrocarbon group having from 1 to 30 carbon atoms);
(D7) L¹
   here, L¹ is a silylalkyl group having a siloxane dendron structure and, when i=1, is expressed by the following general formula (3): 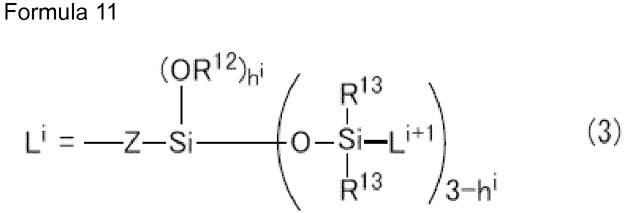 (wherein
   R¹² is a substituted or unsubstituted, straight-chain or branched monovalent hydrocarbon group having from 1 to 30 carbon atoms;
   R¹³ moieties each independently represents an alkyl group or a phenyl group having from 1 to 6 carbon atoms;
   Z is a divalent organic group;
   i represents a generation of the aforementioned silylalkyl group represented by Lⁱ and is an integer of 1 to k when k is the number of generations, which is the number of repetitions of the silylalkyl group; the number of generations k is an integer from 1 to 10; Lⁱ⁺¹ is the silylalkyl group when i is less than k, and R¹³ when i=k; and hⁱ is a number in a range from 0 to 3); (D8) an alkyl group substituted by a chain polysiloxane structure expressed by the following general formula (4): 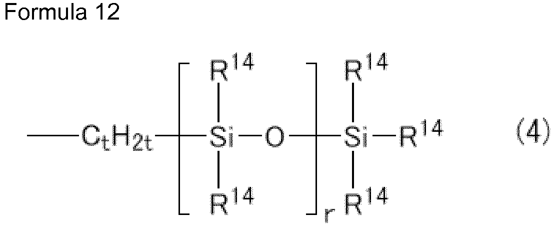
   (wherein R¹⁴ moieties are each independently substituted or unsubstituted, straight-chain or branched monovalent hydrocarbon groups having from 1 to 30 carbon atoms, hydroxyl groups, or hydrogen atoms, and at least one of the R¹⁴ moieties is the monovalent hydrocarbon group; t is a number in a range from 2 to 10; and r is a number in a range from 1 to 100);
(D9) an epoxy group expressed by the following general formula (5): 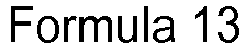 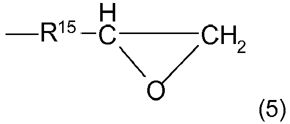 (wherein R¹⁵ is a substituted or unsubstituted, straight-chain or branched divalent hydrocarbon group having from 2 to 20 carbon atoms); and
(D10) a cycloaliphatic epoxy group expressed by the following general formula (6): 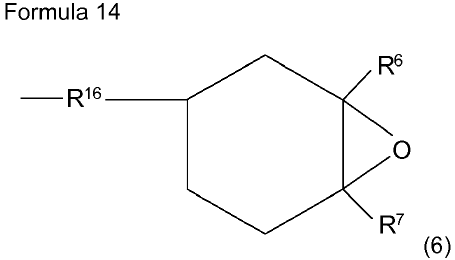 (wherein R¹⁶ is a substituted or unsubstituted, straight-chain or branched divalent hydrocarbon group having from 2 to 20 carbon atoms, and R⁶ and R⁷ are synonymous with those described above).

Further, the first object of the present invention can be achieved by a production method for a liquid organopolysiloxane comprising a process of reacting:
(A) an organohydrogenpolysiloxane;
(B) a sugar alcohol-group containing organic compound having an unsaturated bond; and
(C) at least one type of organic compound selected from the group consisting of (C1) an organic compound having an average number of unsaturated bonds in the molecule that is greater than 1 and (C2) an organic compound having at least one unsaturated bond and at least one epoxy group in the molecule.

In the production method for the liquid organopolysiloxane described above, (A) an organohydrogenpolysiloxane, (B) a sugar alcohol-group containing organic compound having an unsaturated bond, and (C) at least one type of organic compound selected from the group consisting of (C1) an organic compound having an average number of unsaturated bonds in the molecule that is greater than 1 and (C2) an organic compound having at least one unsaturated bond and at least one epoxy group in the molecule are essential components, and other components are preferably reacted with the component (A) successively in the presence of a hydrosilylation reaction catalyst.

In one aspect of the production method for the liquid organopolysiloxane described above, the component (A) and the component (B) are reacted first, and a crosslinking reaction is then performed by adding the component (C). In this case, any component (Q) shown below may be reacted with the component (A) prior to the reaction between the component (A) and the component (B), further reacted after the reaction between the component (A) and the component (B), or further reacted after crosslinking with the component (C).
(Q): a compound having one unsaturated group in the molecule (excluding the compound of (C2))

In one aspect of the production method for the liquid organopolysiloxane described above, a reaction between the component (A) and the component (C) is first performed to derive a crosslinked portion, and the component (B) is then added and reacted thereafter. In this case, any component (Q) shown below may be reacted with the component (A) prior to the reaction between the component (A) and the component (C), further reacted after the reaction between the component (A) and the component (C), or further reacted after the reaction of the component (B).
(Q): a compound having one unsaturated group in the molecule (excluding the compound of (C2)).

The second object of the present invention can be achieved by a raw material for use in a preparation for external use or a cosmetic composition containing the liquid organopolysiloxane, or an external use preparation or a cosmetic composition containing the same; a composition containing at least one type of oil agent in addition to the liquid organopolysiloxane; and a raw material for an external use preparation or a cosmetic composition containing the composition, or an external use preparation or a cosmetic composition containing the same. The composition may be in the form of an emulsion.

The raw material for use in an external use preparation or a cosmetic composition can be a tactile sensation improver, a film-forming agent, a binder, a viscosity adjuster, a surfactant, an emulsifier, or a powder dispersing agent.

The third object of the present invention can be achieved by further performing an acidizing process of treating the liquid organopolysiloxane obtained by the production method for the liquid organopolysiloxane described above with at least one type of an acidic substance; a composition containing at least one type of acidic substance in addition to the liquid organopolysiloxane of the present invention; and a raw material for an external use preparation or a cosmetic composition containing the composition, or an external use preparation or a cosmetic composition containing the same. A process of heating and/or decompressing the composition is preferably performed after the acidizing process. Further, it is preferable to neutralize the composition by adding at least one type of a basic substance after the acidizing treatment process.

### Advantageous Effects of Invention

With the present invention, it is possible to provide a novel liquid organomodified organopolysiloxane, wherein the novel liquid organopolysiloxane has affinity with various oil agents, has excellent emulsion characteristics, imparts excellent tactile sensation, and, when compounded in an external use preparation or a cosmetic composition containing water and oil, takes advantage of the effects of water to suppress oiliness and provide a smooth, thick feeling when applied, a natural feeling to the skin with good compatibility with the skin, excellent moisturizing effects, and an sensation during use without any oily stickiness.

In addition, the liquid organomodified organopolysiloxane of the present invention can demonstrate excellent emulsifying performance with respect to both nonpolar oil agents and polar oil agents in emulsions in which both water and an oil agent are present. The emulsion may also contain a polyhydroxy alcohol. Accordingly, it is possible to design an external use preparation or a cosmetic composition with various formulations by compounding the liquid organomodified organopolysiloxane of the present invention in an external use preparation or a cosmetic composition. It is unnecessary for the preparation for external use or the cosmetic composition of the present invention to include a polyether-modification substance. Further, the liquid organomodified organopolysiloxane of the present invention has excellent powder dispersion stability, which makes it possible to uniformly and finely disperse a powder and, in particular, to enhance the storage stability of a composition containing the powder.

Due to the functionality thereof, the liquid organomodified organopolysiloxane of the present invention can be advantageously used as a raw material for an external use preparation or a cosmetic composition such as a tactile sensation improver, a film-forming agent, a binder, a viscosity adjuster, a surfactant, an emulsifier, or a powder dispersing agent and can be compounded in a cosmetic composition or a preparation for external use as necessary. In particular, the present invention can provide a nonaqueous emulsion composition that is usable as a drug delivery system. Similarly, the present invention can provide a water-in-oil type or an oil-in-water type emulsion composition having excellent stability. Additionally, the liquid organomodified organopolysiloxane of the present invention can be used as a composition with an oil agent in a composition because it can be uniformly mixed with oil agents. Furthermore, a composition comprising an oil agent in conjunction with the liquid organomodified organopolysiloxane of the present invention has superior storage stability.

With the present invention, the odor of the liquid organomodified organopolysiloxane can be reduced. The liquid organomodified organopolysiloxane of the present invention having a reduced odor is particularly suitable as a raw material for an external use preparation or a cosmetic composition or as a component of an external use preparation or a cosmetic composition. In particular, in the present invention, it is possible to provide a liquid organopolysiloxane or a composition containing the same which is essentially odorless and demonstrates suppressed odor generation at high temperatures or over time with a simple acidizing process.

The odor-reducing effect of the sugar alcohol-modified silicone of the present invention is very high, and the odor-reducing effect achieved in the present invention cannot be achieved even by acidizing another modified silicone in the same manner as in the present invention. Although an odor-reducing effect such as that achieved in the present invention can be achieved by performing hydrogenation on another modified silicone, the hydrogenation process is complex and requires relatively expensive reagents and special equipment. The present invention is advantageous in industrial scale implementation because the implementation of such hydrogenation treatment is unnecessary. Moreover, the present invention can provide a sugar alcohol-modified silicone or a composition containing the same which is deodorized simply and at low cost.

In addition, the odor of the liquid organomodified organopolysiloxane of the present invention having a reduced odor does not need to be masked when compounded in an external use preparation or a cosmetic composition, and there is a high degree of flexibility in the design of the formulations of an external use preparation or a cosmetic composition. This is particularly advantageous in cosmetic compositions, in which functions that contain an odor are emphasized, and, therefore, design of a fragrance free cosmetic composition, a faintly scented cosmetic composition, or a cosmetic composition with a desired fragrance is easy.

### DETAILED DESCRIPTION OF THE INVENTION

### (Liquid organopolysiloxane and production method thereof)

A first aspect of the present invention is a liquid organopolysiloxane having fluidity at a temperature of at least 100°C, having a silicon-bonded sugar alcohol-modified group, and having a crosslinked structure comprising a carbon-silicon bond in the crosslinking portion.

The liquid organopolysiloxane of the present invention has a crosslinked structure comprising a crosslinking portion containing a carbon-silicon bond, and the crosslinked structure also contains a polysiloxane chain. Here, the crosslinking portion has a different binding site than the sugar alcohol-modified group and is preferably a portion (crosslinking point) forming the crosslinked structure between the chain, cyclic, or branched-chain molecules comprising an organopolysiloxane chain, a chain-like organic molecular chain, or a combination thereof originating from each reaction component described above. The liquid organopolysiloxane of the present invention has a structure in which the molecules of the polysiloxane chain and the like are loosely crosslinked. More specifically, since the crosslinking density is low to a degree that fluidity is exhibited at a temperature of at least 100°C, the liquid organopolysiloxane of the present invention is a liquid when heated from room temperature (25°C) to at least 100°C, and the properties and characteristics of the liquid organopolysiloxane differ from those of three-dimensionally crosslinked organopolysiloxanes or rubber-like siloxanes with a high crosslinking density in that the liquid organopolysiloxane of the present invention has miscibility and solubility with other oil agents and solvents.

The liquid organopolysiloxane of the present invention is a liquid having fluidity at a temperature of at least 100°C. In the present invention, "having fluidity at a temperature of at least 100°C" means that after the liquid surface of the organopolysiloxane in a prescribed container is made horizontal, the liquid surface can return to the horizontal state one hour after the container is inclined. Here, "horizontal" means to form a plane that intersects the direction of gravitational force at a right angle. Note that it is obvious that the organopolysiloxane of the present invention has fluidity at temperatures of 100°C and higher, but the scope of the invention of the present application also encompasses a liquid organopolysiloxane with a low crosslinking density to the degree that a liquidity is expressed by heating to at least 100°C even when the liquid organopolysiloxane is a semi-gel or soft solid that does not exhibit fluidity at temperatures of room temperature (25°C) and lower. The organopolysiloxane of the present invention has fluidity at a temperature of at least 100°C but more preferably also exhibits liquidity in a range from 100°C or less to room temperature. Specifically, the organopolysiloxane of the present invention is preferably a liquid having fluidity at 80°C, more preferably having fluidity at 40°C, and even more preferably having fluidity at room temperature (25°C).

The liquid organopolysiloxane of the present invention has a silicon-bonded sugar alcohol-modified group. The sugar alcohol-modified group constitutes a hydrophilic site of the liquid organopolysiloxane of the present invention. The structure of the sugar alcohol-modified group is not limited as long as the structure has a sugar alcohol site, but the sugar alcohol residue is preferably bonded to a silicon atom via a divalent organic group.

Accordingly, the sugar alcohol-modified group is preferably expressed by the following general formula (4-1): 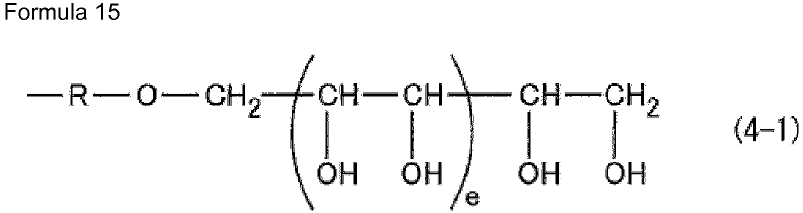 (wherein
R is a divalent organic group, and
e is 1 or 2), the following general formula (4-2): 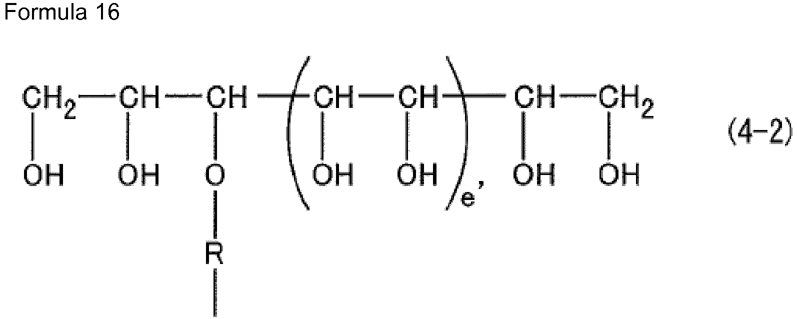
(wherein
R is as defined above, and
e' is 0 or 1).

The liquid organopolysiloxane of the present invention is characterized in that at least one type of a sugar alcohol-modified group expressed by the general formula (4-1) or (4-2) above is bonded to a silicon atom. Further, the organopolysiloxane may also have two or more types of sugar alcohol-modified groups selected from these sugar alcohol-modified groups in the molecule. Similarly, a mixture of organopolysiloxanes having different sugar alcohol-modified groups may also be used.

The divalent organic group represented by R in the general formula (4-1) or (4-2) is not particularly limited, but examples include substituted or unsubstituted, straight-chain or branched divalent hydrocarbon groups having from 1 to 30 carbon atoms. Substituted or unsubstituted, straight-chain or branched divalent hydrocarbon groups having from 3 to 5 carbon atoms are preferable. Examples of substituted or unsubstituted, straight-chain or branched divalent hydrocarbon groups having from 1 to 30 carbon atoms include straight-chain or branched-chain alkylene groups having from 1 to 30 carbon atoms such as methylene groups, dimethylene groups, trimethylene groups, tetramethylene groups, pentamethylene groups, hexamethylene groups, heptamethylene groups, and octamethylene groups; alkenylene groups having from 2 to 30 carbon atoms such as vinylene groups, allylene groups, butenylene groups, hexenylene groups, and octenylene groups; arylene groups having from 6 to 30 carbon atoms such as phenylene groups and diphenylene groups; alkylene arylene groups having from 7 to 30 carbon atoms such as dimethylene phenylene groups; and groups in which the hydrogen atoms bonded to the carbon atoms of these groups are at least partially substituted with halogen atoms such as fluorine atoms or organic groups such as carbinol groups, epoxy groups, glycidyl groups, acyl groups, carboxyl groups, amino groups, methacryl groups, mercapto groups, amide groups, and oxyalkylene groups. The divalent hydrocarbon groups are preferably alkylene groups having from 1 to 30 carbon atoms, more preferably are alkylene groups having from 1 to 6 carbon atoms, and even more preferably alkylene groups having from 3 to 5 carbon atoms.

In the sugar alcohol-modified group of the general formula (4-1), it is particularly preferable for R to be a propylene group and for e=1. Similarly, in the sugar alcohol-modified group of the general formula (4-2), it is particularly preferable for R to be a propylene group and for e'=0. The sugar alcohol-modified group in this case is a xylitol residue expressed by the structural formula: -C₃H₆-OCH₂[CH(OH)]₃CH₂OH or the structural formula: -C₃H₆-OCH[CH(OH)CH₂OH]₂ corresponding to the general formula (4-1) or (4-2) (hereinafter, simply referred to as a "xylitol residue" or a "xylitol modified group").

The bond position of the sugar alcohol-modified group may be either the side chain or the terminal of the polysiloxane, which is the main chain, and the liquid organopolysiloxane may have two or more sugar alcohol-modified groups. Further, these two or more sugar alcohol-modified groups may be the same or different sugar alcohol-modified groups. These two or more sugar alcohol-modified groups can be structured such that bonding occurs only in a side chain of polysiloxane, which is the main chain, only at a terminal, or in a side chain and at a terminal.

The liquid organopolysiloxane of the present invention can be produced by reacting:
(A) an organohydrogenpolysiloxane;
(B) a sugar alcohol-group containing organic compound having an unsaturated bond; and
(C) at least one type of organic compound selected from the group consisting of (C1) an organic compound having an average number of reactive unsaturated bonds in the molecule that is greater than 1 and (C2) an organic compound having at least one unsaturated bond and at least one epoxy group in the molecule.

The (A) organohydrogenpolysiloxane is not particularly limited as long as it has silicon-bonded hydrogen atoms, but an organohydrogenpolysiloxane having more than one - preferably from 1.01 to 100, more preferably from 1.1 to 50, even more preferably from 1.2 to 25, and particularly preferably from 1.3 to 10 - silicon-bonded hydrogen atoms in the molecule on average is preferable, and a straight-chain, branched, or reticulated organopolysiloxane may be used. The positions of the silicon-bonded hydrogen atoms in the organohydrogenpolysiloxane is not limited, and can be on the main chain or at the terminals. However, the atoms are preferably positioned at the terminals from the perspective of reducing the degree of crosslinking. One type or two or more types of organohydrogenpolysiloxanes may be used as the component (A).

Examples of the component (A) include 1,1,3,3-tetramethyldisiloxane, 1,3,5,7-tetramethylcyclotetrasiloxane, methylhydrogenpolysiloxane capped at both molecular terminals with trimethylsiloxy groups, dimethylsiloxane-methylhydrogensiloxane copolymers capped at both molecular terminals with trimethylsiloxy groups, dimethylsiloxane capped at both molecular terminals with dimethylhydrogensiloxy groups, dimethylpolysiloxane capped at both molecular terminals with dimethylhydrogensiloxy groups, dimethylsiloxane-methylhydrogensiloxane copolymers capped at both molecular terminals with dimethylhydrogensiloxy groups, methylhydrogensiloxane-diphenylsiloxane copolymers capped at both molecular terminals with trimethylsiloxy groups, methylhydrogensiloxane-diphenylsiloxane-dimethylsiloxane copolymers capped at both molecular terminals with trimethylsiloxy groups, copolymers comprising (CH₃)₂HSiO_{1/2} units and SiO_{4/2} units, and copolymers comprising (CH₃)₂HSiO_{1/2} units, SiO_{4/2} units, and (C₆H₅)SiO_{3/2} units.

The component (A) is preferably expressed by the average composition formula (1):

Formula 17 R¹ₐH_{b}SiO_{(4-a-b)/2} (1)

(wherein R¹ moieties are each independently monovalent organic groups, 1.0≤a≤3.0, and 0.001≤b≤1.5).

Although the molecular structure of the (A) organohydrogenpolysiloxane is not limited, examples include straight-chain, partially branching straight-chain, branched-chain, cyclic, and dentric structures, and straight-chain is preferable. The molecular weight is not particularly limited, and products having a low molecular weight to products having a high molecular weight can be used. Specifically, the number-average molecular weight is preferably in a range from 100 to 1,000,000 and more preferably in a range from 300 to 500,000.

Examples of such organohydrogenpolysiloxanes includes those expressed by the following structural formulas:
(i) R¹₃SiO(R¹₂SiO)ᵥ(R¹SiHO)_{w}SiR¹₃
(ii) HR¹₂SiO(R¹₂SiO)ᵥ(R¹SiHO)_{z}SiR¹₃
(iii) HR¹₂SiO(R¹₂SiO)ᵥ(R¹SiHO)_{z}SiR¹₂H
(wherein R¹ is as described above, v is 0 or a positive integer, w is a positive integer, and z is 0 or a positive integer). These organohydrogenpolysiloxanes are straight-chain organohydrogenpolysiloxanes having a silicon-bonded hydrogen atom on (i) only the side chain, (ii) the side chain or one molecular terminal, or (iii) the side chain or both molecular terminals.

The monovalent organic group is not particularly limited but is preferably selected from the following (D1) to (D10):
(D1) a substituted or unsubstituted, straight-chain or branched monovalent hydrocarbon group having from 1 to 60 carbon atoms;
(D2) a polyoxyalkylene group expressed by -R⁸O(AO)_{z}R⁹ (wherein AO is an oxyalkylene group having from 2 to 4 carbon atoms; R⁸ is a substituted or unsubstituted, straight-chain or branched divalent hydrocarbon group having from 3 to 5 carbon atoms; R⁹ is a hydrogen atom, a substituted or unsubstituted, straight-chain or branched monovalent hydrocarbon group having from 1 to 24 carbon atoms, or a substituted or unsubstituted, straight-chain or branched acyl group having from 2 to 24 carbon atoms; and z=1 to 100);
(D3) a substituted or unsubstituted, straight-chain or branched alkoxy group having from 1 to 30 carbon atoms;
(D4) a hydroxyl group;
(D5) an ester group expressed by -R¹⁰-COOR¹¹ (wherein R¹⁰ is a substituted or unsubstituted, straight-chain or branched divalent hydrocarbon group having from 2 to 20 carbon atoms, and R¹¹ is a substituted or unsubstituted, straight-chain or branched monovalent hydrocarbon group having from 1 to 30 carbon atoms);
(D6) an ester group expressed by -R¹⁷-OCOR¹⁸ (wherein R¹⁷ is a substituted or unsubstituted, straight-chain or branched divalent hydrocarbon group having from 2 to 20 carbon atoms, and R¹⁸ is a substituted or unsubstituted, straight-chain or branched monovalent hydrocarbon group having from 1 to 30 carbon atoms);
(D7) L¹
   here, L¹ is a silylalkyl group having a siloxane dendron structure and, when i=1, is expressed by the following general formula (3): 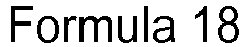 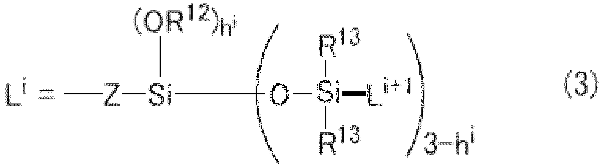 (wherein
   R¹² is a substituted or unsubstituted, straight-chain or branched monovalent hydrocarbon group having from 1 to 30 carbon atoms;
   R¹³ moieties each independently represents an alkyl group or phenyl group having from 1 to 6 carbon atoms;
   Z represents a divalent organic group;
      i represents a generation of the aforementioned silylalkyl group represented by Lⁱ and is an integer of 1 to k when k is the number of generations, which is the number of repetitions of the silylalkyl group; the number of generations k is an integer from 1 to 10; Lⁱ⁺¹ is the silylalkyl group when i is less than k, and R¹³ when i=k; and hⁱ is a number in a range from 0 to 3); (D8) an alkyl group substituted by a chain polysiloxane structure expressed by the following general formula (4): 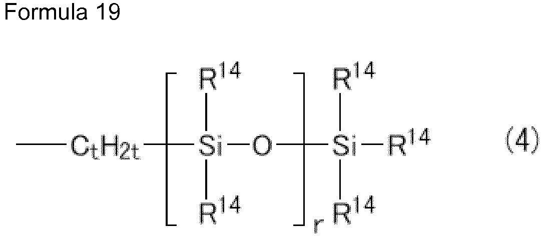 (wherein R¹⁴ moieties are each independently substituted or unsubstituted, straight-chain or branched monovalent hydrocarbon groups having from 1 to 30 carbon atoms, hydroxyl groups, or hydrogen atoms, and at least one of the R¹⁴ moieties is the monovalent hydrocarbon group; t is a number in a range from 2 to 10; and r is a number in a range from 1 to 100);
(D9) an epoxy group expressed by the following general formula (5): 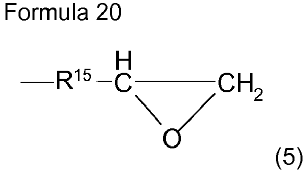 (wherein R¹⁵ is a substituted or unsubstituted, straight-chain or branched divalent hydrocarbon group having from 2 to 20 carbon atoms); and
(D10) a cycloaliphatic epoxy group expressed by the following general formula (6): 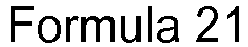 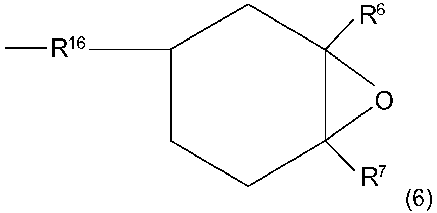
(wherein R¹⁶ is a substituted or unsubstituted, straight-chain or branched divalent hydrocarbon group having from 2 to 20 carbon atoms, and R⁶ and R⁷ are synonymous with those described above).

Examples of the substituted or unsubstituted, straight-chain or branched monovalent hydrocarbon group in (D1), (D2), and (D5) to (D8) include alkyl groups such as methyl groups, ethyl groups, propyl groups, butyl groups, pentyl groups, hexyl groups, heptyl groups, and octyl groups; cycloalkyl groups such as cyclopentyl groups and cyclohexyl groups; alkenyl groups such as vinyl groups, allyl groups, and butenyl groups; aryl groups such as phenyl groups and tolyl groups; aralkyl groups such as benzyl groups; and groups in which the hydrogen atoms bonded to the carbon atoms of these groups are substituted at least partially by halogen atoms such as fluorine atoms or organic groups such as epoxy groups, glycidyl groups, acyl groups, carboxyl groups, amino groups, methacryl groups, and mercapto groups. The monovalent hydrocarbon group is preferably a group other than an alkenyl group, and is particularly preferably a methyl group, an ethyl group, or a phenyl group.

The substituted or unsubstituted, straight-chain or branched divalent hydrocarbon group in (D2), (D5), (D6), (D9), and (D10) is as described above.

Examples of the substituted or unsubstituted, straight-chain or branched alkoxy group in (D3) include lower alkoxy groups such as methoxy groups, ethoxy groups, isopropoxy groups, and butoxy groups and higher alkoxy groups such as lauryl alkoxy groups, myristyl alkoxy groups, palmityl alkoxy groups, oleyl alkoxy groups, stearyl alkoxy groups, and behenyl alkoxy groups.

Among the phenyl group or the alkyl group having from 1 to 6 carbon atoms of (D7), examples of the alkyl group having from 1 to 6 carbon atoms include methyl, ethyl, n-propyl, i-propyl, n-butyl, i-butyl, s-butyl, pentyl, neopentyl, cyclopentyl, hexyl, and similar straight, branched, or cyclic alkyl groups.

In the aforementioned general formula (3), in the case of i = k, R⁴ is preferably a methyl group or a phenyl group. In particular, R⁴ is preferably a methyl group when i=k.

From a technical standpoint, the number of generations k is preferably an integer from 1 to 3, and more preferably is 1 or 2. In each of the number of generations, the group represented by L¹ is expressed as follows. In these formulae, R¹² , R¹³ , and Z are groups synonymous with the groups described above.

When the number of generations is k=1, L¹ is expressed by the following general formula (3-1).

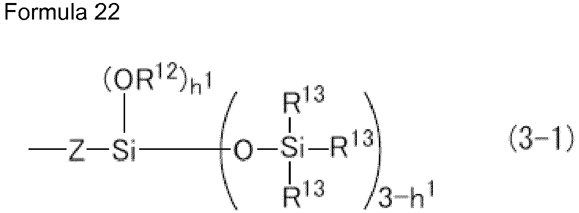

When the number of generations is k=2, L¹ is expressed by the following general formula (3-2).

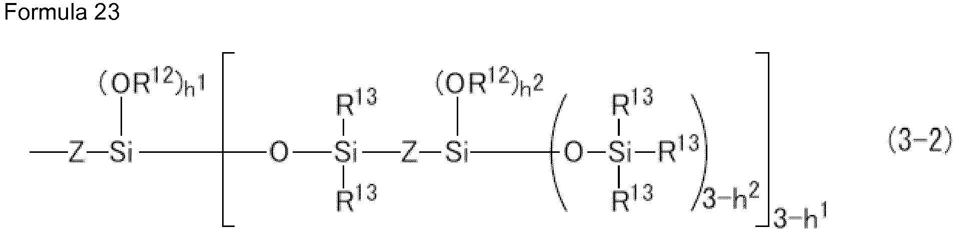

When the number of generations is k=3, L¹ is expressed by the following general formula (3-3).

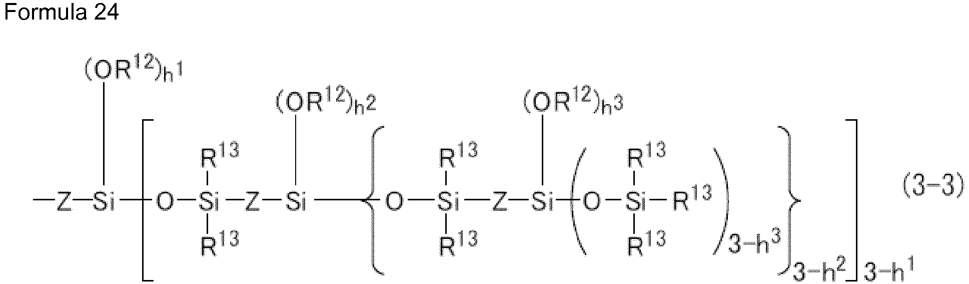

In the structures expressed by the general formulae (3-1) to (3-3) in the case of the number of generations is from 1 to 3, each of h¹ h² and h³ moieties is independently a number in a range from 0 to 3. These hⁱ moieties are preferably a number in a range from 0 to 1, and hⁱ is, in particular, preferably 0.

In general formulae (3) and (3-1) to (3-3), Z are each independently a divalent organic group, and specific examples thereof include a divalent organic group formed by addition-reacting a silicon-bonded hydrogen atom and a functional group having an unsaturated hydrocarbon group such as an alkenyl group, an acryloxy group, a methacryloxy group, or the like at the terminal. Depending on the method for introducing the silylalkyl group having a siloxane dendron structure, the functional group can be appropriately selected and is not restricted to the functional groups described above. Preferably, Z are each independently a group selected from divalent organic groups expressed by the following general formula.

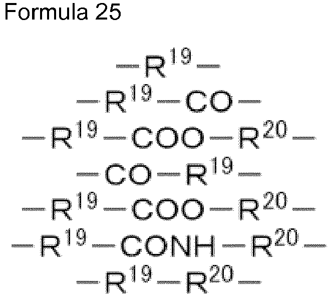

Of these, Z in L¹ is preferably a divalent organic group expressed by general formula -R¹⁹- that is introduced by a reaction between a silicon-bonded hydrogen atom and an alkenyl group. Likewise, Z is preferably a divalent organic group expressed by general formula -R¹⁹-COO-R²⁰- that is introduced by a reaction between a silicon-bonded hydrogen atom and an unsaturated carboxylic ester group. On the other hand, in the silylalkyl group represented by Lⁱ, in which the number of generations k is 2 or more, and Lⁱ is L² to L^{k}, Z is preferably an alkylene group having 2 to 10 carbon atoms and, in particular, is preferably a group selected from an ethylene group, a propylene group, a methylethylene group, and a hexylene group, and most preferably is an ethylene group.

In the general formula described above, R¹⁹ moieties are each independently a substituted or unsubstituted, straight or branched chain alkylene group or alkenylene group having from 2 to 22 carbon atoms or an arylene group having from 6 to 22 carbon atoms. More specifically, examples of R¹⁹ include an ethylene group, a propylene group, a butylene group, a hexylene group, and similar straight alkylene groups; a methylmethylene group, a methylethylene group, a 1-methylpentylene group, a 1,4 -dimethylbutylene group, and similar branched alkylene groups. R⁷ is preferably a group selected from an ethylene group, a propylene group, a methylethylene group, and a hexylene group.

In the general formula described above, R²⁰ is a group selected from divalent organic groups expressed by the following formula.

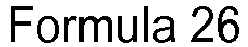

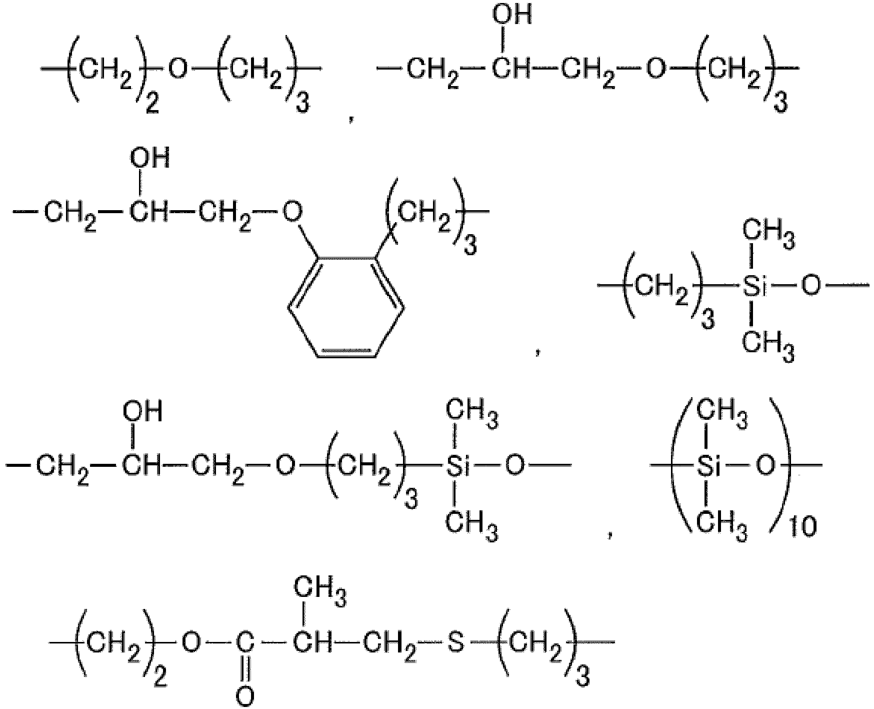

The (B) sugar alcohol-modified group-containing compound having an unsaturated bond is not particularly limited as long as the compound has at least one reactive unsaturated group and one sugar alcohol-modified group in the molecule, but a monounsaturated ether compound of sugar alcohol represented by the following general formula (4'-1): 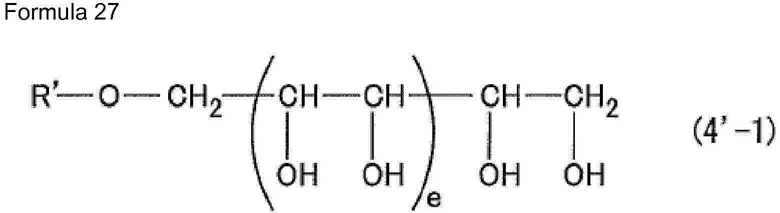 (wherein
R' is an unsaturated organic group, and
e is 1 or 2 and more preferably 1) or the following general formula (4'-2): 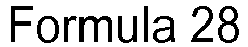 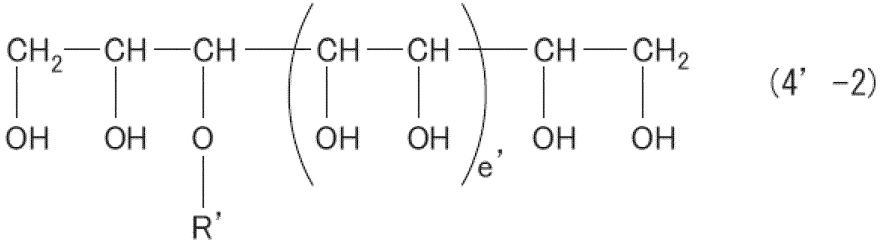
(wherein
R' is an unsaturated organic group, and
e' is 0 or 1 and more preferably 0) is preferable.

The unsaturated organic groups are not particularly limited as long as it has an unsaturated group, but a substituted or unsubstituted, straight-chain or branched unsaturated hydrocarbon group having from 3 to 5 carbon atoms is preferable. Examples of the unsaturated hydrocarbon group having from 3 to 5 carbon atoms include vinyl groups, allyl groups, butenyl groups, methallyl groups and similar alkenyl groups. Among these, allyl groups are preferable.

As the monounsaturated ether compound of the sugar alcohol, a monoallyl ether of a sugar alcohol is preferable, and a xylitol monoallyl ether expressed by the structural formula: CH₂=CH-CH₂-OCH₂[CH(OH)]₃CH₂OH or the structural formula: CH₂=CH-CH₂-OCH[CH(OH)CH₂OH]₂ (hereinafter, referred to as a "xylitol monoallyl ether) is more preferable. The xylitol monoallyl ether can be synthesized with a conventionally known method, and a commercially available product may also be used.

The xylitol monoallyl ether may be only one of the compounds represented by the structural formula: CH₂=CH-CH₂-OCH₂[CH(OH)]₃CH₂OH and the structural formula: CH₂=CH-CH₂-OCH[CH(OH)CH₂OH]₂, or mixtures thereof can be used without any particularly restrictions. In particular, it is preferable to refine any one of xylitol monoallyl ethers represented by the structural formula: CH₂=CH-CH₂-OCH₂[CH(OH)]₃CH₂OH and the structural formula: CH₂=CH-CH₂-OCH[CH(OH)CH₂OH]₂ and use the product as a raw material or to use a xylitol monoallyl ether containing xylitol monoallyl ethers represented by the structural formula: CH₂=CH-CH₂-OCH₂[CH(OH)]₃CH₂OH and the structural formula: CH₂=CH-CH₂-OCH[CH(OH)CH₂OH]₂ at a compositional ratio in a range from 5:5 to 10:0 as a raw material. In the latter case, it is more preferable to use a xylitol monoallyl ether containing the substances at a ratio in a range from 8:2 to 10:0. Moreover, if the compositional ratio is 10:0, the raw material is essentially a refined product comprising only a xylitol monoallyl ether represented by the structural formula: CH₂=CH-CH₂-OCH₂[CH(OH)]₃CH₂OH.

In order to obtain the liquid organopolysiloxane of the present invention, a derivative (ketal derivative) of a sugar alcohol compound prepared by protecting a hydroxyl group of the sugar alcohol-modified group corresponding to the sugar alcohol-modified group to be introduced with a ketalizing agent such as 2,2-dimethoxypropane in the presence of an acid catalyst can be used as a raw material. Specifically, a ketal derivative of a sugar alcohol having a carbon-carbon double bond in the molecule obtained by refining the reaction product of the ketal compound described above and an alkenyl halide is subjected to an addition reaction with an organohydrogenpolysiloxane instead of the monounsaturated ether compound of the sugar alcohol described above. After the addition reaction, the organopolysiloxane can be produced by performing a deketalation reaction by acid hydrolysis treatment to deprotect the hydroxyl group, but this method is typically inefficient. Even with a production method using the ketal derivative described above, an organopolysiloxane having a sugar alcohol-modified group can be obtained after deprotection, so either production method may be selected in accordance with conditions such as the desired yield, the production equipment, and the refinement of the raw material. Either production method may also be selected for the purpose of improving the quality such as the purity or the desired characteristics of the organopolysiloxane.

There are no particularly restrictions regarding the structure of (C1) the organic compound having an average number of unsaturated bonds in the molecule that is greater than 1 serving as the component (C) as long as the compound has more than 1 - preferably from 1.01 to 10, more preferably from 1.2 to 8, even more preferably from 1.5 to 6, and particularly preferably from 2.0 to 4.5 - unsaturated bonds and preferably carbon-carbon double bonds on average in the molecule, straight-chain, branched, or reticulated organic compounds may be used. An organopolysiloxane or an unsaturated aliphatic hydrocarbon is preferable as an organic compound. There are also no restrictions regarding the position of the organic compound, preferably the organopolysiloxane, the unsaturated aliphatic hydrocarbon, or the aforementioned unsaturated bond, and the component may be positioned on the main chain or on a terminal. However, from the perspective of the ease of controlling the crosslinking density, it is preferable to use a compound of high purity having two unsaturated groups in the molecule, each of which is positioned at either terminal, for example.

An unsaturated bond is preferably present in an unsaturated aliphatic hydrocarbon group. The unsaturated aliphatic hydrocarbon group preferably has from 2 to 30 carbon atoms and more preferably has from 2 to 20 carbon atoms. Examples of the monovalent unsaturated aliphatic hydrocarbon group having from 2 to 30 carbon atoms include straight-chain or branched alkenyl groups such as vinyl groups, 1-propenyl groups, allyl groups, isopropenyl groups, 1-butenyl groups, 2-butenyl groups, pentenyl groups, and hexenyl groups; cycloalkenyl groups such as cyclopentenyl groups and cyclohexenyl groups; cycloalkenylalkyl groups such as cyclopentenylethyl groups, cyclohexenylethyl groups, and cyclohexenylpropyl groups; and alkynyl groups such as ethynyl groups and propargyl groups. Alkenyl groups are preferred, and vinyl groups and hexenyl groups are particularly preferred.

When the component (C1) is an organopolysiloxane, the unsaturated aliphatic hydrocarbon group containing an unsaturated bond is preferably bonded to a silicon atom. In addition, when the component (C1) is an organopolysiloxane, the group bonding to silicon atoms other than the unsaturated aliphatic hydrocarbon may be a substituted or unsubstituted monovalent hydrocarbon group or a monovalent organic group having a reactive functional group.

Substituted or unsubstituted monovalent hydrocarbon groups are typically substituted or unsubstituted, straight or branched monovalent saturated hydrocarbon groups having from 1 to 30 carbon atoms, preferably from 1 to 10 carbon atoms, and more preferably from 1 to 4 carbon atoms, and substituted or unsubstituted monovalent aromatic hydrocarbon groups having from 6 to 30 carbon atoms, and more preferably from 6 to 12 carbon atoms. Moreover, component (C1) may contain, as a monovalent organic group, a hydroxyl group or an alkoxy group having from 1 to 12 carbon atoms, such as a methoxy group, an ethoxy group, a propoxy group or a butoxy group.

Examples of the monovalent saturated hydrocarbon group having from 1 to 30 carbon atoms include straight chain or branched chain alkyl groups such as methyl groups, ethyl groups, n-propyl groups, isopropyl groups, n-butyl groups, isobutyl groups, sec-butyl groups, tert-butyl groups, pentyl groups, hexyl groups, heptyl groups, octyl groups, nonyl groups, decyl groups, and the like; and cycloalkyl groups such as cyclopentyl groups, cyclohexyl groups, cycloheptyl groups, cyclooctyl groups, and the like.

Examples of the monovalent aromatic hydrocarbon group having from 6 to 30 carbon atoms include aryl groups such as phenyl groups, tolyl groups, xylyl groups, mesityl groups, and the like. Of these, a phenyl group is preferable. Note that, in the present specification, "aromatic hydrocarbon group" also includes groups in which an aromatic hydrocarbon and a saturated aliphatic hydrocarbon are conjugated in addition to groups formed only from an aromatic hydrocarbon. Examples of groups in which an aromatic hydrocarbon and a saturated hydrocarbon are conjugated include aralkyl groups such as benzyl groups, phenethyl groups, and the like.

Hydrogen atoms in the above-mentioned monovalent hydrocarbon groups may be substituted by one or more substituted groups, and said substituted groups may be selected from the group consisting of, for example, a halogen atom (a fluorine atom, a chlorine atom, a bromine atom, or an iodine atom), a hydroxyl group, an amide group, an ester group, a carboxyl group and an isocyanate group. A monovalent saturated or aromatic hydrocarbon group having at least one of the above-mentioned substituted groups is preferred. Specifically, it is possible to use a 3,3,3-trifluoropropyl group, a 3-chloropropyl group, a 3-hydroxypropyl group, a 3-(2-hydroxyethoxy)propyl group, a 3-carboxypropyl group, a 10-carboxydecyl group, a 3-isocyanatopropyl group and the like.

Examples of monovalent organic groups having reactive functional groups include monovalent saturated or aromatic hydrocarbon groups having reactive functional groups selected from the group consisting of, for example, hydroxyl groups, mercapto groups, epoxy groups, amino groups, amide groups, ester groups, carboxyl groups and isocyanate groups. One or a plurality of reactive functional groups may exist in the monovalent organic group. R¹ is preferably a monosaturated or aromatic hydrocarbon group having at least one of the reactive functional groups described above. Specific examples of the reactive functional group include 3-hydroxypropyl groups, 3-(2-hydroxyethoxy)propyl groups, 3-mercaptopropyl groups, 2,3-epoxypropyl groups, 3,4-epoxybutyl groups, 4,5-epoxypentyl groups, 2-glycidoxyethyl groups, 3-glycidoxypropyl groups, 4-glycidoxybutyl groups, 2-(3,4-epoxycyclohexyl) ethyl groups, 3-(3,4-epoxycyclohexyl)propyl groups, aminopropyl groups, N-methylaminopropyl groups, N-butylaminopropyl groups, N,N-dibutylaminopropyl groups, 3-(2-aminoethoxy)propyl groups, 3-(2-aminoethylamino)propyl groups, 3-carboxypropyl groups, 10-carboxydecyl groups, 3-isocyanate propyl groups, and the like.

A straight-chain, cyclic, or branched polysiloxane is preferable as the component (C1). A straight-chain component (C1) is preferably a polymer having a diorganosiloxane unit and a triorganosiloxane unit, examples of which include dimethylpolysiloxanes capped at both molecular terminals with dimethylvinylsiloxy groups, copolymers of dimethylsiloxane and methylphenylsiloxane capped at both molecular terminals with dimethylvinylsiloxy groups, copolymers of dimethylsiloxane and methylvinylsiloxane capped at both molecular terminals with dimethylvinylsiloxy groups, copolymers of dimethylsiloxane and methylvinylsiloxane capped at both molecular terminals with trimethylsiloxy groups, copolymers of dimethylsiloxane, methylvinylsiloxane and methylphenylsiloxane capped at both molecular terminals with trimethylsiloxy groups, copolymers of dimethylsiloxane and methylvinylsiloxane capped at both molecular terminals with silanol groups, polymers in which some of the methyl groups in these polymers are substituted by alkyl groups other than methyl groups, such as ethyl groups or propyl groups, or halogenated alkyl groups such as 3,3,3-trifluoropropyl groups, and mixtures of two or more of these polymers, with straight-chain diorganopolysiloxanes having unsaturated aliphatic hydrocarbon groups, and especially alkenyl groups, at both molecular terminals only being particularly preferred.

It is particularly preferable for a branched chain polysiloxane of component (C1) to be a polymer that contains a diorganosiloxane unit, an organosilsesquioxane unit and a triorganosiloxy unit. Silicon-bonded organic groups in these units are preferably monovalent hydrocarbon groups including alkyl groups such as methyl groups, ethyl groups and propyl groups; alkenyl groups such as vinyl groups, allyl groups, butenyl groups and hexenyl groups; aryl groups such as phenyl groups and tolyl groups; and halogenated alkyl groups such as 3,3,3-trifluoropropyl groups, and the like, and may contain extremely small quantities of hydroxyl groups and alkoxy groups such as methoxy groups, but at least two silicon-bonded organic groups in this polymer must be unsaturated aliphatic hydrocarbon groups, and especially alkenyl groups. In addition, the proportions of these units are not limited, but in this polymer, it is preferable for diorganosiloxane units to account for in the range of 80.00 to 99.65 mol% and organosilsesquioxane units to account for in the range of 0.10 to 10.00 mol%, with the balance comprising triorganosiloxy units.

Examples of the component (C1), which is a cyclic polysiloxane, include methylvinylcyclosiloxanes and methylhexenylcyclosiloxanes.

Examples of the component (C1) include (C1-5) unsaturated group-containing silicone compounds expressed by the average composition formula (2-5):

Formula 29 R²ₚR³_{q}SiO_{(4-p-q)/2} (2-5)

(wherein R² moieties may each be independent from one another but are monovalent organic groups that are different from R³;
R³ moieties are each independently monovalent unsaturated aliphatic hydrocarbon groups having from 2 to 30 carbon atoms, 1≤p≤2.5, and 0.001≤q≤1.5). The monovalent unsaturated aliphatic hydrocarbon group having from 2 to 30 carbon atoms is as described above.

In the average composition formula (2-5), the monovalent organic group represented by R² is not particularly limited, but is preferably selected from the following (E1) to (E6):
(E1) a substituted or unsubstituted, straight-chain or branched monovalent hydrocarbon group having from 1 to 60 carbon atoms (excluding monovalent hydrocarbon groups having from 2 to 20 carbon atoms and an aliphatic unsaturated group);
(E2) a hydroxyl group;
(E3) an ester group expressed by -R¹⁰-COOR¹¹ (wherein R¹⁰ and R¹¹ are as defined above);
(E4) an ester group expressed by -R¹⁷-OCOR¹⁸ (wherein R¹⁷ and R¹⁸ are as defined above);
(E5) an amide group expressed by -R²¹-NR²²COR²³ (wherein R²¹ is a substituted or unsubstituted, straight-chain or branched divalent hydrocarbon group having from 2 to 20 carbon atoms, R²² is a hydrogen atom, or a substituted or unsubstituted, straight-chain or branched monovalent hydrocarbon group having from 1 to 20 carbon atoms, and R²³ is a substituted or unsubstituted, straight-chain or branched monovalent hydrocarbon group having from 1 to 30 carbon atoms); and
(E6) an amide group expressed by -R²⁴-CONR²⁵R²⁶ (wherein R²⁴ is a substituted or unsubstituted, straight-chain or branched divalent hydrocarbon group having from 2 to 20 carbon atoms, and R²⁵ and R²⁶ are each independently a hydrogen atom or a substituted or unsubstituted, straight-chain or branched monovalent hydrocarbon group having from 1 to 20 carbon atoms).

The definitions, types, and the like of the substituted or unsubstituted, straight-chain or branched monovalent hydrocarbon groups or divalent hydrocarbon groups are as described above.

On the other hand, the component (C1) may be an unsaturated aliphatic hydrocarbon. Examples of unsaturated aliphatic hydrocarbons include various dienes, diynes, enynes and similar products having two or more unsaturated bonds. In view of crosslinking, dienes, diynes, and enynes are preferable. Dienes, diynes, and enynes are compounds having a structure in which at least two unsaturated bonds are separated by one or more, and preferably two or more single bonds in a molecule. The unsaturated aliphatic hydrocarbon group may be present at the terminal of the molecular chain, or as a pendant group in the molecular chain.

Examples of unsaturated aliphatic hydrocarbons serving as the component (C1) include α,ω-unsaturated alkenes and alkynes having from 2 to 30 carbon atoms. Examples of the component (C1) include (C1-1) an α,ω-diene expressed by the general formula (2-1):

Formula 30 CH₂=CH(CH₂)ₓCH=CH₂ (2-1)

(wherein 1≤x≤20); (C1-2) an α,ω-diyne expressed by the general formula (2-2):

Formula 31 CH=C(CH₂)ₓC=CH (2-2)

(wherein 1≤x≤20); (C1-3) an α,ω-ene-yne expressed by the general formula (2-3):

Formula 32 CH₂=CH(CH₂)ₓC=CH (2-3)

(wherein 1≤x≤20); and (C1-4) a bisalkenyl polyether compound expressed by the general formula (2-4):

Formula 33 CₘH₂ₘ₋₁O(CₙH₂ₙO)_{y}CₘH₂ₘ₋₁ (2-4)

(wherein 2≤m≤20, 2≤n≤4, y is the total value of the number of repetitions of the oxyethylene unit, the oxypropylene unit, and the oxybutylene unit, and 1≤y≤180).

Specific examples of unsaturated aliphatic hydrocarbons serving as the component (C1) include 1,4-pentadiene, 1,5-hexadiene, 1,6-heptadiene, 1,7-octadiene, 1,8-nonadiene, 1,9-decadiene, 1,11-dodecadiene, 1,13-tetradecadiene, 1,19-eicosadiene, 1,3-butadiene, 1,5-hexadiyne, and 1-hexene-5-yne.

The component (C1) may be a single component, but may also be a mixture of two or more components having different structures. That is, the component (C1) may be a mixture of one or more types of organopolysiloxanes and one or more types of unsaturated aliphatic hydrocarbons. Therefore, "having a number of unsaturated bonds greater than 1 on average" means having more than one unsaturated bond on average per molecule when two or more types of organopolysiloxanes and/or unsaturated aliphatic hydrocarbons are used.

The (C2) organic compound having at least one unsaturated bond and at least one epoxy group in the molecule serving as the component (C) is not structurally limited as long as the compound has a total of two or more - preferably from 2 to 10, more preferably from 2 to 7, even more preferably from 2 to 5, and particularly preferably from 2 to 4 - unsaturated bonds and epoxy groups in the molecule, and straight-chain, branched, or reticulated organic compounds can be used. An organopolysiloxane or an unsaturated aliphatic hydrocarbon is preferable as an organic compound. There are also no restrictions regarding the position of the organic compund, preferably, the organopolysiloxane, the unsaturated aliphatic hydrocarbon, or the aforementioned unsaturated bond, and the component may be positioned on the main chain or on a terminal. However, from the perspective of the ease of controlling the crosslinking density, it is preferable to use a compound of high purity in which the total of unsaturated groups and epoxy groups in the molecule is two.

An unsaturated bond is preferably present in an unsaturated aliphatic hydrocarbon group. Examples of unsaturated aliphatic hydrocarbon groups are as described above.

When the component (C2) is an organopolysiloxane, the unsaturated aliphatic hydrocarbon group containing an unsaturated bond and/or the epoxy group is preferably bonded to the silicon atom. In addition, when the component (C2) is an organopolysiloxane, the group bonding to silicon atoms other than the unsaturated aliphatic hydrocarbon or the epoxy group may be a substituted or unsubstituted monovalent hydrocarbon group or a monovalent organic group having a reactive functional group as described above.

The component (C2) is preferably an epoxy group-containing unsaturated aliphatic hydrocarbon having at least one epoxy group. Examples of the unsaturated aliphatic hydrocarbon include compounds having the unsaturated aliphatic hydrocarbon groups described above. A compound having a monovalent unsaturated aliphatic hydrocarbon group is preferable.

Examples of the component (C1) include: (C2-1) an unsaturated epoxy compound expressed by the general formula (2-6): 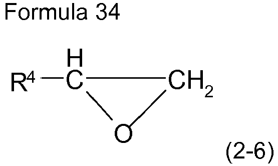 (wherein R⁴ is a substituted or unsubstituted, straight-chain or branched monovalent hydrocarbon group having one unsaturated bond and from 2 to 20 carbon atoms); and (C2-2) an unsaturated group-containing cycloaliphatic epoxy compound expressed by the general formula (2-7): 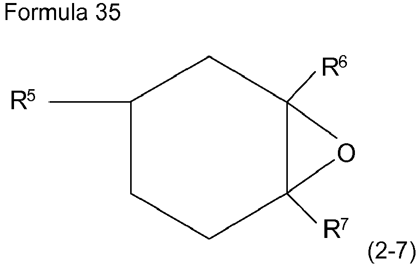 (wherein R⁵ is a substituted or unsubstituted, straight-chain or branched monovalent hydrocarbon group having one unsaturated bond and from 2 to 20 carbon atoms;
R⁶ is a hydrogen atom or a methyl group; and
R⁷ is a hydrogen atom or a methyl group). The definitions, types, and the like of the unsaturated bonds in general formulas above and the substituted or unsubstituted, straight-chain or branched monovalent hydrocarbon groups are as described above.

Specific epoxy group-containing unsaturated aliphatic hydrocarbons serving as the component (C2) include an allylglycidylether, methallylglycidylether, 1-methyl-4-isopropenylcyclohexene oxide, 1,4-dimethylcyclohexene oxide, 4-vinylcyclohexene oxide, vinylnorbornene monooxide, dicyclopentadiene monooxide, butadiene monooxide, 1,2-epoxy-5-hexene, 1,2-epoxy-9-decene, and 2,6-dimethyl-2,3-epoxy-7-octene. Among these, 4-vinyl cyclohexane oxide is preferable. Examples of unsaturated aliphatic hydrocarbons having a cyclic structure include dicyclopentanediene, divinylbenzene, cyclohexanediene, cyclooctadiene, and cyclopentadiene.

The component (C2) may be a single component, but may also be a mixture of two or more components having different structures.

The reaction for producing the liquid organopolysiloxane of the present invention may be performed in the presence or in the absence of a reaction solvent in accordance with a known method. The reaction between the unsaturated group and the Si-H group in the present invention is a hydrosilylation reaction. In addition, when crosslinking is performed using an epoxide of (C2) the organic compound having one or more unsaturated bonds and one or more epoxy groups in the molecule, bonding caused by the reaction of the unsaturated group and the Si-H group and ether bond generation caused by the self ring-opening polymerization of the epoxy groups (cationic polymerization reaction that occurs in the presence of a SiH group and a platinum catalyst) both occur, resulting in crosslinking. In order to accelerate this reaction, irradiation using high energy beams such as ultraviolet light can be applied, or a common cation polymerization catalyst can be further added.

The reaction solvent is not particularly limited as long as the solvent is nonreactive, and examples thereof include alcohol-based solvents such as ethanol and isopropyl alcohol; aromatic hydrocarbon-based solvents such as toluene and xylene; ether-based solvents such as dioxane and THF; aliphatic hydrocarbon-based solvents such as n-hexane, cyclohexane, n-heptane, cycloheptane, and methylcyclohexane; and chlorinated hydrocarbon-based organic solvents such as carbon tetrachloride. An oil agent described below may also be used as a reaction solvent. When an oil agent is used as a reaction solvent, a composition comprising an organopolysiloxane and an oil agent can be obtained directly after the hydrosilylation reaction (crosslinking).

The hydrosilylation reaction may be performed in the presence or absence of a catalyst, but preferably is performed in the presence of a catalyst because the reaction can be carried out at a low temperature and in a shorter period of time. Examples of the hydrosilylation reaction catalyst include platinum, ruthenium, rhodium, palladium, osmium, iridium, and similar compounds, and platinum compounds are particularly effective due to their high catalytic activity. Examples of the platinum compound include chloroplatinic acid; platinum metal; platinum metal supported on a carrier such as platinum supported on alumina, platinum supported on silica, platinum supported on carbon black, or the like; and a platinum complex such as platinum-vinylsiloxane complex, platinum-phosphine complex, platinum-phosphite complex, platinum alcoholate catalyst, or the like. A usage amount of the catalyst is about 0.5 to 1000 ppm in terms of platinum metal, when using a platinum catalyst.

A reaction temperature of the hydrosilylation reaction is typically from 30 to 150°C, and a reaction time is typically from 10 minutes to 24 hours and preferably from 1 to 10 hours.

The component (A) is crosslinked by the component (C) as a result of the hydrosilylation reaction or the cationic polymerization reaction of the epoxy groups, and the polysiloxane chains originating from the component (A) are linked by the crosslinking portion having a carbon-silicon bond originating from the component (C). The component (A) also comprises a sugar alcohol-modified group originating from the component (B). In this way, the liquid organopolysiloxane of the present invention can be obtained.

Note that although the liquid organopolysiloxane of the present invention essentially has a structure that is linked by the crosslinking portion having a carbon-silicon bond originating from the component (C), it may also have a portion crosslinked by the Si-O-C bond. This is because when the structure has a condensation-reactable functional group such as a silanol group or an alkoxy group in the components (A) to (C), links can not only be formed between polysiloxane chains but can also be formed intermittently as a result of a partial reaction between the hydroxyl groups in the sugar alcohol-modified group originating from the component (B) and the Si-H groups of (A) when the crosslinking conditions are severe.

The component (A) is crosslinked by the component (C) as a result of the hydrosilylation reaction, and the polysiloxane chains originating from the component (A) are linked by the crosslinking portion having a carbon-silicon bond originating from the component (C). In addition, the component (A) comprises a sugar alcohol-modified group originating from the component (B). In this way, the liquid organopolysiloxane of the present invention can be obtained.

Further, the liquid organopolysiloxane may be subjected to hydrogenation treatment for the purpose of improving the odor after the reaction caused by the remaining unsaturated compound. Methods of hydrogenation treatment include a method using a pressurized hydrogen gas and a method using a hydrogenation agent such as a metal hydride, and hydrogenation treatment may also entail homogeneous reactions and heterogeneous reactions. One of these methods may be performed alone, or multiple methods may be performed in combination. However, taking into consideration the advantage that the catalyst that is used will not remain in the finished product, a heterogeneous catalytic hydrogenation reaction using a solid catalyst is most preferable.

Examples of solid catalysts (hydrogenation catalyst) that can be used include noble metal-based catalysts such as common platinum-based catalysts and palladium-based catalysts as well as nickel-based catalysts. More specific examples include single substances such as nickel, palladium, platinum, rhodium, and cobalt and catalysts combining a plurality of metals such as platinum-palladium, nickel-copper-chromium, nickel-copper-zinc, nickel-tungsten, and nickel-molybdenum. Examples of an optional catalyst carrier include activated carbon, silica, silica alumina, alumina, and zeolite. In addition, copper-containing hydrogenation catalysts such as Cu-Cr, Cu-Zn, Cu-Si, Cu-Fe-Al, and Cu-Zn-Ti may be used. The form of the hydrogenation catalyst differs depending on the type of the reaction vessel and therefore cannot be determined generally, but the form is typically selected appropriately from forms such as a powder, granules, or pellets. In addition, the platinum catalyst used in the synthesis process (hydrosilylation reaction) can be used directly. These hydrogenation catalysts may be used alone or as a combination of two or more types of catalysts.

The hydrogenation treatment can also be used to refine a crude product of the liquid organopolysiloxane obtained by the hydrosilylation reaction described above. Specifically, a crude product can be refined by deodorization resulting from the hydrogenation treatment in a solvent or without a solvent in the presence of a hydrogenation catalyst, and such a refined product can be used much more preferably when applied to an external use preparation or a cosmetic composition in which the reduction of odor and compatibility with other components are desired. Moreover, the deodorizing treatment preferably has, as a pre-process or a post-process, a stripping treatment in which nitrogen gas is brought into contact with the crude liquid organopolysiloxane or the hydrogenated product to remove light matter under reduced pressure.

In the production method for the liquid organopolysiloxane of the present invention, (A) an organohydrogenpolysiloxane, (B) a sugar alcohol-group containing organic compound having an unsaturated bond, and (C) at least one type of organic compound selected from the group consisting of (C1) an organic compound having an average number of reactive unsaturated bonds in the molecule that is greater than 1 and (C2) an organic compound having at least one unsaturated bond and at least one epoxy group in the molecule are essential components, and other components are preferably reacted with the component (A) successively in the presence of a hydrosilylation reaction catalyst.

In the production of the liquid organopolysiloxane of the present invention, the component (C) may be further reacted with the component (A) after the reaction between the component (A) and the component (B), or the component (B) can be further reacted with the component (A) after the reaction between the component (A) and the component (C).

When the component (C) is further reacted with the component (A) after the reaction between the component (A) and the component (B), the average value of the number of silicon-bonded hydrogen atoms per molecule of the component (A) reacting with the unsaturated bonds of the component (C) is preferably greater than 0.1 and less than 2. That is, the number of silicon-bonded hydrogen atoms per molecule of the component (A) which constitute the crosslinking portion and react with the unsaturated bonds of the component (C) is, on average, preferably in a range from 0.2 to 15 and more preferably in a range from 0.6 to 1.3.

In the production of the liquid organopolysiloxane of the present invention, (Q) an organic compound having one unsaturated bond in the molecule (excluding the component (C2) may be further reacted in addition to the components (A), (B), and (C). One type of the component (Q) may be used, or two or more types of the component (Q) may be used in combination. The reactions are preferably performed sequentially in the presence of a hydrosilylation reaction catalyst. Note that the definitions, types, and the like of the unsaturated bonds in the component (Q) are as described above.

For example, when the component (C) is further reacted with the component (A) after the reaction between the component (A) and the component (B), the component (Q) may be reacted with the component (A) prior to the reaction between the component (A) and the component (B), the component (Q) may be reacted with the component (A) after the reaction between the component (A) and the component (B), or the component (Q) may be further reacted with the component (A) after the reaction of the component (C).

For example, when the component (B) is further reacted with the component (A) after the reaction between the component (A) and the component (C), the component (Q) may be reacted with the component (A) prior to the reaction between the component (A) and the component (C), the component (Q) may be reacted with the component (A) after the reaction between the component (A) and the component (C), or the component (Q) may be further reacted with the component (A) after the reaction of the component (B).

Examples of the component (Q) include (Q1) a siloxane dendron compound having one reactive unsaturated group in the molecule and (Q2) a hydrocarbon compound having one reactive unsaturated group in the molecule, a chain organopolysiloxane having one reactive unsaturated group in the molecule, or the like.

Preferable examples of (Q1) the siloxane dendron compound having one reactive unsaturated group in the molecule include compounds having a siloxane dendron structure that have one carbon-carbon double bond at a molecular terminal, the compounds being expressed by the following general formula (3'): 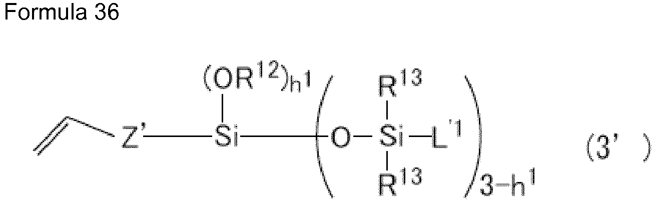 (wherein
R¹² and R¹³ are synonymous with those described above;
Z' is a divalent organic group;
h¹ is a number in a range from 0 to 3;
L¹ is the R¹³ moiety or, when j=1, a silylalkyl group expressed by the general formula (3") below: 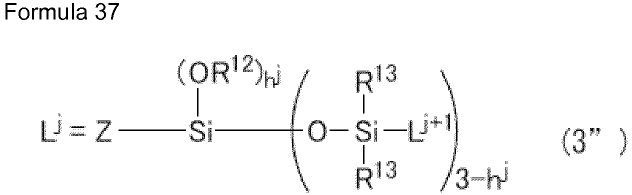
(wherein R¹² and R¹³ are synonymous with those described above;
Z represents a divalent organic group;
j indicates the number of generations of the silylalkyl group represented by L^{j}, when the number of generations (the number of repetitions) of the silylalkyl group is k', j is an integer of 1 to k', and the number of generations k' is an integer from 1 to 9; L^{j+1} is the silylalkyl group when j is less than k' and is the R¹³ moiety when j = k'; and h^{j} is a number in a range from 0 to 3). The divalent organic groups in the general formulas (3') and (3") are as described above.

Preferable examples of (Q2) the hydrocarbon compound having one reactive unsaturated group in the molecule or the chain organopolysiloxane having one reactive unsaturated group in the molecule include monounsaturated hydrocarbon compounds expressed by the following general formula:

Formula 38 R'-R^{2'}

(wherein R' is synonymous with that described above; and
R^{2'} is a substituted or unsubstituted, straight or branched monovalent hydrocarbon group having 7 to 58 carbon atoms, or the chain organosiloxane group represented by the following general formula (4-1): 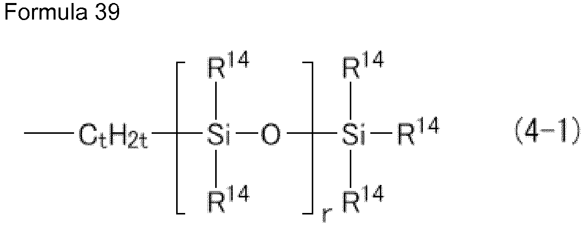 (wherein R¹⁴, t, and r are synonymous with those described above); or the following general formula (4-2): 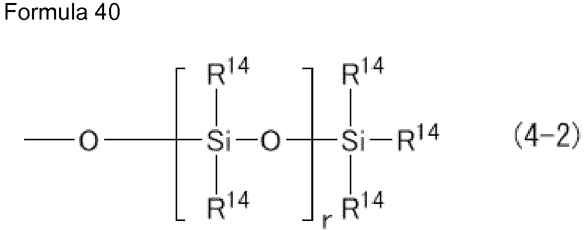 (wherein R¹⁴ and r are as defined above).

The hydrocarbon compound having one reactive unsaturated group in the molecule (Q2) is preferably a monounsaturated hydrocarbons having from 9 to 30 carbon atoms and is more preferably a 1-alkene. Examples of the 1-alkene include 1-nonene, 1-decene, 1-undecene, 1-dodecene, 1-tridecene, 1-tetradecene, and the like. Examples of the chain organopolysiloxane having one reactive unsaturated group in the molecule include a dimethylpolysiloxane capped at one molecular terminal with a vinyl group, a methylphenylpolysiloxane capped at one molecular terminal with a vinyl group, and the like.

In the production of the liquid organopolysiloxane of the present invention, it is preferable to further perform an acidizing process of treating the liquid organopolysiloxane of the present invention obtained by the hydrosilylation reaction of the component (A), the component (B), the component (C), and the optional component (Q) using at least one type of acidic substance. As a result, the odor of the liquid organopolysiloxane can be reduced.

The acidic substance is not particularly limited and may be any acid that matches the definition of a Lewis-acid, a Bronsted acid, or an Arrhenius acid. The acidic substance used in the present invention is preferably a water soluble acid. Therefore, the acidic substance used in the present invention is preferably an Arrheinius acid which releases protons in an aqueous solution. One type of the acidic substance may be used alone, or two or more types of acidic substances may be used in combination. In the present invention, by using such an acidic substance, the liquid organopolysiloxane can be essentially deodorized and the generation of odor over time can be almost completely suppressed without severing the carbon-oxygen bonds or the silicon-oxygen bonds.

The acidic substance can be selected from the group consisting of inorganic acids, organic acids, acidic inorganic salts, solid acids, and acidic platinum catalysts.

The inorganic acid is not particularly limited, and examples thereof include hydrochloric acid, sulfuric acid, nitric acid, phosphoric acid, carbonic acid, boric acid, sulfonic acid, sulfinic acid, and the like. Note that substances containing organic groups such as benzene sulfonic acid are not preferable as inorganic acids.

The organic acid is not particularly limited, and can be a monocarboxylic acid (including monohydroxy monocarboxylic acid and dihydroxy monocarboxylic acid), a dicarboxylic acid (including monohydroxy dicarboxylic acid and dihydroxy dicarboxylic acid), a polycarboxylic acid, or the like. Examples thereof include:
Straight saturated aliphatic monocarboxylic acids (alkanoic acids) such as formic acid, acetic acid, trifluoroacetic acid, propionic acid, butyric acid, valeric acid, caproic acid, enanthic acid, caprylic acid, pelargonic acid, capric acid, undecanoic acid, and the like;
Branched saturated aliphatic monocarboxylic acids (alkanoic acids) such as 2-methylpropanoic acid, 2-methylbutanoic acid, trimethylpropanoic acid, 2-methylpentanoic acid, trimethyl acetic acid, and the like;
Unsaturated aliphatic monocarboxylic acids (alkenoic acids) such as acrylic acid, methacrylic acid, crotonic acid, isocrotonic acid, vinyl acetic acid, allyl acetic acid, hexenoic acid, heptenoic acid, octenoic acid, and the like;
Unsaturated aliphatic monocarboxylic acids (alkynoic acids) such as propiolic acid, tetrolic acid, allyl acetic acid, hexynoic acid, octynoic acid, and the like;
Polyunsaturated aliphatic monocarboxylic acids such as pentadienoic acid, sorbic acid, and the like; α-hydroxymonocarboxylic acids such as citric acid, lactic acid, glycolic acid, α-oxybutyric acid, and the like;
β-hydroxymonocarboxylic acids such as 2-hydroxyvaleric acid, 2-hydroxycaproic acid, β-oxybutyric acid, and the like;
γ-hydroxymonocarboxylic acids such as γ-oxybutyric acid and the like;
Dihydroxymonocarboxylic acids such as glyceric acid and the like;
Other hydroxymonocarboxylic acids such as hydroxy(meth)acrylic acid and the like;
Saturated aliphatic dicarboxylic acids such as oxalic acid, malonic acid, succinic acid, glutaric acid, adipic acid, and the like;
Monohydroxy saturated aliphatic dicarboxylic acids such as tartronic acid, malic acid, and the like; Dihydroxy saturated aliphatic dicarboxylic acids such as tartaric acid and the like;
Unsaturated aliphatic dicarboxylic acids such as maleic acid, fumaric acid, and the like; Aromatic monocarboxylic acid such as benzoic acid and the like;
Aromatic dicarboxylic acids such as phthalic acid and the like;
Amino acids such as glycine, alanine, valine, leucine, glutamic acid, aspartic acid, PL-pyrrolidone carboxylic acid, and the like; and
Polycarboxylic acids such as gallic acid and the like.

In addition, alkyl sulfuric acid, alkyl phosphoric acid, phenol, and the like can be used as the organic acid. Note that higher fatty acids or salts thereof are not preferable as organic acids.

The acidic inorganic salt is not limited, but a water soluble salt is preferable. Particularly preferable is a water soluble acidic inorganic salt that is solid at 25°C and, when 50 g thereof is dissolved in 1 L of ion exchanged water, the solution has a pH at 25°C of not more than 4, preferably not more than 3.5, and more preferably not more than 2.0. When the acidic inorganic salt is solid at room temperature (25°C), the acidic inorganic salt can be easily removed by filtration as necessary. In addition, when the acidic inorganic salt is water soluble, the acidic inorganic salt can be easily rinsed off with water as necessary. Note that the pH values in the present invention are values measured using a pH meter having a glass electrode in a sample aqueous solution at room temperature (25°C).

Examples that can be used as the acidic inorganic salt include acidic inorganic salts in which at least a monovalent hydrogen atom of the inorganic acid that is at least divalent is neutralized by a base. Examples of the inorganic acid that is at least divalent include sulfuric acid, sulfurous acid, and the like. Examples of the base include an alkali metal, ammonia, and the like.

More specifically, the acidic inorganic salt is preferably at least one type of acidic inorganic salt comprising a hydrogensulfate ion (HSO₄⁻) or a hydrogensulfite ion (HSO₃⁻) and a monovalent cation (M⁺). Examples of the monovalent cation (M⁺) include alkali metal ions or an ammonium ion. Particularly, the monovalent cation is preferably at least one type selected from the group consisting of a sodium ion, a potassium ion, and an ammonium ion.

Specific examples of the acidic inorganic salt include lithium hydrogensulfate, sodium hydrogensulfate, potassium hydrogensulfate, rubidium hydrogensulfate, cesium hydrogensulfate, ammonium hydrogensulfate, sodium hydrogensulfite, or hydrates thereof, and Lewis-acids such as AlCl₃, FeCl₃, TiCl₄, and BF₃-Et₂O. The pH values of several aqueous solutions in which 50 g of acidic inorganic acid is dissolved in 1 L of ion exchanged water are as shown in the tables below. From the perspective of the technical benefit of reducing odor, the water soluble acidic inorganic salt having a pH of not higher than 2.0 is most preferably at least one type of acidic inorganic salt selected from the group consisting of sodium hydrogensulfate, potassium hydrogensulfate, and ammonium hydrogensulfate.

### [Table 1]

**Table 1**

| **Acidic inorganic salt** | pH (50 g/L) |
|---|---|
| **Sodium hydrogensulfate** | 1.5 or lower |
| **Potassium hydrogensulfate** | 2.0 or lower |
| **Ammonium hydrogensulfate** | 1.5 or lower |
| **Sodium hydrogensulfite** | 3.5 |

Examples of solid acids that can be used include acidic solid substances such as activated clay, acidic clay, solid acidic zirconium dioxide, strong acidic cation exchange resins, fluorinated sulfonic acid resins, alumina, silica alumina, and zeolite. Of these, a solid acidic zirconium dioxide is preferable. Examples of the solid acidic zirconium dioxide include products prepared at a temperature of at least 300°C after treating zirconium hydroxide with sulfuric acid; more specifically, aluminum hydroxides or hydrous oxides, zirconium hydroxides or hydrous oxides, a solid acidic zirconium - specifically zirconia sulfate - prepared by first obtaining a molded product by mixing and molding a sulfuric acid-containing compound and then baking the molded product at a temperature at which a tetragonal-structured zirconia is formed - specifically at a temperature of at least 300°C. A commercially available product of the solid zirconium dioxide is SZA-60 (manufactured by Japan Energy Corporation). The strong acidic cation exchange resin is, for example, a cation exchange resin in which a functional group is a sulfonic acid group (-SO₃H), and examples of commercially available products thereof include Amberlyst 15, Amberlyst 16, Amberlyst 31, and Amberlyst 35 (manufactured by Organo Corporation). The fluorinated sulfonic acid resin is a perfluorinated polymer having a suspended sulfonic acid group bonded to the polymer chain, and specific examples thereof include the product described in Japanese Examined Patent Application Publication No. S59-4446 and the like.

Examples of the acidic platinum catalyst include chloroplatinic acids, alcohol-modified chloroplatinic acids, olefin complexes of chloroplatinic acids, ketone complexes of chloroplatinic acids, vinylsiloxane complexes of chloroplatinic acids, and platinum tetrachlorides. Of these, chloroplatinic acid is preferable.

The acidizing process described above can be performed by bringing the liquid organopolysiloxane into contact with the acidic substance in a desired manner.

Specifically, the acidizing process can be performed by means of an operation such as adding at least one type of the acidic substance described above and, optionally, an organic solvent such as water or alcohol to a reaction system containing the liquid organopolysiloxane (for example, in a reaction vessel such as a flask) and stirring.

In particular, it is preferable to add at least one type of the acidic substance and water to a reaction system containing the liquid organopolysiloxane and to then agitate or knead and pulverize the mixture using a mechanical force while heating. In addition, this treatment is preferably performed in the presence of a solvent such as a lower monohydric alcohol. The acid treatment can be carried out at any temperature and treatment time, and can be carried out at a temperature from 0 to 200°C and more preferably from 50 to 100°C for a reaction time of from 0.5 to 24 hours and more preferably from about 1 to 10 hours. The amount of the acidic substance that is used can be appropriately selected in accordance with the acid strength, the treatment apparatus, the treatment time, and the treatment temperature. However, when the acidic substance is one with moderate acid strength such as sodium hydrogensulfate, potassium hydrogensulfate, ammonium hydrogensulfate, citric acid, glycolic acid, or phosphoric acid, the content is preferably in a range from 10 to 500 ppm and more preferably in a range from 20 to 200 ppm in the liquid organopolysiloxane. In addition, when the acidic substance is an acidic substance with higher acid strength such as hydrochloric acid or sulfuric acid, the content is preferably in a range from 0.1 to 50 ppm in the liquid organopolysiloxane, and when the acidic substance is a substance with weak acid strength or a solid acid exemplified by activated clay, acidic clay, solid acidic zirconium dioxide, strong acidic cation exchange resin, fluorinated sulfonic acid resin, zeolite, or the like, the content is preferably in a range from 500 to 10,000 ppm in the liquid organopolysiloxane.

In the production method for a liquid organopolysiloxane of the present invention, a process of heating and/or decompressing the composition (stripping process) is preferably included after the acidizing process. Low-boiling-point components, which are odor-causing substances, can be removed (stripped) by the heating and/or decompression described above. In addition, a large amount of the odor-causing substances can be removed by performing the acidizing process once again after stripping. At this time, when the acidic substance remains in the reaction system, it is unnecessary to newly add an acidic substance, which yields the advantage that it is sufficient to add only water. That is, the acidizing process and the stripping process can be respectively repeated two or more times for the purpose of increasing the degree of reduction in odor.

The "low-boiling-point components" removed by the stripping process include, in addition to carbonyl compounds such as propionaldehyde considered to be the odor causing substance, volatile compounds such as the reaction solvent used in the synthesis and the like of the liquid organopolysiloxane.

Note that the stripping process may be performed before the acidizing process.

Known reaction conditions may be used in the stripping method, but stripping under normal pressure or under reduced pressure is preferable, and stripping is preferably performed at a temperature of 120°C or lower. In order to effectively perform the stripping, the stripping is preferably performed under reduced pressure or, for example, performed under a nitrogen gas or similar inert gas stream. A specific example of the low-boiling-point component removal operation is one in which the liquid organopolysiloxane or composition thereof or a hydrogenated product thereof comprising a low boiling point component is placed in a flask having a refluxing cooler, a nitrogen injection port, or the like. While nitrogen gas is supplied, the internal pressure is reduced and the temperature is increased, and the pressure and temperature are then kept constant so as to remove light matter. Here, typically, a pressure reduction parameter is from 0.1 to 10.0 kPa, a heating temperature is from 50 to 170°C, and a treatment time is from 10 minutes to 24 hours.

In the present invention, after the acidizing process, a basic substance may be used to neutralize the liquid organopolysiloxane. One type of the basic substance may be used alone, or two or more types of substances may be used in combination. Examples of the basic substance include organic bases such as sodium hydroxide, potassium hydroxide, calcium hydroxide, barium hydroxide, ammonia water, and sodium hydrogen carbonate; basic buffers such as trisodium phosphate, tripotassium phosphate, trisodium citrate, and sodium acetate; and organic bases such as basic amino acids, amines and pyridines. An amount of the basic substance is preferably an amount needed to neutralize a reaction system comprising the liquid organopolysiloxane but, as necessary, the amount of the basic substance may be adjusted to an amount by which weak acidity or weak alkalinity is obtained.

In the present invention, hydrogenation treatment may be performed before and/or after the acidizing process or before and/or after the stripping process. Although a sufficient odor-reducing effect can be achieved by performing deodorization treatment by means of hydrogenation, the hydrogenation process is complex and requires relatively expensive reagents and special equipment. On the other hand, in the present invention, it is possible to achieve a sufficient odor-reducing effect with the acidizing process described above, so it is unnecessary to perform such hydrogenation. Therefore, hydrogenation can be omitted in the present invention.

### (Composition comprising the liquid organopolysiloxane)

The present invention relates to a composition comprising the liquid organopolysiloxane described above. The compounded amount of the liquid organopolysiloxane in the composition is not particularly limited but can be set in a range from 1 to 99 wt.% (mass%), for example, preferably from 5 to 95 wt.% (mass%), more preferably from 10 to 90 wt.% (mass%), even more preferably from 20 to 80 wt.% (mass%), and even more preferably from 30 to 70 wt.% (mass%) based on the total weight (mass) of the composition. The composition of the present invention preferably has fluidity at 25°C.

The composition of the present invention may contain at least one type of oil agent in addition to the liquid organopolysiloxane. The oil agent is not particularly limited, and a solid, semi-solid, or liquid oil agent may be used. Specific examples of oil agent include one type or two or more types of oil agent selected from the group consisting of include silicone oils, hydrocarbon oils, ester oils, vegetable oils and fats, animal oils and fats, fatty acids, higher alcohols, triglycerides, artificial sebums, and fluorine-based oil agents.

Specific examples of the silicone oils include straight organopolysiloxanes expressed by the following general formula (7), cyclic organopolysiloxanes expressed by the general formula (8), and branched organopolysiloxanes expressed by the general formula (9).

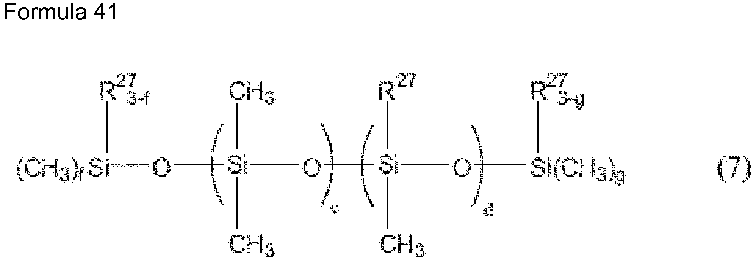

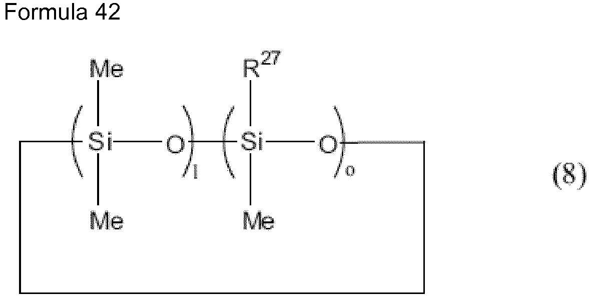

Formula 43 R²⁷₄₋ₛSi(OSiMe₃)ₛ (9)

In the formulas (7) to (9) above, R²⁷ is a hydrogen atom, hydroxyl group or a group selected from a monovalent unsubstituted or fluorine substituted alkyl group having from 2 to 30 carbon atoms, an aryl group, an amino substituted alkyl group, an alkoxy group, and a group expressed by (CH₃)₃SiO[(CH₃)₂SiO]ᵤSi(CH₃)₂CH₂CH₂-. Specific examples thereof include saturated aliphatic hydrocarbon groups such as ethyl groups, propyl groups, butyl groups, pentyl groups, hexyl groups, heptyl groups, octyl groups, decyl groups, and dodecyl groups; unsaturated aliphatic hydrocarbon groups such as vinyl groups, allyl groups, and hexenyl groups; saturated cycloaliphatic hydrocarbon groups such as cyclopentyl groups and cyclohexyl groups; aromatic hydrocarbon groups such as phenyl groups, tolyl groups, and naphthyl groups; and groups in which the hydrogen atoms bonded to the carbon atoms of these groups are substituted partially by an organic group having a halogen atom, an epoxy group, a carboxyl group, an amino group, a methacryl group, a mercapto group, or the like or a group substituted by a trimethylsiloxy group bonded via a divalent hydrocarbon group and/or a chain polydimethyl siloxane bond. Here, c is an integer from 0 to 1,000; d is an integer from 0 to 1,000; c+d is an integer from 1 to 2,000; f and g are each independently 0, 1, 2, or 3; I and o are each independently an integer from 0 to 8, provided that 3≤I+o≤8; s is an integer from 1 to 4; and u is an integer from 0 to 500.

Examples of silicone oils having the structure described above include cyclic organopolysiloxanes such as hexamethyl cyclotrisiloxane (D3), octamethyl cyclotetrasiloxane (D4), decamethyl cyclopentasiloxane (D5), dodecamethyl-cyclohexasiloxane (D6), 1,1-diethylhexamethyl cyclotetrasiloxane, phenylheptamethyl cyclotetrasiloxane, 1,1-diphenylhexamethyl cyclotetrasiloxane, 1,3,5,7-tetravinyltetramethyl cyclotetrasiloxane, 1,3,5,7-tetramethyl cyclotetrasiloxane, 1,3,5,7-tetracyclohexyltetramethyl cyclotetrasiloxane, tris(3,3,3-trifluoropropyl) trimethylcyclotrisiloxane, 1,3,5,7-tetra(3-methacryloxypropyl) tetramethyl cyclotetrasiloxane, 1,3,5,7-tetra(3-acryloxypropyl) tetramethyl cyclotetrasiloxane, 1,3,5,7-tetra(3-carboxypropyl) tetramethyl cyclotetrasiloxane, 1,3,5,7-tetra(3-vinyloxypropyl) tetramethyl cyclotetrasiloxane, 1,3,5,7-tetra(p-vinylphenyl) tetramethyl cyclotetrasiloxane, 1,3,5,7-tetra[3-(p-vinylphenyl) propyl] tetramethyl cyclotetrasiloxane, 1,3,5,7-tetra(N-acryloyl-N-methyl-3-aminopropyl) tetramethyl cyclotetrasiloxane, 1,3,5,7-tetra(N,N-bis (lauroyl)-3-aminopropyl) tetramethyl cyclotetrasiloxane, and the like. Examples of straight organopolysiloxanes include dimethylpolysiloxane in which both molecular terminals are capped with trimethylsiloxy groups (dimethylsilicone with a low viscosity such as 2 cst or 6 cst to dimethylsilicone with a high viscosity such as 1,000,000 cst), organohydrogenpolysiloxane, methylphenylpolysiloxane in which both molecular terminals are capped with trimethylsiloxy groups, a copolymer of methylphenylsiloxane and dimethylsiloxane in which both molecular terminals are capped with trimethylsiloxy groups, diphenylpolysiloxane in which both molecular terminals are capped with trimethylsiloxy groups, a copolymer of diphenylsiloxane and dimethylsiloxane in which both molecular terminals are capped with trimethylsiloxy groups, trimethylpentaphenyltrisiloxane, phenyl (trimethylsiloxy) siloxane, methylalkylpolysiloxane in which both molecular terminals are capped with trimethylsiloxy groups, a copolymer of methylalkylsiloxane and dimethylpolysiloxane in which both molecular terminals are capped with trimethylsiloxy groups, a copolymer of methyl (3,3,3-trifluoropropyl) siloxane and dimethylsiloxane in which both molecular terminals are capped with trimethylsiloxy groups, α,ω-dihydroxypolydimethylsiloxane, α,ω-diethoxypolydimethylsiloxane, 1,1,1,3,5,5,5-heptamethyl-3-octyltrisiloxane, 1,1,1,3,5,5,5-heptamethyl-3-dodecyltrisiloxane, 1,1,1,3,5,5,5-heptamethyl-3-hexadecyltrisiloxane, tristrimethylsiloxymethylsilane, tristrimethylsiloxyalkylsilane, tetrakistrimethylsiloxysilane, tetramethyl-1,3-dihydroxydisiloxane, octamethyl-1,7-dihydroxytetrasiloxane, hexamethyl-1,5-diethoxytrisiloxane, hexamethyldisiloxane, octamethyltrisiloxane, a higher alkoxy-modified silicone, a higher fatty acid-modified silicone, a carbinol-modified silicone (hydrocarbyl functional siloxane), long-chain alkyl-modified silicone, amino-modified silicone, amide-modified silicone, quaternary ammonium salt-modified silicone, and the like. The liquid organopolysiloxane of the present invention is able to finely disperse various powders in an oil phase containing these silicone oils so as to form a low-viscosity slurry, to stably maintain the dispersion state and characteristics, and to stably emulsify/disperse an aqueous phase in an oil phase containing these silicone oils.

Examples of the hydrocarbon oil include liquid paraffin, light liquid isoparaffin, heavy liquid isoparaffin, vaseline, n-paraffin, isoparaffin, isododecane, isohexadecane, polyisobutylene, hydrogenated polyisobutylene, polybutene, ozokerite, ceresin, microcrystalline wax, paraffin wax, polyethylene wax, polyethylene/polypropylene wax, squalane, squalene, pristane, polyisoprene, and the like. The liquid organopolysiloxane of the present invention is able to finely disperse various powders in an oil phase containing these hydrocarbon oils so as to form a low-viscosity slurry, to stably maintain the dispersion state and characteristics, and to stably emulsify/disperse an aqueous phase in an oil phase containing these hydrocarbon oils.

Examples of the ester oil include hexyldecyl octanoate, cetyl octanoate, isopropyl myristate, isopropyl palmitate, butyl stearate, hexyl laurate, myristyl myristate, oleyl oleate, decyl oleate, octyldodecyl myristate, hexyldecyl dimethyloctanoate, cetyl lactate, myristyl lactate, diethyl phthalate, dibutyl phthalate, lanolin acetate, ethylene glycol monostearate, propylene glycol monostearate, propylene glycol dioleate, glyceryl monostearate, glyceryl monooleate, glyceryl tri(2-ethylhexanoate), trimethylolpropane tri(2-ethylhexanoate), ditrimethylolpropane triethylhexanoate, ditrimethylolpropane isostearate/sebacate, trimethylolpropane trioctanoate, trimethylolpropane triisostearate, diisopropyl adipate, diisobutyl adipate, 2-hexyldecyl adipate, di-2-heptylundecyl adipate, diisostearyl malate, hydrogenated castor oil monoisostearate, N-alkylglycol monoisostearate, octyldodecyl isostearate, isopropyl isostearate, isocetyl isostearate, ethylene glycol di-2-ethylhexanoate, cetyl 2-ethylhexanoate, pentaerythritol tetra-2-ethylhexanoate, octyldodecyl gum ester, ethyl oleate, octyldodecyl oleate, neopentylglycol dicaprate, triethyl citrate, 2-ethylhexyl succinate, dioctyl succinate, isocetyl stearate, diisopropyl sebacate, di-2-ethylhexyl sebacate, diethyl sebacate, dioctyl sebacate, dibutyloctyl sebacate, cetyl palmitate, octyldodecyl palmitate, octyl palmitate, 2-ethylhexyl palmitate, 2-hexyldecyl palmitate, 2-heptylundecyl palmitate, cholesteryl 12-hydroxystearate, dipentaerythritol fatty acid ester, 2-hexyldecyl myristate, ethyl laurate, 2-octyldodecylester N-lauroyl-L-glutamate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, isopropyl N-lauroylsarcosinate, diisostearyl malate, neopentylglycol dioctanoate, isodecyl neopentanoate, isotridecyl neopentanoate, isostearyl neopentanoate, isononyl isononanoate, isotridecyl isononanoate, octyl isononanoate, isotridecyl isononanoate, diethylpentanediol dineopentanoate, methylpentanediol dineopentanoate, octyldodecyl neodecanoate, 2-butyl-2-ethyl-1,3-propanediol dioctanoate, pentaerythrityl tetraoctanoate, pentaerythrityl hydrogenated rosin, pentaerythrityl triethylhexanoate, dipentaerythrityl (hydroxystearate/stearate/rosinate), polyglyceryl tetraisostearate, polyglyceryl-10 nonaisostearate, polyglyceryl-8 deca(erucate/isostearate/ricinoleate), (hexyldecanoic acid/sebacic acid) diglyceryl oligoester, glycol distearate (ethylene glycol distearate), diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensate, dimer dilinoleic acid hydrogenated castor oil, hydroxyalkyl dimer dilinoleyl ether, glyceryl triisooctanoate, glyceryl triisostearate, glyceryl trimyristate, glyceryl triisopalmitate, glyceryl trioctanoate, glyceryl trioleate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate eicosane dioate, glyceryl di-2-heptylundecanoate, diglyceryl myristate isostearate, cholesteryl acetate, cholesteryl nonanoate, cholesteryl stearate, cholesteryl isostearate, cholesteryl oleate, cholesteryl 12-hydroxystearate, cholesteryl ester of macadamia nut oil fatty acid, phytosteryl ester of macadamia nut oil fatty acid, phytosteryl isostearate, cholesteryl ester of soft lanolin fatty acid, cholesteryl ester of hard lanolin fatty acid, cholesteryl ester of long-chain branched fatty acid, cholesteryl ester of long-chain α-hydroxy fatty acid, octyldodecyl ricinoleate, octyldodecyl ester of lanolin fatty acid, octyldodecyl erucate, isostearic acid hydrogenated castor oil, ethyl ester of avocado fatty acid, isopropyl ester of lanolin fatty acid, and the like. The liquid organopolysiloxane of the present invention is able to finely disperse various powders in an oil phase containing these ester oils so as to form a low-viscosity slurry, to stably maintain the dispersion state and characteristics, and to stably emulsify/disperse an aqueous phase in an oil phase containing these ester oils.

Examples of natural animal or vegetable oils and fats and semi-synthetic oils and fats include oils and fats such as avocado oil, linseed oil, almond oil, ibota wax, perilla oil, olive oil, cacao butter, kapok wax, kaya oil, carnauba wax, liver oil, candelilla wax, beef tallow, neatsfoot oil, beef bone fat, hydrogenated beef tallow, apricot kernel oil, spermaceti wax, hydrogenated oil, wheat germ oil, sesame oil, rice germ oil, rice bran oil, sugar cane wax, sasanqua oil, safflower oil, shea butter, Chinese tung oil, cinnamon oil, jojoba wax, olive squalane, shellac wax, turtle oil, soybean oil, tea seed oil, camellia oil, evening primrose oil, corn oil, lard, rapeseed oil, Japanese tung oil, rice bran wax, germ oil, horse fat, persic oil, palm oil, palm kernel oil, castor oil, hydrogenated castor oil, castor oil fatty acid methyl ester, sunflower oil, grape oil, bayberry wax, jojoba oil, hydrogenated jojoba ester, macadamia nut oil, beeswax, mink oil, cottonseed oil, cotton wax, Japanese wax, Japanese wax kernel oil, montan wax, coconut oil, hydrogenated coconut oil, tri-coconut oil fatty acid glyceride, mutton tallow, peanut oil, lanolin, liquid lanolin, reduced lanolin, lanolin alcohol, hard lanolin, lanolin acetate, lanolin fatty acid isopropyl ester, POE lanolin alcohol ether, POE lanolin alcohol acetate, lanolin fatty acid polyethylene glycol, POE hydrogenated lanolin alcohol ether, egg yolk oil, and the like. Herein, "POE" means "polyoxyethylene".

Examples of higher fatty acids include lauric acid, myristic acid, palmitic acid, stearic acid, behenic acid, undecylenic acid, oleic acid, linolic acid, linolenic acid, arachidonic acid, eicosapentaenoic acid (EPA), docosahexaenoic acid (DHA), isostearic acid, 12-hydroxystearic acid, and the like.

Examples of higher alcohols include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, sitosterol, phytosterol, lanosterol, POE cholesterol ether, monostearyl glycerol ether (batyl alcohol), monooleyl glycerol ether (selachyl alcohol), and the like.

Examples of the fluorine-based oil agent include perfluoropolyether, perfluorodecalin, perfluorooctane, and the like, and one or two or more types of these oil agents can be used as necessary.

The compounded amount of the oil agent in the composition of the present invention is not particularly limited but can be set in a range from 0.1 to 95 wt.% (mass%), for example, preferably from 1 to 90 wt.% (mass%), more preferably from 2 to 80 wt.% (mass%), even more preferably from 3 to 70 wt.% (mass%), and even more preferably from 5 to 60 wt.% (mass%) based on the total weight (mass) of the composition.

The liquid organopolysiloxane of the present invention has a hydrophobic silicone chain and a hydrophilic sugar alcohol-modified group and therefore functions as a surfactant or an emulsifier. Accordingly, a composition containing the liquid organopolysiloxane of the present invention and at least one type of oil agent can take the form of an emulsion. The emulsion form is not particularly limited and can be a water-based-oil-based oil composition such as an oil-in-water emulsion or a water-in-oil emulsion; or an arbitrary form of emulsion such as an oil-in-alcohol (polyol) emulsion or an alcohol(polyol)-in-oil emulsion.

The composition of the present invention can contain at least one type of acidic substance in addition to the organopolysiloxane. The definitions, types, and the like of the acidic substances are as described above.

The compounded amount of the acidic substance in the composition of the present invention is not particularly limited but can be set in a range from 0.001 to 50 wt.% (mass%), for example, preferably from 0.003 to 25 wt.% (mass%), more preferably from 0.005 to 15 wt.% (mass%), even more preferably from 0.01 to 5 wt.% (mass%)%, and even more preferably from 0.01 to 2 wt.% (mass%) based on the total weight (mass) of the composition.

The composition of the present invention can contain water. The compounded amount of the water in the composition of the present invention is not particularly limited but can be set in a range from 1 to 90 wt.% (mass%), for example, preferably from 5 to 80 wt.% (mass%), more preferably from 10 to 70 wt.% (mass%), even more preferably from 20 to 60 wt.% (mass%), and even more preferably from 30 to 50 wt.% (mass%) based on the total weight (mass) of the composition.

In particular, since the liquid organopolysiloxane of the present invention has a hydrophobic silicone chain and a hydrophilic sugar alcohol-modified group, the liquid organopolysiloxane functions as a surfactant or an emulsifier. That is, by mixing the organopolysiloxane of the present invention and water (or water and a hydrophilic medium), each component can be uniformly dispersed, so the present invention can be advantageously used as a hydrous composition.

The hydrous composition described above is a composition containing the liquid organopolysiloxane of the present invention and water and can be in the form of a hydrous gel composition or an emulsion composition. The emulsion form is not particularly limited and can be a water-based-oil-based oil composition such as an oil-in-water emulsion or a water-in-oil emulsion; or an arbitrary form of emulsion such as an oil-in-alcohol (polyol, for example) emulsion or an alcohol(polyol, for example)-in-oil emulsion. In particular, a water-in-oil emulsion composition or an alcohol(polyol, for example)-in-oil emulsion is preferable.

The average particle size of the emulsion particle formed by emulsification using the liquid organopolysiloxane of the present invention can be measured by a conventional measurement device using a laser diffraction/scattering method or the like. The emulsion composition of the present invention is preferably a polar solvent-in-oil emulsion but may also be an oil-in-polar solvent emulsion. In addition, the emulsion composition of the present invention may be a transparent micro-emulsion in which the measured average particle size is not more than 0.1 µm or may be a large particulate white turbid emulsion in which the average particle size is more than 10.0 µm. Furthermore, the emulsion particles may be micronized for the purpose of improving the stability and transparency of the appearance of the emulsion. An emulsion having a particle diameter from 0.5 to 20 µm can be selected for the purpose of improving sensation during use and adhesion characteristics to hair and skin.

The emulsion and the like described above may be produced by blending the liquid organopolysiloxane of the present invention or composition comprising the same and water by means of a mechanical force using an apparatus such as a homomixer, a paddle mixer, a Henschel mixer, a homo-disper, a colloid mill, a propeller stirrer, a homogenizer, an in-line continuous emulsifier, an ultrasonic emulsifier, or a vacuum kneader. In addition, in the production method of the emulsion composition, the content and compounding ratio of the water is as described above and is preferably appropriately selected within a range from 1 to 99 mass% of the entire emulsion composition in accordance with the form and use of the emulsion.

The composition of the present invention can contain at least one type of alcohol. The alcohol preferably has water miscibility and more preferably is a lower alcohol or a polyhydric alcohol.

Examples of lower alcohols include ethanol, isopropanol, n-propanol, t-butanol, s-butanol, and the like. Examples of polyhydric alcohols include divalent alcohols such as 1,3-butylene glycol, 1,2-butylene glycol, propylene glycol, trimethylene glycol, tetramethylene glycol, 2,3-butylene glycol, pentamethylene glycol, 2-buten-1,4-diol, dibutylene glycol, pentyl glycol, hexylene glycol, octylene glycol, and the like; trivalent alcohols such as glycerol, trimethylol propane, 1,2,6-hexanetriol, and the like; polyhydric alcohols having 4 or more valences such as pentaerythritol, xylitol, and the like; and sugar alcohols such as sorbitol, mannitol, maltitol, maltotriose, sucrose, erythritol, glucose, fructose, a starch-decomposed product, maltose, xylitose, starch-decomposed sugar-reduced alcohol, and the like. Furthermore, examples other than these low-molecule polyhydric alcohols include polyhydric alcohol polymers such as diethylene glycol, dipropylene glycol, triethylene glycol, polypropylene glycol, tetraethylene glycol, diglycerol, polyethylene glycol, triglycerol, tetraglycerol, polyglycerol, and the like.

The compounded amount of the alcohol in the composition of the present invention is not particularly limited but can be set in a range from 0.1 to 50 wt.% (mass%), for example, preferably from 1 to 40 wt.% (mass%), more preferably from 2 to 30 wt.% (mass%), even more preferably from 3 to 20 wt.% (mass%), and even more preferably from 4 to 10 wt.% (mass%) based on the total weight (mass) of the composition.

### (Carbonyl value measurement method)

The degree of odor of the liquid organopolysiloxane of the present invention or the composition containing the same can be determined by the carbonyl value measured from the absorbance of a reaction solution obtained by reacting the liquid organopolysiloxane or the composition containing the same and 2,4-dinitrophenylhydrazine (2,4-DNPH) in a reaction medium containing at least one type of monovalent lower alcohol having from 1 to 4 carbon atoms. Furthermore, in addition to a compound having a carbonyl group such as an aldehyde or a ketone, the "carbonyl compound" also includes a potential carbonyl compound such as an acetal, propenyl ether, or a similar compound that does not comprise a carbonyl group but generates a carbonyl group by decomposing under certain conditions.

Accordingly, in order to assay the degree of odor of the liquid organopolysiloxane of the present invention or the composition containing the same, it is possible to measure the carbonyl value of the liquid organopolysiloxane or the composition containing the same from the absorbance of a reaction solution obtained by reacting the liquid organopolysiloxane containing a carbonyl compound or the composition containing the same and 2,4-dinitrophenylhydrazine in a reaction medium containing at least one type of monovalent lower alcohol having from 1 to 4 carbon atoms.

The carbonyl value measured with the method described above for the liquid organopolysiloxane of the present invention, which is a sugar alcohol-modified silicone, or the composition containing the same is preferably not more than 2.5 Abs/g, more preferably not more than 1.6 Abs/g, and even more preferably not more than 1.2 Abs/g.

In the present invention, the carbonyl value of a liquid organopolysiloxane or a composition comprising the same is determined based on the absorbance of the reaction solution obtained by reacting 2,4-DNPH and a carbonyl compound in the liquid organopolysiloxane or the composition comprising the same, and from this carbonyl value, it is possible to measure the total amount of carbonyl (calculated on a propanal basis) in the liquid organopolysiloxane or in the composition by using a pre-plotted calibration curve.

The "carbonyl value" is the carbonyl content index value, and is a value obtained by converting the absorbance (absorbance at 430 nm or 460 nm) of the reaction solution, obtained by reacting 2,4-DNPH with the sample, to per 1 g of sample.

Measurement of the carbonyl value uses the property of hydrazone, produced by reacting a carbonyl and 2,4-DNPH in the presence of an acid, that the hydrazone becomes quinoid ions in a base and gives color. The carbonyl value is determined based on the absorbance, which indicates the degree of coloring at 430 nm (a maximum wavelength attributable to saturated carbonyl exists in the vicinity thereof) and at 460 nm (a maximum wavelength attributable to unsaturated carbonyl exists in the vicinity thereof).

The "total amount of carbonyl" is the total amount of carbonyl compound for a liquid organopolysiloxane or a composition comprising the same. The concentration of carbonyl compounds (total amount of carbonyl) of various samples (a liquid organopolysiloxane or a composition comprising the same) can be measured by obtaining a calibration curve by measuring carbonyl values of standard samples having known concentrations of the carbonyl compound (concentration of propionaldehyde).

In the measurement method of the present invention, at least a monovalent lower alcohol having from 1 to 4 carbon atoms is used as the reaction solvent in the reaction between a carbonyl compound and 2,4-DNPH, but it is preferable to use water at the same time.

By using water together with a monovalent lower alcohol having from 1 to 4 carbon atoms as the reaction solvent, it is possible to reliably determine the carbonyl value with high precision, even for a sample containing an aldehyde condensation product (potential carbonyl compound) such as an acetal. Thus, it is possible to quantitate the total amount of carbonyls, while taking into consideration carbonyl compounds attributable to these odor-causing substances. Although the reason is unclear, it is inferred that, because water is present in the reaction system, an aldehyde condensation product is decomposed, and the reaction with 2,4-DNPH is reliably performed. In the reaction solvent comprising a monovalent lower alcohol having from 1 to 4 carbon atoms and water, the mixing ratio (mass ratio) of the monovalent lower alcohol having from 1 to 4 carbon atoms to water is preferably from 99.9:0.1 to 50:50, and more preferably from 99:1 to 75:25.

In the present invention, "reaction solvent" indicates a solvent that is present in the reaction system containing 2,4-DNPH and a carbonyl compound in a sample. A reaction solvent is formed by using, in addition to (a) a solvent used to prepare a sample solution, (b) a solvent used to prepare the added acid solution, (c) a solvent used to prepare a solution of 2,4-DNPH, and the like.

Each of (a) an alcohol that forms a sample solution, (b) an alcohol that forms an acid solution, and (c) an alcohol that forms a 2,4-DNPH solution need not be a monovalent lower alcohol having from 1 to 4 carbon atoms, provided that the alcohol in the reaction solvent resulting from mixture thereof contains a monovalent lower alcohol having from 1 to 4 carbon atoms.

In the measurement method of the present invention, it is preferable to use an alcohol as the solvent (hereinafter, also referred to as "diluent solvent") added to make the volume of a reaction solution to be a prescribed amount when measuring the absorbance of a reaction solution, and it is preferable to use a monovalent lower alcohol having from 1 to 4 carbon atoms. Furthermore, all of the diluent solvent need not be an alcohol. Water and/or an organic solvent (organic solvent not having a carbonyl group in its structure, and having low toxicity) can be used as a part of the diluent solvent, within the range that does not impede the effect of the present invention.

The monovalent lower alcohol having from 1 to 4 carbon atoms is preferably a saturated alcohol. Examples thereof include methanol, ethanol, n-propanol, i-propanol, n-butanol, i-butanol, sec-butanol, and tert-butanol. Among these monovalent lower alcohols, it is preferable to appropriately select the monovalent lower alcohol capable of dissolving or uniformly dispersing the sample, according to the structure and composition of a liquid organopolysiloxane or a composition comprising the same of the present invention. For example, in the case of a liquid organopolysiloxane or a composition comprising the same that has a high modification rate attributable to a sugar alcohol and has high hydrophilicity, it is appropriate to select methanol or ethanol. On the other hand, it is often appropriate to select n-propanol or i-propanol, in the case of a liquid organopolysiloxane or a composition comprising the same that has a not high hydrophilicity and a low modification rate attributable to a sugar alcohol. Further, it is appropriate to select n-butanol, i-butanol, or sec-butanol in the case of a liquid organopolysiloxane or a composition containing the same that is comodified by an organic, highly oil group such as a long-chain alkyl group in addition to a sugar alcohol group. As the monovalent lower alcohol having from 1 to 4 carbon atoms, one type can be used independently, or two or more types can be mixed and used.

These alcohols lack toxicity (toxicity such as the toxicity that benzene has), and the alcohols are capable of dissolving various substances having different polarities and molecular weights. Consequently, by using these alcohols as the reaction solvent and diluent solvent, it is possible to safely and easily perform each operation for determining the carbonyl value.

Normally, a monovalent lower alcohol having from 1 to 4 carbon atoms having a purity equivalent to the reagent-grade can be used as the monovalent lower alcohol having from 1 to 4 carbon atoms without a problem. However, if precise analysis is particularly necessary, it is preferable to use the monovalent lower alcohol having a total content of aldehydes and ketones of not more than 3 ppm, preferably not more than 2 ppm, and more preferably not more than 1 ppm (hereinafter, also referred to as "ultrapure alcohol"). By using an ultrapure alcohol with total content of aldehydes and ketones of not more than 3 ppm as the reaction solvent, it is possible to accurately determine, up to three significant digits, the carbonyl value of a sample with a low carbonyl content (e.g., a carbonyl value of less than 2).

As a method of preparing (refining) an ultrapure alcohol, a method that distills an alcohol under normal pressure or under reduced pressure after adding proper quantities of 2,4-DNPH and an acid lacking oxidative action (e.g., hydrochloric acid or trichloroacetic acid) to the alcohol to be refined and heating and stirring this system over several hours, can be used. Furthermore, it is preferable to perform these refining treatments within 24 hours before measuring the absorbance of the reaction solution.

Additionally, as the ultrapure alcohol, it is preferable to use a commercially available highly pure reagent that has been refined so that a total amount of aldehydes and ketones is 3 ppm or less. Examples of commercially available highly pure reagents that can be used as an ultrapure alcohol include ethanol (99.8%) Infinity Pure; ethanol (99.8%) for precise analysis; ethanol (99.5%) for high-performance liquid chromatography; ethanol (99.5%) for spectrometry; 2-propanol (99.9%) Infinity Pure, 2-propanol (99.9%) for precise analysis; 2-propanol (99.5%) for high-performance liquid chromatography; 2-propanol (99.5%) for spectrometry; 1-propanol (99.8%) Infinity Pure, 1-propanol (99.5%) for high-performance liquid chromatography; methanol (99.8%) Infinity Pure, methanol (99.8%) for precise analysis; methanol (99.5%) for high-performance liquid chromatography; methanol (99.5%) for spectrometry, n-butyl alcohol for high-performance liquid chromatography; n-butyl alcohol for spectrometry (all manufactured by Wako Pure Chemical Industries, Ltd.), and the like.

Furthermore, even for such highly pure reagents, the total amount of aldehydes and ketones increases over time, and sometimes exceeds 3 ppm. In addition, the total amount of aldehydes and ketones exceeds 3 ppm in a relatively short time period after opening (e.g. within 24 hours). Therefore, when particularly precise analysis is required, from the standpoint of satisfying the essential conditions for ultrapure alcohols (i.e. a total amount of aldehydes and ketones is not more than 3 ppm), the following highly pure reagents are preferable:
(a) a reagent produced within 6 months before use, and
(b) a reagent opened within 24 hours before use.

In the measurement method of the present invention, the solvent used as the reaction solvent need not be composed solely of a monovalent lower alcohol having from 1 to 4 carbon atoms, or a mixed solvent of water and a monovalent lower alcohol having from 1 to 4 carbon atoms. A low toxicity organic solvent also may be used as a part of the reaction solvent as long as it does not have a carbonyl group in the structure thereof, within a range that does not impede the effect of the present invention. However, when an organic solvent other than a monovalent lower alcohol having from 1 to 4 carbon atoms is used as a part of the reaction solvent, the total amount of aldehydes and ketones contained in the reaction solvent (total excluding water), which is formed by mixing the organic solvent (part) and a monovalent lower alcohol having from 1 to 4 carbon atoms (remaining part), is preferably not more than 3 ppm.

In the measurement method of the present invention, a basic reaction solution (the reaction solution for absorbance measurement) can be prepared by adding an acid and 2,4-DNPH to a sample solution prepared by dissolving a sample in a solvent, reacting the carbonyl compound in the sample and 2,4-DNPH by heat treating this system, adding an alkali in the system after cooling, and then adjusting the volume to a prescribed volume with a diluent solvent. Here, it is preferable to use a volumetric flask with a volume of 10 to 100 mL, as the container for preparing a basic reaction solution.

### (1) Sample solution

The solvent used to prepare the sample solution also constitutes the reaction solvent as is, so such a solvent is preferably a mixed solvent of an ultrapure alcohol and water. The mass of the sample solution (sample and solvent) used to measure absorbance is normally about 2 to 6 g, and preferably about 5 g. The mass of the sample contained in a sample solution differs according to the carbonyl content of the sample (i.e., the carbonyl value) and the prepared quantity of reaction solution for absorbance measurement (i.e., the volume of the used volumetric flask). However, when, for example, a 50 mL volumetric flask is used to prepare a reaction solution (i.e., the reaction solution for absorbance measurement), the mass of the sample contained in a sample solution is preferably from 5 to 250 mg, and more preferably from 10 to 150 mg.

### (2) Acids

Examples of acids added to the sample solution include mineral acids such as dilute sulfuric acid, hydrochloric acid, dilute nitric acid, phosphoric acid, and the like; organic acids such as trichloroacetic acid, trifluoroacetic acid, formic acid, acetic acid, sulfonic acid, phenolic acid, and the like; and Lewis acids such as AlCl₃, FeCl₃, TiCl₄, and the like; and the like. These acids can be used alone or in combinations of two or more. Of these, from the standpoint of accurately quantitating the total amount of carbonyl in a highly refined liquid organopolysiloxane or a composition comprising the same, trichloroacetic acid, dilute sulfuric acid (particularly, the dilute sulfuric acid with a concentration of 20% or less), and hydrochloric acid (particularly, the hydrochloric acid with a concentration of 37% or less) are preferable. In addition, the acid used in the present invention is preferably an acid with the highest purity (reagent-grade or higher purity).

These acids may be added as is to a sample solution. However, from the perspective of performing accurate measurement or the like, it is preferable to add the acids in a solution state obtained by dissolving in an appropriate solvent. Furthermore, the solvent used to prepare an acid solution also constitutes the reaction solvent as is, thus, as such the solvent, it is preferable to use a monovalent lower alcohol having from 1 to 4 carbon atoms or a mixed solvent of monovalent lower alcohol having from 1 to 4 carbon atoms and water. When preparing a reaction solution (a reaction solution that contains from 5 to 250 mg of sample) in a 50 mL volumetric flask, the added amount of acid is preferably from 0.03 to 5.0 g.

### (3) 2,4-DNPH

The 2,4-DNPH added to the sample solutions is preferably a 2,4-DNPH having a purity of at least a reagent-grade and containing an equal amount of water. Also, the purity can be further raised by recrystallization or a similar refining operation. 2,4-DNPH can be added as is to a sample solution. However, from the standpoint of performing an accurate measurement, it is preferable to add the 2,4-DNPH in a solution state obtained by dissolving in an appropriate solvent. Furthermore, the solvent used to prepare the 2,4-DNPH solution also constitutes the reaction solvent as is. Thus, as such the solvent, it is preferable to use a monovalent lower alcohol having from 1 to 4 carbon atoms, or a mixed solvent of water and a monovalent lower alcohol having from 1 to 4 carbon atoms. When preparing a reaction solution (reaction solution that contains from 5 to 250 mg of sample) in a 50 mL volumetric flask, it is preferable to add 0.5 to 100 mg of 2,4-DNPH.

### (4) Heat treatment

A condition for the heat treatment of a mixed solution containing a sample, acid, and 2,4-DNPH is from 20 to 180 minutes at 30 to 120°C (however, the temperature is lower than the boiling point of the reaction solvent). At a treatment temperature below 30°C, it takes a long time to react the 2,4-DNPH and the carbonyl compound in a sample, which is inefficient. On the other hand, when heating at a temperature above 120°C, there is a risk that the generated hydrazone will decompose. When the treatment time is less than 20 minutes, it becomes difficult to complete the reaction with 2,4-DNPH. On the other hand, if the treatment time exceeds 180 minutes, there is a risk that the generated hydrazone will decompose.

### (5) Alkali

It is preferable to use an inorganic strong base such as potassium hydroxide, sodium hydroxide, and the like as the alkali added to the reaction solution resulting from the reaction between the 2,4-DNPH and carbonyl compound in a sample. These alkalis can be added as is to a sample solution. However, from the perspective of performing accurate measurement or the like, it is preferable to add these alkalis in a solution state obtained by dissolving in an appropriate solvent. As such solvents, one or two or more solvents can be selected from among solvents that can dissolve an alkali, do not have a carbonyl group in the structure thereof, are compatible with the solvent used as the reaction solvent, and that have low toxicity. Specific examples thereof include monovalent saturated lower alcohols such as methanol, ethanol, 2-propanol, 1-propanol, and the like, or mixed solvents prepared by mixing a proper quantity of water and/or other organic solvent (the organic solvent without a carbonyl group in their structure and having low toxicity) in these solvents. It is preferable to use an ultrapure alcohol or a mixed solvent of an ultrapure alcohol and water. When a reaction solution (reaction solution that contains from 5 to 250 mg of sample) is prepared in a 50 mL volumetric flask, the added amount of alkali is preferably from 0.05 to 5.0 g.

### (6) Diluent solvent

The reaction solution to which alkali was added is adjusted to a specific volume (for example, 50 mL) with a diluent solvent having an alcohol as a main constituent. The alcohol forming the diluent solvent is preferably an ultrapure alcohol.

### (7) Specific preparation method

An example of a method of preparing a reaction solution for absorbance measurement is as follows. In a 50 mL volumetric flask, 5 g of a sample solution is prepared by dissolving from 5 to 250 mg of a sample in a mixed solvent of water and a monovalent lower alcohol having from 1 to 4 carbon atoms. Next, a solution prepared by dissolving from 0.03 to 5.0 g of acid in a monovalent lower alcohol having from 1 to 4 carbon atoms and a solution prepared by dissolving from 0.5 to 500 mg of 2,4-DNPH in a monovalent lower alcohol having from 1 to 4 carbon atoms are added to the flask. This volumetric flask is then stoppered, and heat treatment is performed for 20 to 180 minutes at 30 to 120°C, thereby reacting the 2,4-DNPH and the carbonyl compound in the sample. After this mixture is cooled to room temperature, a solution prepared by dissolving from 0.05 to 5.0 g of an alkali in an alcohol is added to the flask. A diluent solvent composed of an alcohol is then added to adjust the volume of the mixture to 50 mL.

In the measurement method of the present invention, the basic reaction solution obtained as aforementioned is subjected to filtration, as required, after which the absorbance at 430 nm or 460 nm is measured. Here, when the carbonyl compound contained in a sample is estimated to be mainly a saturated carbonyl compound, the absorbance at 430 nm is measured for the reaction solution, and when the carbonyl compound contained in a sample is estimated to be mainly an unsaturated carbonyl compound, the absorbance at 460 nm is measured for the reaction solution. When absorbance is measured, it is preferable that the absorption cell that contains the reaction solution is made of quartz. Also, the length (thickness) of the liquid layer specified by the absorption cell is preferably 1 cm.

It is preferable to measure the absorbance within 10 to 20 minutes after the addition of an alkali to the reaction solution resulting from the reaction of the 2,4-DNPH and the carbonyl compound in a sample. Absorbance measured before 10 minutes after the addition of an alkali sometimes lacks stability. Also, more than 20 minutes after the addition of an alkali, the absorbance tends to drop as a result of the discoloration of the reaction solution. Based on experience with various samples, if the absorbance is measured 15 minutes after the addition of an alkali, the value with best reproducibility is obtained.

In the measurement method of the present invention, the carbonyl value of a sample (a liquid organopolysiloxane or a composition comprising the same) is determined based on the absorbance measured as described above. It is possible to measure the total amount of carbonyl in the sample, by using a premeasured calibration curve based on this carbonyl value. Here, the calibration curve is obtained by measuring, according to the method (carbonyl value measurement method)described above, the carbonyl values of a plurality of standard samples whose total amount of carbonyl (propionaldehyde concentration) is known.

For example, a calibration curve is obtained by measuring the carbonyl values of standard samples, for which total amounts of carbonyl (propionaldehyde concentrations) are known, by a method that determines the carbonyl value (CV) by substituting the absorbance (A₁) and the absorbance (A₂), which are measured by the following processes (1) to (9), into the formula CV = (A₁ - A₂)/B (where B is the mass (g) of the sample contained in 5.000 g of sample solution). The same method as aforementioned, which was adopted to obtain this calibration curve, is used to measure the carbonyl value of a sample (a liquid organopolysiloxane or a composition comprising the same) for which the total amount of carbonyl is unknown. Based on this carbonyl value and the calibration curve, it is possible to measure the total amount of carbonyl in the sample. Furthermore, if a particularly precise analysis is required, it is preferable that the solvent described below for use in the following processes (1) and (9) contains an ultrapure alcohol and water, and that the solvent described below for use in the following process (7) contains an ultrapure alcohol.

[Processes]
(1) Process that prepares a sample solution by dissolving a sample in a solvent
(2) Process that adds 3 mL of an alcohol solution of 4.3% (wt/vol) trichloroacetic acid to 5.000 g of the sample solution
(3) Process that adds 5 mL of an alcohol solution (0.025% (wt/vol)) of 2,4-DNPH to a mixed solution obtained in the aforementioned process (2). Here, it is preferable to further add an appropriate amount of water.
(4) Process that reacts the carbonyl compound in a sample and 2,4-DNPH by heating the mixed solution obtained in the aforementioned process (3) for 30 minutes at 60°C
(5) Process that lets stand the reaction solution obtained in the aforementioned process (4) for 30 to 70 minutes at room temperature
(6) Process that adds 10 mL of an alcohol solution (4.0% (wt/vol)) of potassium hydroxide to a reaction solution that was left standing in the aforementioned process (5)
(7) Process that adds a solvent to said reaction solution, prepares a total of 50 mL of reaction solution, and if necessary, performs filtration of the reaction solution, 5 to 10 minutes after the aforementioned process (6)
(8) Process that measures the absorbance (A₁) at 430 nm or 460 nm, of the reaction solution obtained in the aforementioned process (7), 10 to 20 minutes after the aforementioned process (6)
(9) Process that, as a blank measurement, measures the absorbance (A₂) at 430 nm or 460 nm of a solution obtained by using 5.000 g of solvent instead of using the sample solution and performing the same operations as those in the aforementioned processes (2) to (7)

Furthermore, either the calibration curve measurements (measurements of carbonyl value of standard samples) or the measurements of carbonyl values of unknown samples can be performed before the other measurements.

Next, each process will be described below. Furthermore, the processes (2) to (7) for the preparation of the reaction solution are normally performed using a 50 mL volumetric flask.

Process (1) is a process that prepares a sample solution by dissolving a sample in a solvent containing a monovalent lower alcohol having from 1 to 4 carbon atoms. The proportion of sample in a sample solution is modified in accordance with the carbonyl value estimated for the sample. For example, the following settings are preferable: if the carbonyl value is estimated to be less than 6 in the sample, from 2 to 3 mass% (from 100 to 150 mg per 5.000 g of sample solution); if the carbonyl value is estimated to be in a range from 6 to 15 in the sample, from 0.8 to 2 mass% (from 40 to 100 mg per 5.000 g of sample solution); if the carbonyl value is estimated to be in a range from 15 to 30 in the sample, from 0.4 to 0.8 mass% (from 20 to 40 mg per 5.000 g of sample solution); if the carbonyl value is estimated to be in a range from 30 to 60 in the sample, from 0.2 to 0.4 mass% (from 10 to 20 mg per 5.000 g of sample solution); and if the carbonyl value is estimated to be greater than 60 in the sample, less than 0.2 mass% (less than 10 mg per 5.000 g of sample solution).

Additionally, when preparing a sample solution, it is preferable to dilute the sample processwise. For example, as a method of preparing 5.000 g of 2 mass% sample solution, first, 25.00 g of 8 mass% solution is prepared by dissolving 2.00 g of sample in 23.00 g of a monovalent lower alcohol having from 1 to 4 carbon atoms. Next, in a 50 mL volumetric flask,1.250 g of the 8 mass% solution and a 3.750 g of a monovalent lower alcohol having from 1 to 4 carbon atoms are accurately added to dilute the solution four-fold.

In process (2), 3 mL of an alcohol solution of 4.3% (wt/vol) trichloroacetic acid was added to 5.000 g (sample: 0.100 g) of the 2 mass% sample solution obtained in the aforementioned process (1) using a volumetric pipette or the like. The solvent (a monovalent lower alcohol having from 1 to 4 carbon atoms) of this alcohol solution is preferably an ultrapure alcohol, when particularly precise analysis is required. In this case, it is preferable to prepare the alcohol solution of trichloroacetic acid by opening a bottle containing 100 mL of ultrapure alcohol, directly adding 4.3 g of trichloroacetic acid to this bottle, covering the bottle, and then shaking the bottle to uniformly mix the contents in the bottle. Also, alcohol solution of trichloroacetic acid is preferably prepared within 24 hours before measuring the absorbance of the reaction solution.

Process (3) is a process that adds 5 mL of an alcohol solution (0.025% (wt/vol)) of 2,4-DNPH to the mixed solution obtained in the aforementioned process (2) using a volumetric pipette or the like. The solvent (a monovalent lower alcohol having from 1 to 4 carbon atoms) of this alcohol solution of 2,4-DNPH is preferably an ultrapure alcohol, when particularly precise analysis is required. In this case, the alcohol solution of 2,4-DNPH is preferably prepared by opening a bottle containing 100 mL of ultrapure alcohol, directly adding 50 mg of 2,4-DNPH (reagent-grade product containing an equal amount of water) to this bottle, covering the bottle, and then placing the bottle in an ultrasonic bath for about 5 minutes to completely dissolve the 2,4-DNPH in the bottle. In addition, the alcohol solution of 2,4-DNPH is preferably prepared within 24 hours before measuring the absorbance of the reaction solution. It is preferable to add more water to hydrolyze the precursors of the carbonyl compound of acetal and the like present in the sample, to detect the precursors as carbonyl compounds.

In process (4), the mixed solution obtained in the aforementioned process (3) is heated for 30 minutes at 60°C to react the 2,4-DNPH and the carbonyls in the sample, thereby yielding a reaction solution containing hydrazone.

In process (5), the reaction solution obtained in the process (4) is cooled by letting the reaction solution stand for 30 to 70 minutes at room temperature.

In process (6), 10 mL of alcohol solution of potassium hydroxide (4.0% (wt/vol)) was added and mixed to the reaction solution after letting it stand, using a volumetric pipette or the like. As a result, the reaction solution exhibits basicity, and the generated hydrazone becomes quinoid ions and gives color. The solvent (a monovalent lower alcohol having from 1 to 4 carbon atoms) of this alcohol solution of potassium hydroxide is preferably an ultrapure alcohol, when particularly precise analysis is required. In this case, the alcohol solution of potassium hydroxide is preferably prepared by opening a bottle containing 100 mL of ultrapure alcohol, directly adding 4.0 g of potassium hydroxide (pellet shaped reagent-grade product) to this bottle, and covering the bottle, and after shaking until the pellets disappear, this bottle is placed in an ultrasonic bath for about 5 to 10 minutes, to completely dissolve the potassium hydroxide in the bottle. Also, it is preferable to prepare the alcohol solution of potassium hydroxide within 24 hours before measuring the absorbance of the reaction solution.

In process (7), 5 to 10 minutes after the aforementioned process (6), a diluent solvent composed of a monovalent lower alcohol having from 1 to 4 carbon atoms (preferably, an ultrapure alcohol) is added to the reaction solution, to prepare a total amount of 50 mL of reaction solution (basic reaction solution). If this reaction solution is nonuniform as a result of the precipitation of neutralized salt, it is possible to obtain a uniform solution by performing additional filtration.

In process (8), for the reaction solution obtained in the aforementioned process (7), the absorbance (A₁) is measured at 430 nm (when the carbonyl in the sample is estimated to be mainly a saturated carbonyl) or at 460 nm (when the carbonyl in the sample is estimated to be mainly an unsaturated carbonyl).)The absorbance must be measured 10 to 20 minutes after addition of the alcohol solution of potassium hydroxide of the aforementioned process (6), and it is most preferable to measure the absorbance 15 minutes after the addition of the alcohol solution of potassium hydroxide.

In process (9), for blank measurement, 5.000 g of a monovalent lower alcohol having from 1 to 4 carbon atoms are used instead of the aforementioned sample solution, and for the solution obtained by performing the same operations as in the aforementioned processes (2) to (7) (i.e., addition of alcohol solution of trichloroacetic acid; addition of alcohol solution of 2,4-DNPH and addition of water; heating and cooling of the obtained mixed solution; addition of alcohol solution of potassium hydroxide; and addition of diluent solvent composed of a monovalent lower alcohol having from 1 to 4 carbon atoms), the absorbance (A<+lower>2) at 430 nm or 460 nm is measured.

It is possible to determine the carbonyl value (CV) by substituting the absorbance (A₁) obtained by the aforementioned processes (1) to (8) and the absorbance (A₂) obtained by the aforementioned process (9), respectively, into the formula: CV = (A₁ - A₂)/B.)). In the above formula, B is the mass (g) of the sample contained in 5.000 g of sample solution, and in a 2 mass% sample solution, B is 0.1 (5.000 × 0.02).

The liquid organopolysiloxane of the present invention or the composition containing the same has, in essence, a small tendency to deteriorate due to oxidation caused by the oxygen in the air. Thus, it is not necessary to add a phenol, a hydroquinone, a benzoquinone, an aromatic amine, a vitamin, or similar antioxidant in order to prevent oxidation deterioration; or take steps to increase oxidation stability. However, adding such an antioxidant, for example, BHT(2,6-di-t-butyl-p-cresol), vitamin C, vitamin E, or the like, will result in a further increase in stability. In this case, an added amount of the antioxidant that is used is in a range (by weight (mass)) from 10 to 1,000 ppm, and preferably from 50 to 500 ppm, of the liquid organopolysiloxane.

Raw material for use in an external use preparation or a cosmetic composition The liquid organopolysiloxane of the present invention or the composition containing the same can be advantageously used as a raw material for an external use preparation and a cosmetic composition for use on a human body.

A proportion of the liquid organopolysiloxane or the composition comprising the same in the raw material for an external use preparation and a cosmetic composition is preferably from 50 to 100 wt.% (mass%), more preferably from 80 to 100 wt.% (mass%), and even more preferably from 90 to 100 wt.% (mass%) based on the total weight (mass) of the raw material. A proportion of the raw material compounded in the external use preparation or the cosmetic composition is not particularly limited but, for example, can be from 0.1 to 90 wt.% (mass%), and is preferably from 1 to 80 wt.% (mass%), more preferably from 2 to 70 wt.% (mass%), and even more preferably from 5 to 50 wt.% (mass%) based on the total weight (mass) of the external use preparation or the cosmetic composition.

Examples of raw materials for use in an external use preparation and a cosmetic composition of the present invention include a tactile sensation improver, a film-forming agent, a binder, a viscosity adjuster, a surfactant, an emulsifier, or a powder dispersing agent.

### External use preparation and cosmetic composition

The liquid organopolysiloxane of the present invention or the composition comprising the same, or the raw material for use in an external use preparation and a cosmetic composition comprising the liquid organopolysiloxane or the composition comprising the same, can be advantageously compounded in an external use preparation or a cosmetic composition and can constitute the external use preparation or the cosmetic composition of the present invention. The external use preparation or the cosmetic composition of the present invention is preferably stored in a container formed from a thermoplastic material or a container formed from a non-thermoplastic material. In addition, at least one compartment can be defined in the container so as to constitute a cosmetic product unit or external use preparation unit consisting of the container and the cosmetic composition or the external use preparation according to the present invention. The external use preparation or the cosmetic composition of the present invention can be primarily applied to and used on keratinous substances such as skin or hair as a non-therapeutic beauty technique for the purpose of doing applying cosmetics (makeup) or performing care (that is, dry skin care).

The external use preparation is a product to be applied to human skin, nails, hair, and the like and, for example, medicament active components can be compounded therein and used in the treatment of various disorders. The cosmetic composition is also a product to be applied to human skin, nails, hair, and the like, and is used for beauty purposes. The external use preparation or the cosmetic composition is preferably a skin external use preparation or a skin cosmetic composition (skin care cosmetic composition, sun care cosmetic composition, antiperspirant, foundation, color cosmetic, or the like), or a hair external use preparation or a hair cosmetic composition product.

The skin external use preparation or the skin cosmetic composition product according to the present invention comprises the liquid organopolysiloxane of the present invention or the composition containing the same and, although the form thereof is not particularly limited, the product may be in the form of a solution, cream-like, solid, semi-solid, paste-like, gel-like, powder-like, multi-layer, mousse-like, or spray-like form. Specific examples of the skin external use preparation or the skin cosmetic composition product according to the present invention include skin lotions, emulsions, creams, sunscreen emulsions, sunscreen creams, hand creams, cleansing compositions, massage lotions, cleansing agents, anti-perspirants, deodorants, and similar basic cosmetic products; foundations, make-up bases, blushers, rouges, eye shadows, eye liners, mascaras, nail enamels, and similar make-up cosmetic products; and the like.

Similarly, the hair external use preparation or the hair cosmetic composition product according to the present invention comprises the liquid organopolysiloxane of the present invention or the composition containing the same and can be used in various forms. For example, the hair external use preparation or the hair cosmetic composition product according to the present invention may be dissolved or dispersed in an alcohol, a hydrocarbon, a volatile cyclic silicone, or the like and used, or the product may also be used in the form of an emulsion by dispersing the liquid organopolysiloxane of the present invention and a desired emulsifier in water. Additionally, the hair external use preparation or the hair cosmetic composition product according to the present invention can be used as a spray by using propane, butane, trichloromonofluoromethane, dichlorodifluoromethane, dichlorotetrafluoroethane, carbonic acid gas, nitrogen gas, or a similar propellant. Examples of other forms include milk-like, cream-like, solid, semi-solid, paste-like, gel-like, powder-like, multi-layer, mousse-like, and other forms. These various forms can be used as shampooing agents, rinsing agents, conditioning agents, setting lotions, hair sprays, permanent wave agents, mousses, hair colorants, and the like.

The following other components generally used in external use preparations or cosmetic compositions may be added to the external use preparation or the cosmetic composition of the present invention, provided that such components do not inhibit the effectiveness of the present invention: water, powders or coloring agents, alcohols, water-soluble polymers, film-forming agents, oil agents, oil-soluble gelling agents, organomodified clay minerals, surfactants, resins, mediums allowable in cosmetic products, adipose phases, film-forming polymers, fibers, light protection systems capable of blocking UV rays, UV absorbers, moisturizing agents, preservatives, antimicrobial agents, perfumes, salts, antioxidants, pH adjusting agents, chelating agents, refreshing agents, anti-inflammatory agents, skin beautifying components (skin-lightening agents, cell activating agents, agents for ameliorating skin roughness, circulation promoters, skin astringents, antiseborrheic agents, and the like), vitamins, amino acids, nucleic acids, hormones, clathrates, and the like; bioactive substances, medicament active ingredients, and perfumes. However, the additives are not particularly limited thereto.

The water must be free of components that are harmful to the human body and to be clean, and examples thereof include tap water, purified water, mineral water, and deep sea water. When the external use preparation or the cosmetic composition of the present invention is water-based, water soluble additives can be compounded as needed, provided that such components do not inhibit the effectiveness of the present invention. Examples of components that can be compound to constitute the aqueous phase include water soluble active materials such as vitamin Bs (described below), vitamin C and derivatives thereof, pantothenic acid and derivatives thereof, vitamins such as biotins; antiperspiration active components, water soluble UV absorbers, and various water soluble pigments, but the components are not limited thereto. In addition, a known pH adjusting agent, preservative, antimicrobial agent, or antioxidant can be compounded as needed for the purpose of improving the storage stability of the external use preparation or the cosmetic composition.

The powder and/or coloring agent can be any powder provided that it is normally used in external use preparations and cosmetic compositions, and is not limited to form (sphere, bar, needle, plate, amorphous, spindle, or the like), particle diameter (aerosol, micro-particle, pigment-grade particle, or the like), or particle structure (porous, nonporous, or the like) thereof. When compounding the powder and/or coloring agent as a pigment, preferably, one or two or more selected from an inorganic pigment powder, an organic pigment powder, and a resin powder having an average particle diameter in a range from 1 nm to 20 µm is compounded. In addition, when a pigment is used, a coated pigment is more preferable. Note that recessed fine particles formed from a silicone material, and particularly recessed fine particles with a structure that is partially spherical and hollow having an average particle size of less than 5 µm (having an arch shape or a horse hoof-shaped cross section), can also be advantageously used for the purpose of thickening the oil phase, improving tactile sensation, or the like.

Examples of the powder or coloring agent include flakes, inorganic powders, organic powders, surfactant metal salt powders (metallic soaps), colored pigments, pearl pigments, organo-modified clay minerals, and metal powder pigments. Further, a compounded products of these pigments can also be used. Specific examples of inorganic powders include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, phlogopite, lepidolite, black mica, lithia mica, silicic acid, silicic acid anhydride, aluminum silicate, sodium silicate, magnesium sodium silicate, magnesium silicate, aluminum magnesium silicate, calcium silicate, barium silicate, strontium silicate, metal salts of tungstic acid, hydroxyapatite, vermiculite, higilite, bentonite, montmorillonite, hectorite, zeolite, ceramic powder, dicalcium phosphate, alumina, aluminum hydroxide, boron nitride, and the like. Examples of organic powders include polyamide powder, polyester powder, polyethylene powder, polypropylene powder, polystyrene powder, polyurethane powder, benzoguanamine powder, polymethylbenzoguanamine powder, polytetrafluoroethylene powder, poly (methyl methacrylate) powder, cellulose, silk powder, nylon powder, nylon 12, nylon 6, silicone powder, silicone rubber spherical powder, silicone rubber spherical powder that is surface-coated with polymethylsilsesquioxane, polymethylsilsesquioxane spherical powder, copolymers of styrene and acrylic acid, copolymers of divinylbenzene and styrene, vinyl resin, urea resin, phenol resin, fluorine resin, silicone resin, acrylic resin, melamine resin, epoxy resin, polycarbonate resin, macrocrystalline fiber powder, starch powder, lauroyl lysine, and the like. Examples of surfactant metal salt powders include zinc stearate, aluminum stearate, calcium stearate, magnesium stearate, zinc myristate, magnesium myristate, zinc palmitate, zinc laurate, zinc cetylphosphate, calcium cetylphosphate, sodium zinc cetylphosphate, and the like. Examples of colored pigments include inorganic red pigments such as red iron oxide, iron oxide, iron hydroxide, iron titanate, and the like; inorganic brown pigments such as gamma-iron oxide and the like; inorganic yellow pigments such as yellow iron oxide, ocher, and the like; inorganic black iron pigments such as black iron oxide, carbon black and the like; inorganic purple pigments such as manganese violet, cobalt violet, and the like; inorganic green pigments such as chromium hydroxide, chromium oxide, cobalt oxide, cobalt titanate, and the like; inorganic blue pigments such as Prussian blue, ultramarine blue, and the like; laked pigments of tar pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207, and the like, laked pigments of natural pigments such as carminic acid, laccaic acid, carthamin, brazilin, crocin, and the like. Examples of pearl pigments include a pearl essence agent, titanium oxide-coated mica, titanium mica, iron oxide-coated titanium mica, titanium oxide-coated mica, bismuth oxychloride, titanium oxide-coated bismuth oxychloride, titanium oxide-coated talc, fish scale foil, titanium oxide-coated colored mica, and the like. Examples of the metal powder pigment include powders of metals such as aluminum, gold, silver, copper, platinum, stainless steel, and the like.

In particular, a powder that absorbs and scatters ultraviolet light, such as microparticulate titanium oxide, microparticulate iron-containing titanium oxide, microparticulate zinc oxide, microparticulate cerium oxide, compound products thereof, and the like may be used as the inorganic powder. More specifically, an inorganic ultraviolet light blocking component may be compounded as an ultraviolet light scattering agent such as the inorganic powder pigments and metal powder pigments described above, and examples thereof include metal oxides such as titanium oxide, zinc oxide, cerium oxide, titanium suboxide, and iron-doped titanium oxides; metal hydroxides such as iron hydroxides; metal flakes such as platy iron oxide and aluminum flakes; and ceramics such as silicon carbide. Of these, at least one type of a material selected from fine particulate metal oxides and fine particulate metal hydroxides with an average particle size in a range from 1 to 100 nm is particularly preferable.

Examples of the organo-modified clay mineral include dimethylbenzyl dodecylammonium montmorillonite clay, dimethyldioctadecylammonium montmorillonite clay, dimethylalkylammonium hectorite, benzyldimethylstearylammonium hectorite, distearyldimethylammonium chloride-treated aluminum magnesium silicate, and the like. Examples of commercially available products include Benton 27 (benzyldimethylstearylammonium chloride-treated hectorite, manufactured by Nationalred Co.), Benton 38 (distearyldimethylammonium chloride-treated hectorite, manufactured by Nationalred Co.), and the like.

The silicone rubber spherical powder (also known as a silicone elastomer spherical powder) preferably has a primary particle size in a range from 0.1 to 50 µm. Examples of commercially available products of the silicone rubber spherical powder include Trefil E-506S, Trefil E-508, 9701 Cosmetic Powder, 9702 Powder, EP-9215 Cosmetic Powder, EP-9261 TI Cosmetic Powder, EP-9293 AL Cosmetic Powder, EP-9289 LL Cosmetic Powder (all manufactured by Dow Coming Toray Co., Ltd.). In addition, the silicone rubber spherical powder can also be used in the external use preparation or cosmetic composition of the present invention in the form of an aqueous dispersion liquid. Examples of such commercially available aqueous dispersion liquids include BY29-129, and PF-2001 PIF Emulsion manufactured by Dow Corning Toray Co., Ltd.

Furthermore, the powder or coloring agent is preferably subjected to a water-repellent treatment. Additionally, a product can be used in which these powders or coloring agents are compounded together; or subjected to surface treatment using a general oil agent, a silicone compound other than the liquid organopolysiloxane according to the present invention, a fluorine compound, a surfactant, or the like. One type thereof or two or more types thereof can be used, as necessary.

Examples of these water-repellent treatments include various treatments in which the powder and/or coloring agent is surface treated with a water repellency agent. Specific examples thereof include organosiloxane treatments such as a methylhydrogenpolysiloxane treatment, a silicone resin treatment, a silicone gum treatment, an acryl silicone treatment, a fluorinated silicone treatment, a glycerin-modified silicone treatment, and the like; metallic soap treatments such as a zinc stearate treatment and the like; silane treatments such as a silane coupling agent treatment, an alkylsilane treatment, and the like; fluorine compound treatments such as a perfluoroalkylsilane treatment, a perfluoroalkyl phosphate treatment, a perfluoro polyether treatment, and the like; amino acid treatments such as an N-lauroyl-L-lysine treatment and the like; oil agent treatments such as a squalane treatment and the like; and acryl treatments such as an alkyl acrylate treatment and the like. A combination of one or more of the treatments described above can be used.

Particularly preferable examples of these powders or coloring agents include at least one type of powder or coloring agent selected from the group consisting of a silicone resin powder, a silicone rubber powder, an organic resin powder (excluding silicone resin powders), an organo-modified clay mineral, titanium oxide, zinc oxide, a titanated mica, a metal soap, an inorganic body pigment, an inorganic coloration pigment and a coated pigment.

Examples of alcohols include at least one type selected from a lower alcohol, a sugar alcohol, and a higher alcohol. Specific examples include lower alcohols such as ethanol and isopropanol; sugar alcohols such as sorbitol and maltose; and higher alcohols such as lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, sitosterol, phytosterol, lanosterol, POE cholesterol ether, monostearyl glycerol ether (batyl alcohol), and monooleyl glycerol ether (selachyl alcohol).

The water-soluble polymer can be compounded for the purpose of enhancing sensation during use of the external use preparation or the cosmetic composition or as a water soluble moisturizing agent or film-forming polymer. Any of amphoteric, cationic, anionic, and nonionic polymers, and water-swellable clay minerals can be used, provided that the water-soluble polymer is one that is commonly used in external use preparations or cosmetic products, and it is possible to use one or two or more types of these water-soluble polymers. These water-soluble polymers have an effect of thickening the hydrous component, so the polymers are particularly useful for stabilizing the system when obtaining a gel-like hydrous external use preparation or cosmetic composition, a water-in-oil emulsion external use preparation or cosmetic composition, and an oil-in-water emulsion external use preparation or cosmetic composition.

Examples of amphoteric water-soluble polymers include amphoteric starches, dimethyldiallylammonium chloride derivatives (for example, acrylamide-acrylic acid-dimethyldiallylammonium chloride copolymers and acrylic acid-dimethyldiallylammonium chloride copolymers), and methacrylic acid derivatives (for example, polymethacryloylethyldimethylbetaines, N-methacryloyloxyethyl-N,N-dimethylammonium-α-methylcarboxybetaine-alkyl methacrylate copolymers, and the like).

Examples of cationic water-soluble polymers include quaternary nitrogen-modified polysaccharides (for example, cation-modified cellulose, cation-modified hydroxyethylcellulose, cation-modified guar gum, cation-modified locust bean gum, cation-modified starch, and the like); dimethyldiallylammonium chloride derivatives (for example, copolymers of dimethyldiallylammonium chloride and acrylamide, poly(dimethylmethylene piperidinium chloride), and the like); vinylpyrrolidone derivatives (for example, copolymers of vinylpyrrolidone and dimethylaminoethyl methacrylic acid, copolymers of vinylpyrrolidone and methacrylamide propyltrimethylammonium chloride, copolymers of vinylpyrrolidone and methylvinylimidazolium chloride, and the like); and methacrylic acid derivatives (for example, methacryloylethyldimethylbetaine-methacryloylethyltrimethyl ammonium chloride-2-hydroxyethyl methacrylate copolymers, methacryloylethyldimethylbetaine-methacryloylethyltrimethyl ammonium chloride-methoxy polyethylene glycol methacrylate copolymers, and the like).

Examples of anionic water-soluble polymers include poly(acrylic acid) and alkali metal salts thereof, poly(methacrylic acid) and alkali metal salts thereof, hyaluronic acid and alkali metal salts thereof, acetylated hyaluronic acid and alkali metal salts thereof, water-soluble polymers of aliphatic carboxylic acids or metal salts thereof, such as hydrolysates of methyl vinyl ether-maleic anhydride copolymers, carboxymethyl cellulose and alkali metal salts thereof, methyl vinyl ether-maleic acid half ester copolymers, alkanolamide solutions of acrylic resins, carboxyvinyl polymers, 2-acrylamide-2-methylpropane sulfonate polymers.

Examples of nonionic water-soluble polymers include poly(vinyl pyrrolidone), highly polymerized polyethylene glycols, PEG/PPG-36/41 dimethyl ethers, PEG/PPG-14/7 dimethyl ethers, vinyl pyrrolidone-vinyl acetate copolymers, vinyl pyrrolidone-dimethylaminoethyl methacrylate copolymers, vinyl caprolactam-vinyl pyrrolidone-dimethylaminoethyl methacrylate copolymers, cellulose and derivatives thereof (for example, methyl cellulose, ethyl cellulose, hydroxyethyl cellulose, hydroxypropyl cellulose, and carboxymethyl cellulose), keratin and collagen and derivatives thereof, calcium alginate, pullulan, agar, gelatin, tamarind seed polysaccharides, xanthan gum, carrageenan, high-methoxylpectin, low-methoxylpectin, guar gum, pectin, gum arabic, crystalline cellulose, arabinogalactan, karaya gum, gum tragacanth, alginic acid, albumin, casein, curdlan, gellan gum, dextran, pyrus cydonia seed gum, gum tragacanth, chitin/chitosan derivatives, starches (rice, corn, potato, wheat and the like), keratin and collagen and derivatives thereof, and similar natural polymer compounds.

The water-swellable clay mineral is an inorganic water-soluble polymer and is a type of colloid-containing aluminum silicate having a three-layer structure. Typical examples thereof are expressed by the following formula (A).

(X,Y)₂₋₃(Si,Al)₄O₁₀(OH)₂Z_{1/3}·nH₂O (A)

(Wherein X is Al, Fe(III), Mn(III), or Cr(III), Y is Mg, Fe(II), Ni, Zn, or Li, and Z is K, Na, or Ca). Specific examples of such inorganic water-soluble polymers include bentonite, montmorillonite, beidellite, nontronite, saponite, hectorite, magnesium aluminum silicate, and silicic anhydride, and these may be natural or synthetic products.

An oil agent described above can be used, and one type or two or more types thereof can be used as necessary.

Examples of the oil-soluble gelling agent include metal soaps such as aluminum stearate, magnesium stearate, and zinc myristate; amino acid derivatives such as N-lauroyl-L-glutamic acid, and α,γ-di-n-butylamine; dextrin fatty acid esters such as dextrin palmitate esters, dextrin stearate esters, and dextrin 2-ethylhexanoate palmitate esters; sucrose fatty acid esters such as sucrose palmitate esters and sucrose stearate esters; fructooligosaccharide fatty acid esters such as inulin stearate esters and fructooligosaccharide 2-ethylhexanoate esters; semicrystalline homopolymers or copolymers obtained by the polymerization of a monomer comprising a long chain alkyl acrylate and/or a long chain alkyl methacrylate having from 14 to 24 carbon atoms or the like; benzylidene derivatves of sorbitol such as monobenzylidene sorbitol and dibenzylidene sorbitol; and organo-modified clay materials such as dimethylbenzyl dodecylammonium montmorillonite clay and dimethyldioctadecylammonium montmorillonite clay. One type or two or more types thereof can be used as necessary.

Surfactants other than the components described above can be compounded in the external use preparation or the cosmetic composition of the present invention. In particular, one type or two or more types of surfactants selected from the group consisting of a silicone-based surfactant other than the liquid organopolysiloxane of the present invention, an anionic surfactant, a cationic surfactant, a nonionic surfactant, an amphoteric surfactant, and a semipolar surfactant can be used in combination as the surfactant.

The silicone-based surfactant is a silicone-based surfactant other than the liquid organopolysiloxane, which is a sugar alcohol-modified silicone, according to the present invention. This silicone-based surfactant is often used as a component for the emulsification or washing of an oil agent, and the dispersion or surface treatment of a powder, and representative examples include polyglyceryl-modified silicones, glyceryl-modified silicones, sugar-modified silicones, fluoropolyether-modified silicones, polyether-modified silicones, carboxylic acid-modified silicones, sugar-modified silicones, block copolymers of straight-chain silicones and polyethers (polysilicone-13 and the like), and long-chain alkyl-polyether comodified silicones. Since the liquid organopolysiloxane according to the present invention has a structure comprising a hydrophilic portion and a hydrophobic portion, the liquid organopolysiloxane functions as a powder dispersing agent. Thus, in cases where used in combination with a silicone-based nonionic surfactant, the liquid organopolysiloxane also functions as an aid to enhance the compounding stability of the nonionic surfactant and may improve overall stability of the formulation. In particular, the liquid organopolysiloxane according to the present invention can be advantageously used in combination with a glycerin derivative-modified silicone, a sugar-modified silicone, a sugar alcohol-modified silicone, a carboxylic acid-modified silicone, and a polyglycerin-modified silicone elastomer (otherwise known as a polyglycerated silicone elastomer), and a silicone-based nonionic surfactant in which alkyl branching, straight-chain silicone branching, siloxane dendrimer branching or the like is performed along with the hydrophilic group as necessary can be advantageously used. Note that it is also possible to use the substance in combination with a polyoxyalkylene-modified silicone (a polyether-modified silicone, a fluorine polyether-modified silicone, or the like), a polyether-modified silicone elastomer (otherwise known as a polyoxyalkylated silicone elastomer), or an organopolyoxyalkylene group-containing surfactant.

Examples of the anionic surfactants include those in which carboxylic acid-modified silicone is neutralized using an alkaline substance; saturated or unsaturated fatty acid salts (for example, sodium laurate, sodium stearate, sodium oleate, sodium linolenate, and the like); alkylsulfuric acid salts; alkylbenzene sulfonic acids (for example, hexylbenzenesulfonic acid, octylbenzenesulfonic acid, dodecylbenzenesulfonic acid, and the like) and salts thereof; polyoxyalkylene alkyl ether sulfuric acid salts; polyoxyalkylene alkenyl ether sulfuric acid salts; polyoxyethylene alkylsulfuric ester salts; sulfosuccinic acid alkyl ester salts; polyoxyalkylene sulfosuccinic acid alkyl ester salts; polyoxyalkylene alkylphenyl ether sulfuric acid salts; alkanesulfonic acid salts; octyltrimethylammonium hydroxide; dodecyltrimethylammonium hydroxide; alkyl sulfonates; polyoxyethylene alkylphenyl ether sulfuric acid salts; polyoxyalkylene alkyl ether acetic acid salts; alkyl phosphoric acid salts; polyoxyalkylene alkyl ether phosphoric acid salts; acylglutamic acid salts; α-acylsulfonic acid salts; alkylsulfonic acid salts; alkylallylsulfonic acid salts; α-olefinsulfonic acid salts; alkylnaphthalene sulfonic acid salts; alkanesulfonic acid salts; alkyl- or alkenylsulfuric acid salts; alkylamide sulfuric acid salts; alkyl- or alkenyl phosphoric acid salts; alkylamide phosphoric acid salts; alkyloylalkyl taurine salts; N-acylamino acid salts; sulfosuccinic acid salts; alkyl ether carboxylic acid salts; amide ether carboxylic acid salts; α-sulfofatty acid ester salts; alanine derivatives; glycine derivatives; and arginine derivatives. Examples of salts include alkali metal salts such as sodium salts and the like, alkaline earth metal salts such as magnesium salts and the like, alkanolamine salts such as triethanolamine salts and the like, and ammonium salts.

Examples of cationic surfactants include alkyltrimethylammonium chloride, stearyltrimethylammonium chloride, lauryltrimethylammonium chloride, cetyltrimethylammonium chloride, beef tallow alkyltrimethylammonium chloride, behenyltrimethylammonium chloride, stearyltrimethylammonium bromide, behenyltrimethylammonium bromide, distearyldimethylammonium chloride, dicocoyldimethylammonium chloride, dioctyldimethylammonium chloride, di(POE)oleylmethylammonium (2 EO) chloride, benzalkonium chloride, alkyl benzalkonium chloride, alkyl dimethylbenzalkonium chloride, benzethonium chloride, stearyl dimethylbenzylammonium chloride, lanolin derivative quaternary ammonium salt, diethylaminoethylamide stearate, dimethylaminopropylamide stearate, behenic acid amide propyldimethyl hydroxypropylammonium chloride, stearoyl colaminoformyl methylpyridinium chloride, cetylpyridinium chloride, tall oil alkylbenzyl hydroxyethylimidazolinium chloride, and benzylammonium salt.

Examples of nonionic surfactants include polyglyceryl diisostearate and polyhydroxy diglyceryl stearate, isostearyl glyceryl ethers, polyoxyalkylene ethers, polyoxyalkylene alkyl ethers, polyoxyalkylene fatty acid esters, polyoxyalkylene fatty acid diesters, polyoxyalkylene resin acid esters, polyoxyalkylene (cured) castor oils, polyoxyalkylene alkyl phenols, polyoxyalkylene alkyl phenyl ethers, polyoxyalkylene phenyl phenyl ethers, polyoxyalkylene alkyl esters, polyoxyalkylene alkyl esters, sorbitan fatty acid esters, polyoxyalkylene sorbitan alkyl esters, polyoxyalkylene sorbitan fatty acid esters, polyoxyalkylene sorbitol fatty acid esters, polyoxyalkylene glycerol fatty acid esters, polyglycerol alkyl ethers, polyglycerol fatty acid esters, sucrose fatty acid esters, fatty acid alkanolamides, alkylglucosides, polyoxyalkylene fatty acid bisphenyl ethers, polypropylene glycol, diethyleneglycol, polyoxyethylene-polyoxypropylene block polymers, alkylpolyoxyethylene-polyoxypropylene block polymer ethers, polyoxyethylene-polyoxypropylene block polymers, alkylpolyoxyethylene-polyoxypropylene block polymer ethers, and fluorine-based surfactants.

Examples of amphoteric surfactants include imidazoline-type, amidobetaine-type, alkylbetaine-type, alkylamidobetaine-type, alkylsulfobetaine-type, amidosulfobetaine-type, hydroxysulfobetaine-type, carbobetaine-type, phosphobetaine-type, aminocarboxylic acid-type, and amidoamino acid-type amphoteric surfactants. Specifically, examples include imidazoline-type amphoteric surfactants such as 2-undecyl-N,N,N-(hydroxyethylcarboxymethyl)-2-imidazoline sodium, 2-cocoyl-2-imidazolinium hydroxide-1-carboxyethyloxy disodium salt, and the like; alkylbetaine-type amphoteric surfactants such as lauryl dimethylaminoacetic betaine, myristyl betaine, and the like; amidobetaine-type amphoteric surfactants such as coconut oil fatty acid amidopropyl dimethylamino acetic acid betaine, palm kernel oil fatty acid amidopropyl dimethylamino acetic acid betaine, beef tallow fatty acid amidopropyl dimethylamino acetic acid betaine, hardened beef tallow fatty acid amidopropyl dimethylamino acetic acid betaine, lauric acid amidopropyl dimethylamino acetic acid betaine, myristic acid amidopropyl dimethylamino acetic acid betaine, palmitic acid amidopropyl dimethylamino acetic acid betaine, stearic acid amidopropyl dimethylamino acetic acid betaine, oleic acid amidopropyl dimethylamino acetic acid betaine, and the like; alkylsulfobetaine-type amphoteric surfactants such as coconut oil fatty acid dimethyl sulfopropyl betaine and the like; alkyl hydroxy sulfobetaine-type amphoteric surfactants such as lauryl dimethylaminohydroxy sulfobetaine and the like; phosphobetaine-type amphoteric surfactants such as laurylhydroxy phosphobetaine and the like; and amidoamino acid-type amphoteric surfactants such as sodium N-lauroyl-N'-hydroxyethyl-N'-carboxymethyl ethylenediamine, sodium N-oleoyl-N'-hydroxyethyl-N'-carboxymethyl ethylenediamine, sodium N-cocoyl-N'-hydroxyethyl-N'-carboxymethyl ethylenediamine, potassium N-lauroyl-N'-hydroxyethyl-N'-carboxymethyl ethylenediamine, potassium N-oleoyl-N'-hydroxyethyl-N'-carboxymethyl ethylenediamine, sodium N-lauroyl-N-hydroxyethyl-N'-carboxymethyl ethylenediamine, sodium N-oleoyl-N-hydroxyethyl-N'-carboxymethyl ethylenediamine, sodium N-cocoyl-N-hydroxyethyl-N'-carboxymethyl ethylenediamine, monosodium N-lauroyl-N-hydroxyethyl-N',N'-dicarboxymethyl ethylenediamine, monosodium N-oleoyl-N-hydroxyethyl-N',N'-dicarboxymethyl ethylenediamine, monosodium N-cocoyl-N-hydroxyethyl-N',N'-dicarboxymethyl ethylenediamine, disodium N-lauroyl-N-hydroxyethyl-N',N'-dicarboxymethyl ethylenediamine, disodium N-oleoyl-N-hydroxyethyl-N',N'-dicarboxymethyl ethylenediamine, disodium N-cocoyl-N-hydroxyethyl-N',N'-dicarboxymethyl ethylenediamine, and the like.

Examples of semipolar surfactants include alkylamine oxide-type surfactants, alkylamine oxides, alkylamide amine oxides, alkylhydroxyamine oxides, and the like. Alkyldimethylamine oxides having from 10 to 18 carbon atoms, alkoxyethyl dihydroxyethylamine oxides having from 8 to 18 carbon atoms, and the like are preferably used. Specific examples thereof include dodecyldimethylamine oxide, dimethyloctylamine oxide, diethyldecylamine oxide, bis-(2-hydroxyethyl)dodecylamine oxide, dipropyltetradecylamine oxide, methylethylhexadecylamine oxide, dodecylamidopropyldimethylamine oxide, cetyldimethylamine oxide, stearyldimethylamine oxide, tallow dimethylamine oxide, dimethyl-2-hydroxyoctadecylamine oxide, lauryldimethylamine oxide, myristyldimethylamine oxide, stearyldimethylamine oxide, isostearyldimethylamine oxide, coconut fatty acid alkyldimethylamine oxide, caprylic amide propyldimethylamine oxide, capric amide propyldimethylamine oxide, lauric amide propyldimethylamine oxide, myristic amide propyldimethylamine oxide, palmitic amide propyldimethylamine oxide, stearic amide propyldimethylamine oxide, isostearic amide propyldimethylamine oxide, oleic amide propyldimethylamine oxide, ricinoleic amide propyldimethylamine oxide, 12-hydroxystearic amide propyldimethylamine oxide, coconut fatty acid amide propyldimethylamine oxide, palm kernel oil fatty acid amide propyldimethylamine oxide, castor oil fatty acid amide propyldimethylamine oxide, lauric amide ethyldimethylamine oxide, myristic amide ethyldimethylamine oxide, coconut fatty acid amide ethyldimethylamine oxide, lauric amide ethyldiethylamine oxide, myristic amide ethyldiethylamine oxide, coconut fatty acid amide ethyldiethylamine oxide, lauric amide ethyldihydroxyethylamine oxide, myristic amide ethyldihydroxyethylamine oxide, and coconut fatty acid amide ethyldihydroxyethylamine oxide.

The ultraviolet light blocking agent can be an inorganic ultraviolet light blocking agent or an organic ultraviolet light blocking agent. When the external use preparation or the cosmetic composition of the present invention is to be used for sunblocking, it is preferable for the composition to contain at least one type of organic ultraviolet light blocking agent. In particular, using both inorganic and organic ultraviolet light blocking agents in combination is preferable, and using a UV-A blocking agent in combination with a UV-B blocking agent is more preferable.

The inorganic ultraviolet light blocking agent may be compounded as an ultraviolet light scattering agent such as the inorganic pigment powders and metal powder pigments mentioned above. Examples thereof include metal oxides such as titanium oxide, zinc oxide, cerium oxide, titanium suboxide, iron-doped titanium oxides, and the like; metal hydroxides such as iron hydroxides and the like; metal flakes such as platy iron oxide, aluminum flake, and the like; and ceramics such as silicon carbide, and the like. Of these, at least one type of a material selected from fine particulate metal oxides and fine particulate metal hydroxides with an average particle diameter in a range from 1 to 100 nm and a particulate, plate-like, needle-like, or fiber form is preferable. These powders are preferably subjected to conventionally known surface treatment such as, for example, fluorine compound treatment (of which perfluoroalkyl phosphate treatment, perfluoroalkylsilane treatment, perfluoropolyether treatment, fluorosilicone treatment, and fluorinated silicone resin treatment are preferable), silicone treatment (of which methylhydrogen polysiloxane treatment, dimethylpolysiloxane treatment, and vapor-phase tetramethyltetrahydrogen cyclotetrasiloxane treatment are preferable), silicone resin treatment (of which trimethylsiloxysilicic acid treatment is preferable), pendant treatment (which is a method of adding alkyl chains after vapor-phase silicone treatment), silane coupling agent treatment, titanium coupling agent treatment, silane treatment (of which alkylsilane or alkylsilazane treatment is preferable), oil agent treatment, N-acylated lysine treatment, polyacrylic acid treatment, metal soap treatment (of which a stearic acid or myristic acid salt is preferable), acrylic resin treatment, metal oxide treatment, or the like, and the powders are preferably treated with a plurality of these treatments. For example, the surface of the fine particulate titanium oxide can be coated with a metal oxide such as silicon oxide and alumina, and, thereafter, surface treating using an alkylsilane can be carried out. A total amount of the surface treatment agent is preferably in a range from 0.1 to 50% by mass of the powder.

The organic ultraviolet light blocking agent is a lipophilic ultraviolet light blocking agent, and examples thereof include benzoic acid-based UV absorbers such as paraaminobenzoic acid (hereinafter, abbreviated as "PABA"), PABA monoglycerol ester, N,N-dipropoxy-PABA ethyl ester, N,N-diethoxy-PABA ethyl ester, N,N-dimethyl-PABA ethyl ester, N,N-dimethyl-PABA butyl ester, and 2-[4-(diethylamino)-2-hydroxybenzoyl]hexylester benzoate (trade name: Uvinul A plus); anthranilic acid-based UV absorbers such as homomenthyl-N-acetylanthranilate; salicylic acid-based UV absorbers such as amyl salicylate, menthyl salicylate, homomenthyl salicylate, octyl salicylate, phenyl salicylate, benzyl salicylate, and p-isopropanolphenyl salicylate; cinnamic acid-based UV absorbers such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxy cinnamate, ethyl-α-cyano-β-phenylcinnamate, 2-ethylhexyl-α-cyano-β-phenylcinnamate, glyceryl mono-2-ethylhexanoyl-diparamethoxycinnamate, 3-methyl-4-[methylbis(trimethylsiloxy) silyl]butyl 3,4,5-trimethoxycinnamate, and dimethicodiethyl benzalmalonate (trade name: Parsol SLX (INCI name: Polysilicone-15)); benzophenone-based UV absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, hydroxy-4-n-octoxybenzophenone, and 4-hydroxy-3-carboxybenzophenone; benzotriazole-based UV absorbers such as 3-(4'-methylbenzylidene)-d,1-camphor, 3-benzylidene-d,l-camphor, urocanic acid, urocanic acid ethyl ester, 2-phenyl-5-methylbenzoxazole, 2,2'-hydroxy-5-methylphenyl benzotriazole, 2-(2'-hydroxy-5'-t-octylphenyl)benzotriazole, 2-(2'-hydroxy-5'-methylphenyl)benzotriazole, dibenzaladine, dianisoylmethane, 4-methoxy-4'-t-butyldibenzoylmethane, 5-(3,3-dimethyl-2-norbornylidene)-3-pentan-2-one, 2,2'-methylene bis(6-(2H-benzotriazole-2-yl)-4-(1,1,3,3-tetramethylbutyl)phenol) (trade name: Tinosorb® M), 2-[4-(2-ethylhexyloxy)-2-hydroxyphenyl]-2H-benzotriazole, and 2-(2-hydroxy-4-isobutoxyphenyl)-2H-benzotriazole; triazine-based UV absorbers such as 2,4,6-tris[4-(2-ethylhexyloxycarbonyl)anilino]1 ,3,5-triazine (INCI: octyltriazone) and 2,4-bis-6-(4-methoxyphenyl)-1,3,5-triazine (INCI: bis-ethylhexyloxyphenol methoxyphenyl triazine, trade name: Tinosorb® S); and 2-cyano-3,3-diphenylprop-2-enoate-2-ethylhexyl ester (INCI: octocrylene).

Generally, the organic-based UV absorber has high polarity and does not readily dissolve. Therefore, conventionally, it has been difficult to stably compound a desired (high) amount of the organic-based UV absorber in water-in-oil (W/O) emulsion cosmetic compositions. However, when the liquid organopolysiloxane of the present invention is used as an emulsifier and a medium polarity oil such as an ester oil or the like is combined therewith as a binding agent, a stable UV absorber-containing W/O emulsion cosmetic composition can be obtained even when the oil phase contains a low polarity oil such as a silicone oil, a hydrocarbon oil, or the like. At this time, it is preferable to use a xylitol-modified silicone having a siloxane dendron structure and a long-chain alkyl group or a glycerin-modified silicone having a siloxane dendron structure and a long-chain alkyl group as a second emulsifying agent and to use this agent in combination with the liquid organopolysiloxane of the present invention. In this case, the compounded amount of the organic-based UV absorber is preferably in a range of 0.1 to 10 mass% and the compounded amount of the binding agent is preferably in a range of 0.005 to 5 mass%.

Additionally, it is possible to use a product in which the organo-ultraviolet light blocking agent is comprised in a hydrophobic polymer powder. The polymer powder may be hollow, and preferably has an average primary particle size in a range from 0.1 to 50 µm. Particle size distribution may be broad or sharp. Types of polymer include acrylic resins, methacrylic resins, styrene resins, polyurethane resins, polyethylene, polypropylene, polyethylene terephthalate, silicone resins, nylons, acrylamide resins, and silylated polypeptide resins. A polymer powder comprising from 0.1 to 30 mass% of an organo-ultraviolet light blocking agent is preferable, and a polymer powder comprising 4-tert-butyl-4'-methoxydibenzoylmethane, a UV-A absorber, is particularly preferable.

The ultraviolet light blocking agent that can be preferably used is at least one selected from the group consisting of fine particulate titanium oxide, fine particle zinc oxide, paramethoxy cinnamic acid 2-ethylhexyl, 4-tert-butyl-4'-methoxydibenzoylmethane, a benzotriazole-based UV absorber, and a triazine-based UV absorber. These ultraviolet light blocking agents are generally used, are easy to acquire, and have high ultraviolet light blocking effects and, thus can be beneficially used.

Examples of salts include inorganic salts, organic salts, amine salts, and amino acid salts. Examples of inorganic salts include sodium salts, potassium salts, magnesium salts, calcium salts, aluminum salts, zirconium salts, and zinc salts of inorganic acids such as hydrochloric acid, sulfuric acid, carbonic acid, and nitric acid. Examples of organic acid salts include salts of organic acids such as acetic acid, dehydroacetic acid, citric acid, malic acid, succinic acid, ascorbic acid, and stearic acid. Examples of amine salts and amino acid salts include salts of amines such as triethanolamine and salts of amino acids such as glutamic acid. In addition, salts of hyaluronic acid, chondroitin sulfuric acid, and the like, aluminum zirconium glycine complexes and the like, and acid-alkali neutralization salts and the like used in cosmetic product formulations can be used.

Examples of moisturizing agents include polyhydric alcohols such as glycerin, sorbitol, propylene glycol, dipropylene glycol, 1,3-butylene glycol, glucose, xylitol, maltitol, and polyethyleneglycol; hyaluronic acid, chondroitin sulfuric acid, pyrrolidone carboxylic acid salts, polyoxyethylene methylglucoside, polyoxypropylene methylglucoside, PEG/PPG dimethylether, polyols, glycols, glycol esters, and the like.

Examples of preservatives include alkyl paraoxybenzoates, benzoic acid, sodium benzoate, sorbic acid, potassium sorbate, phenoxyethanol, and the like. Examples of antimicrobial agents include benzoic acid, salicylic acid, carbolic acid, sorbic acid, alkyl paraoxybenzoates, parachloromethacresol, hexachlorophene, benzalkonium chloride, chlorhexidine chloride, trichlorocarbanilide, triclosan, photosensitizers, phenoxy ethanol and the like. However, in cases where the cosmetic composition is a rouge, it is preferable that these are not included.

Examples of the antioxidants include tocopherol, butylhydroxyanisole, dibutylhydroxytoluene, phytic acid, and the like.

Examples of pH adjusting agents include lactic acid, citric acid, glycolic acid, succinic acid, tartaric acid, dl-malic acid, potassium carbonate, sodium hydrogen carbonate, ammonium hydrogen carbonate, and the like.

Examples of the chelating agent include alanine, sodium salt of edetic acid, sodium polyphosphate, sodium metaphosphate, phosphoric acid, and the like.

Examples of the refreshing agents include L-menthol and camphor, and examples of the anti-inflammatory agents include allantoin, glycyrrhetic acid, glycyrrhizinic acid, tranexamic acid, and azulene.

Moreover, examples of skin beautifying components include skin-lightening agents such as placenta extracts, arbutin, glutathione, saxifrageous extracts, and the like; cell activating agents such as royal jelly and the like; agents for ameliorating skin roughness; circulation promoters such as nonylic acid vanillylamide, benzyl nicotinate, beta-butoxyethyl nicotinate, capsaicin, zingerone, cantharide tincture, ichthammol, caffeine, tannic acid, α-borneol, tocopherol nicotinate, inositol hexanicotinate, cyclandelate, cinnarizine, tolazoline, acetylcholine, verapamil, cepharanthine, γ-oryzanol, and the like; astringents such as zinc oxide, tannic acid, and the like; antiseborrheic agents such as sulfur, thianthol, and the like; and the like. Examples of vitamins include vitamin As such as vitamin A oil, retinol, retinol acetate, retinol palmitate, and the like; vitamin Bs such as vitamin B2s such as riboflavin, riboflavin butyrate, flavin adenine dinucleotide, and the like; vitamin B6s such as pyridoxine hydrochloride, pyridoxine dioctanoate, pyridoxine tripalmitate, and the like; vitamin B12 and derivatives thereof; vitamin B15 and derivatives thereof, and the like; vitamin Cs such as L-ascorbic acid, L-ascorbyl dipalmitic acid esters, sodium L-ascorbyl 2-sulfate, dipotassium L-ascorbyl phosphoric acid diester, and the like; vitamin Ds such as ergocalciferol, cholecalciferol, and the like; vitamin Es such as α-tocopherol, β-tocopherol, γ-tocopherol, dl-α-tocopherol acetate, dl-α-tocopherol nicotinate, dl-α-tocopherol succinate, and the like; vitamin H; vitamin P; nicotinic acids such as nicotinic acid, benzyl nicotinate, and the like; pantothenic acids such as calcium pantothenate, D-pantothenyl alcohol, pantothenyl ethyl ether, acetyl pantothenyl ethyl ether, and the like; and the like.

Examples of amino acids include amino acids such as glycine, valine, leucine, isoleucine, serine, threonine, phenylalanine, arginine, lysine, aspartic acid, glutamic acid, cysteine, cysteine, methionine, tryptophan, and the like, and/or salts thereof.

Examples of nucleic acids include deoxyribonucleic acid, and examples of hormones include estradiol and ethenyl estradiol.

The bioactive component is a substance that imparts some sort of bioactivity to the skin or the hair when applied to the skin or the hair, and examples thereof are lipophilic substances. Examples thereof include anti-inflammatory agents, anti-aging agents, tightening agents, hair regrowth agents, hair growth promoters, moisturizing agents, circulation promoters, drying agents, warming agents, vitamins, wound healing accelerators, irritation mitigation agents, analgesics, cell activating agents, enzyme components, and the like. Similarly, natural vegetable extract components, seaweed extract components and herbal medicine components can be preferably blended.

The medicament active component is a substance having a disease-treating effect, and examples thereof include proteins, peptides, and low molecular weight compounds.

The perfume is not particularly limited as long as it is a lipophilic perfume, and examples thereof include perfumes that are extracted from a variety of plant flowers, seeds, leaves, roots, and the like, perfumes extracted from seaweed, perfumes extracted from a variety of animal parts and secretions (for example, musk or sperm oil), and artificially synthesized perfumes (for example, menthol, musk, acetic acid esters, and vanilla). The perfume is compounded for the purpose of imparting a fragrance or scent to the external use preparation or the cosmetic composition. Examples of the pigment include oil-soluble dyes, water-soluble dyes, extender pigments, inorganic pigments, organic pigments, and lipophilic optical brighteners.

[Combinations with other silicone-based cosmetic raw materials] Depending on the dosage form and formulation thereof, the external use preparation or the cosmetic composition according to the present invention may further contain a solid silicone resin or crosslinking organopolysiloxane, an acryl silicone dendrimer copolymer, a silicone raw rubber (silicone gum), a polyamide-modified silicone, an alkyl-modified silicone wax, or an alkyl-modified silicone resin wax. The main chain of the liquid organopolysiloxane of the present invention comprises a polysiloxane chain, and the liquid organopolysiloxane has a hydrophilic glycerin derivative group as a modifying group. This is advantageous in that it is possible to design a cosmetic composition that has excellent compounding stability with these silicone-based compounds and that takes advantage of the tactile sensation that is characteristic to these silicone-based cosmetic raw materials.

### Solid silicone resin or crosslinking organopolysiloxane

The external use preparation or the cosmetic composition of the present invention may further contain a solid silicone resin or crosslinking organopolysiloxane (excluding the liquid organopolysiloxane of the present invention). The solid silicone resin or crosslinking organopolysiloxane preferably is hydrophobic, having absolutely no solubility in water at room temperature, or a solubility of less than 1 wt.% (mass%) per 100 g of water.

The solid silicone resin is an organopolysiloxane having a highly branched structure, a net-like structure, or a cage structure, and is solid at room temperature. Any type of product may be used, provided that it is a silicone resin that is commonly used in cosmetic compositions and does not oppose the object of the present invention. The solid silicone resin may be a spherical powder, a flaky powder, a needle like powder, a plate-like flaky powder (including plate-like powders having appearances and particle aspect ratios commonly identified with plate-like forms), or a similar particulate. Particularly, a silicone resin powder having monoorganosiloxy units (T units) and/or siloxy units (Q units), described below, can be preferably used.

Compounding the liquid organopolysiloxane of the present invention along with the solid silicone resin is useful because the compatibility with the oil agent and uniform dispersibility are improved, improvement effects in sensation during use can be obtained, namely uniform adhesion to the applied area due to the compounding of the solid silicone resin, and improvement effects in cosmetic retainability such as moisture resistance and sebum resistance can be obtained. Note that solid silicone resins are often used as film-forming polymers that can be compounded in an oil-based system.

Examples of the solid silicone resin include MQ resins, MDQ resins, MTQ resins, MDTQ resins, TD resins, TQ resins, and TDQ resins formed from arbitrary combinations of triorganosiloxy units (M units) (where the organo groups are only methyl groups, or are methyl groups and vinyl groups or phenyl groups), diorganosiloxy units (D units) (where the organo groups are only methyl groups, or are methyl groups and vinyl groups or phenyl groups), monoorganosiloxy units (T units) (where the organo groups are methyl groups, vinyl groups, or phenyl groups), and siloxy units (Q units). Furthermore, other examples include trimethylsiloxysilicate, polyalkylsiloxysilicate, dimethylsiloxy unit-containing trimethylsiloxysilicate, and alkyl(perfluoroalkyl)siloxysilicate. These silicone resins are preferably oil soluble and can be dissolved in volatile silicone.

In particular, a phenyl silicone resin having a high refractive index and a high content of phenyl groups (for example, 217 Flake resin or the like manufactured by Dow Corning Toray Co., Ltd.) can be easily used as a flaky silicone resin powder. In particular, when compounded in a cosmetic composition, the phenyl silicone resin can impart a radiant feeling of sheerness to the skin or the hair.

Types of crosslinking organopolysiloxanes (excluding the organopolysiloxane elastomer of the present invention) include non-emulsifiable crosslinking organopolysiloxanes and emulsifiable crosslinking organopolysiloxanes, but the organopolysiloxane chain preferably has a structure which is three-dimensionally crosslinked by a reaction between a crosslinking component consisting of a polyether unit, an alkylene unit having from 4 to 20 carbon atoms, or an organopolysiloxane unit and other optional modifying agents or the like.

Specifically, the crosslinking organopolysiloxane (excluding the liquid organopolysiloxane of the present invention) can be obtained by performing an addition reaction on an organohydrogenpolysiloxane having a silicon-bonded hydrogen atom, a polyether compound or glycerin derivative having reactive unsaturated bonds at both terminals of the molecular chain, an unsaturated hydrocarbon having more than one double bond in the molecule, and an organopolysiloxane having more than one double bond in the molecule. Here, the crosslinking organopolysiloxane may have or may be free of unreacted silicon-bonded hydrogen atoms, phenyl groups, and similar aromatic hydrocarbon groups; octyl groups and similar long chain alkyl groups having from 6 to 30 carbon atoms; polyether groups, carboxyl groups, and similar modifying functional groups. In other words, any crosslinking organopolysiloxane can be used without limitations to physical modes or preparation methods such as dilution, properties, and the like.

As one example, the crosslinking organopolysiloxane can be obtained by performing an addition reaction on a crosslinking component selected from an organohydrogenpolysiloxane which consists of a structural unit selected from the group consisting of a SiO unit, an HSiO unit, an R^{b}SiO unit, an R^{b}HSiO unit, an R^{b}SiO unit, an R^{b}SiO unit, and an R^{b}HSiO unit (where R^{b} is a substituted or unsubstituted monovalent hydrocarbon group having from 1 to 30 carbon atoms, with the exception of aliphatic unsaturated groups; and a portion of R^{b} is a monovalent hydrocarbon group having from 8 to 30 carbon atoms), the organohydrogenpolysiloxane having an average of 1.5 or more silicon-bonded hydrogen atoms in the molecule; and a crosslinking compound selected from the group consisting of a polyoxyalkylene compound having unsaturated hydrocarbon groups at both molecular terminals; a polyether compound such as a polyglycerine compound or a polyglycidylether compound; an α,ω-diene unsaturated hydrocarbon expressed by the general formula: CH=CH-CᵣH-CH=CH₂ (where r is an integer from 0 to 26); and an organopolysiloxane formed from a structural unit selected from the group consisting of a SiO₂ unit, a (CH=CH)SiO unit, an R^{c}SiO_{1.5} unit, an R^{c}(CH=CH)SiO unit, an R^{c}SiO unit, an R^{c}SiO unit, and an R^{c}(CH=CH)SiO unit (where R^{c} is a substituted or unsubstituted monovalent hydrocarbon group having from 1 to 30 carbon atoms, with the exception of aliphatic unsaturated groups), the organopolysiloxane having an average of 1.5 or more silicon-bonded vinyl groups in the molecule. Note that by addition-reacting the unreacted silicon-bonded hydrogen atoms, the modifying functional groups described above can be introduced. For example, by reacting 1-hexene with a crosslinking organopolysiloxane having unreacted silicon-bonded hydrogen atoms, hexyl groups (C6 alkyl groups) are introduced.

Such a crosslinking organopolysiloxane can be used without limitations, regardless of the physical modes or preparation methods such as dilution, properties, and the like, but particularly preferable examples thereof include α,ω-diene crosslinking silicone elastomers (commercially available products include DC 9040 Silicone Elastomer Blend, DC 9041 Silicone Elastomer Blend, DC 9045 Silicone Elastomer Blend, DC 9046 Silicone Elastomer Blend, EL-9140 DM Silicone Elastomer Blend, 9546 Silicone Elastomer Blend, 9027 Silicone Elastomer Blend, FB-9586 Silicone Elastomer Blend, and EL-8040 ID Silicone Organic Blend manufactured by Dow Corning USA) described in US Patent No. 5,654,362. Similarly, examples of partial crosslinking organopolysiloxane polymers include, by INCI names (International Nomenclature Cosmetic Ingredient labeling names), (dimethicone/vinyldimethicone) crosspolymers, (dimethicone/phenylvinyldimethicone) crosspolymers, (PEG-8 to 30/C6 to C30 alkyldimethicone) crosspolymers, (vinyldimethicone/C6 to C30 alkyldimethicone) crosspolymers, and (dimethicone/polyglycerin) crosspolymers.

Examples of other preferable crosslinking organopolysiloxanes include silicone polyether elastomer gels that display increased compatibility with various organic components and stable thickening effects due to the introduction of polyoxypropylene groups described in WO2007/109240 and WO2009/006091, commercially available products of which include Dow Corning EL-8050 ID SILICONE ORGANIC ELASTOMER BLEND, Dow Corning EL-8051 IN SILICONE ORGANIC ELASTOMER BLEND, and Dow Corning EL-7040 HYDRO ELASTOMER BLEND); and the pituitous silicone fluids described in WO2011/028765 and WO2011/028770.

When compounded in an external use preparation or a cosmetic composition as an emulsifiable crosslinking organopolysiloxane that is crosslinked by a polyether compound, the liquid organopolysiloxane of the present invention functions as a dispersing agent, which yields the advantage that a uniform emulsification system (dosage form) can be formed.

On the other hand, when a non-emulsifiable crosslinking organopolysiloxane which is crosslinked by an unsaturated group-containing organopolysiloxane or an unsaturated hydrocarbon group such as a diene, a polyoxypropylene having reactive unsaturated groups at both molecular terminals of the molecular chain, or the like is compounded as a component in an external use preparation or a cosmetic composition, a thick, smooth tactile sensation can be imparted to the skin or the hair, and a matte finish and effects of concealing wrinkles, pigmented spots, and the like can be obtained. Further, this configuration is advantageous in that the feel of adhesion to the skin of the cosmetic composition is improved and cosmetic retainability is enhanced because the effects of retaining various oil agents and increasing the viscosity are high.

One type or two or more types of the solid silicone resin or crosslinking organopolysiloxane (excluding the liquid organopolysiloxane of the present invention) can be compounded in accordance with the purpose thereof and are preferably compounded in a range from 0.05 to 25 wt. (mass) % and more preferably in a range from 0.1 to 15 wt. (mass) % of the entire cosmetic composition or the external use preparation, depending on the purpose and the intention of compounding.

### Acryl silicone dendrimer copolymer

The external use preparation or the cosmetic composition of the present invention may further contain an acryl silicone dendrimer copolymer. The acryl silicone dendrimer copolymer is a vinyl polymer having a carbosiloxane dendrimer structure on the sidechain, and particularly preferable examples thereof include the vinyl polymer described in Japanese Patent No. 4009382 (Japanese Unexamined Patent Application Publication No. 2000-063225). Examples of commercially available products include FA4001 CM Silicone Acrylate, FA4002 ID Silicone Acrylate, and the like manufactured by Dow Corning Toray Co., Ltd. and acryl silicone dendrimer copolymers having a long chain alkyl group on the sidechain or the like having from 8 to 30 carbon atoms and preferably from 14 to 22 carbon atoms. When compounding the acryl silicone dendrimer copolymer alone, superior film formability can be obtained. Therefore, by compounding the acryl silicone dendrimer copolymer in the external use preparation or cosmetic composition according to the present invention, a strong coating film can be formed on the applied part, and cosmetic durability such as sebum resistance, rubbing resistance, and the like can be significantly improved.

Using the liquid organopolysiloxane of the present invention in combination with the acryl silicone dendrimer copolymer yields advantages in that the surface protective properties such as sebum resistance can be improved due to strong water repellency provided by the carbosiloxane dendrimer structure, and irregularities such as pores can also be effectively concealed. In addition, the liquid organopolysiloxane of the present invention causes the acryl silicone dendrimer copolymer to blend well with the other oil agents and has excellent compatibility with the skin and surface of the hair, so the hardness of the acryl silicone dendrimer copolymer can be reduced, and a cosmetic film with an excellent adhesive sensation and a lasting feel of moisture can be achieved. In addition, cosmetic retainability is improved, which has the advantage that degradation of the skin surface or hair can be suppressed for an extended period of time.

A compounded amount of the acryl silicone dendrimer copolymer can be suitably selected based on the purpose and compounding intent thereof, but is preferably in a range from 1 to 99 wt.% (mass%) and more preferably in a range from 30 to 70 wt.% (mass%) of the entire external use preparation or cosmetic composition.

### [Silicone raw rubber (silicone gum)]

In the external use preparation or the cosmetic composition of the present invention, an ultra-high viscous yet fluid component having a viscosity at room temperature of 1,000,000 mm²/s or higher, which is referred to as a silicone raw rubber (silicone gum), can be suitably used as the silicone oil. Silcone rubber is a straight-chained diorganopolysiloxane with an ultra-high degree of polymerization and is also called silicone raw rubber or organopolysiloxane rubber. Silicone rubber is differentiated from the oily silicones described above because the degree of polymerization of silicone rubber is high and, as a result, it has a degree of plasticity that is measurable. These silicone gums can be compounded directly in the external use preparation or the cosmetic composition of the present invention - in a hair cosmetic composition for the purpose of imparting a tactile sensation - or can be compounded as liquid gum dispersions in which an oil-like silicone is dispersed (oil dispersions of silicone gums).

Examples of such a silicone raw rubber include substituted or unsubstituted organopolysiloxanes having a dialkylsiloxy unit (D unit) such as a dimethylpolysiloxane, a methylphenylpolysiloxane, an aminopolysiloxane, or a methylfluoroalkylpolysiloxane, or products having micro-crosslinked structures thereof, and typical examples thereof include products expressed by the general formula: R¹⁰(CH₃)₂SiO[(CH₃)₂SiO]ₛ[(CH₃)R¹²SiO]ₜSi(CH₃)₂R¹⁰ (wherein R¹² moieties are each independently a group selected from vinyl groups, phenyl groups, alkyl groups having from 6 to 20 carbon atoms, aminoalkyl groups having from 3 to 15 carbon atoms, perfluoroalkyl groups having from 3 to 15 carbon atoms, and quaternary ammonium salt group-containing alkyl groups having from 3 to 15 carbon atoms; the terminal group R¹⁰ moieties are each independently a group selected from alkyl groups having from 1 to 8 carbon atoms, phenyl groups, vinyl groups, aminoalkyl groups having from 3 to 15 carbon atoms, hydroxyl groups, and alkoxy groups having from 1 to 8 carbon atoms; s=2,000 to 6,000; t=0 to 1,000; and s+t=2,000 to 6,000). Of these, a dimethylpolysiloxane raw rubber having a degree of polymerization of 3,000 to 20,000 is preferable. Additionally, an amino-modified methylpolysiloxane raw rubber having a 3-aminopropyl group, an N-(2-aminoethyl)3-aminopropyl group, or the like on the molecular sidechain or terminal is preferable. Moreover, in the present invention, one or two or more types of silicone gums can be used as necessary. Further, the use of the liquid organopolysiloxane of the present invention imparts a smooth, sliding feeling to wet hair at the time of rinsing or the like, and the substance is effectively absorbed by the surface of the hair, which yields a sustained moisturizing effect to the hair after drying and a protective effect to the hair surface and makes it possible to suppress flyaway.

Silicone gum has an ultra-high degree of polymerization and, therefore forms a protective film with superior breathability and retention on hair. Therefore, the silicone gum is a component which can particularly provide glossiness and luster to hair and can impart a texture of firmness and body to the entire hair during use and after use. A silicone gum with a high degree of polymerization can also be compounded in the external use preparation or the cosmetic composition in a form that is diluted with a silicone oil to reduce the viscosity.

A compounded amount of the silicone gum is, for example, from 0.05 to 30 wt.% (mass%) and preferably from 1 to 15 wt.% (mass%) of the entire external use preparation or cosmetic composition. When the silicone gum is used as an emulsion composition prepared via a process of pre-emulsifying (including emulsion polymerization), the silicone gum can easily be compounded, and can be stably compounded in the hair cosmetic composition of the present invention. An effect of imparting a specific feeling to touch or glossiness of the hair may be insufficient if the compounded amount of the silicone gum is less than the lower limit described above.

### [Polyamide-modified Silicone]

Examples of polyamide-modified silicones that can be preferably compounded in the external use preparation or the cosmetic composition of the present invention include siloxane-based polyamide compounds described in US Patent No. 5,981,680 (Japanese Unexamined Patent Application Publication No. 2000-038450) and Japanese Unexamined Patent Application Publication (Translation of PCT Application) No. 2001-512164, and examples of commercially available products include 2-8178 Gellant, 2-8179 Gellant and the like (manufactured by Dow Corning USA). This polyamide-modified silicone also functions as an oil-based raw material, specifically as a thickening/gelling agent of a silicone oil.

When the polyamide-modified silicone is used in combination with the liquid organopolysiloxane of the present invention, the external use preparation or the cosmetic composition of the present invention has good spreading and setting as well as an excellent sense of stability and adhesion when applied to the skin, the hair, or the like. Additionally, there are advantages from a quality standpoint such that a glossy feeling of sheerness and superior luster can be provided, the viscosity or hardness (softness) of the entire cosmetic composition containing the oil-based raw material can be appropriately adjusted, and an oily sensation (oily and sticky feeling to touch) can be totally suppressed. Further, by using the liquid organopolysiloxane of the present invention, the dispersion stability of perfumes, powders, and the like is improved, which yields the characteristic that a uniform and fine cosmetic sensation is maintained over an extended period of time.

### [Silicone wax]

Silicone waxes that can be preferably compounded in the external use preparation or the cosmetic composition of the present invention are higher alkyl-modified silicones and alkyl-modified silicone resins. The higher alkyl-modified silicone is a wax at room temperature and is a component that is useful as a portion of the base material of a solid cosmetic composition (for example, an oil-based solid skin cosmetic composition or a solid hair cosmetic composition). Therefore, the higher alkyl-modified silicone can be preferably used in the external use preparation or the cosmetic composition of the present invention. Examples of the higher alkyl-modified silicone wax include methyl (long chain alkyl) polysiloxanes having both molecular terminals capped with trimethylsiloxy groups, copolymers of a dimethylpolysiloxane having both molecular terminals capped with trimethylsiloxy groups and a methyl (long chain alkyl) siloxane, dimethylpolysiloxane modified with long chain alkyls at both terminals, and the like. Examples of commercially available products include AMS-C30 Cosmetic Wax, 2503 Cosmetic Wax, and the like (manufactured by Dow Corning Corporation, in the USA).

The liquid organopolysiloxane of the present invention has good affinity with the higher alkyl-modified silicone wax and has excellent dispersibility properties in wax, so an external use preparation or a cosmetic composition having excellent storage stability over time can be obtained. In addition, the moldability of the external use preparation or the cosmetic composition, and particularly of a solid cosmetic composition, is excellent. In particular, in a system comprising a powder, an effect of uniformly and stably dispersing the powder in the base material containing the higher alkyl-modified silicone wax is obtained, and the hardness of the base material after molding can be moderately mitigated, which makes it possible to provide an external use preparation or a cosmetic composition that spreads smoothly and uniformly when applied.

In the external use preparation or cosmetic composition of the present invention, the higher alkyl-modified silicone wax preferably has a melting point of not lower than 60°C because such will lead to cosmetic retainability effects and stability at high temperatures.

The alkyl-modified silicone resin is a type of film-forming polymer that is compoundable in an oil phase and is a component that imparts sebum durability, moisturizing properties, and a fine tactile sensation of the skin to the external use preparation or the cosmetic composition. An alkyl-modified silicone resin that is in the form of a wax at room temperature can be suitably used. Preferred examples thereof include the silsesquioxane resin wax described in Japanese Unexamined Patent Application Publication (Translation of PCT Application) No. 2007-532754. Examples of commercially available products include SW-8005 C30 RESIN WAX (manufactured by Dow Corning Corporation, in the USA), and the like.

As in the case of a higher alkyl-modified silicone wax, the liquid organopolysiloxane of the present invention has good affinity with the alkyl-modified silicone resin wax and has excellent dispersion properties in wax, so an external use preparation or a cosmetic composition having superior storage stability over time can be obtained. Further, an aqueous phase can be stably emulsified along with other optional surfactants in the oil phase containing the alkyl-modified silicone resin wax, which makes it possible to impart a luxurious tactile sensation, moisturizing effects, and effects of improving cosmetic retainability exemplified by moisture resistance and sebum resistance to the skin or the hair.

### [Antiperspiration active component and deodorant agent]

Additionally, in cases where the external use preparation or the cosmetic composition according to the present invention is an anti-perspirant, or depending on the purpose thereof, the external use preparation or the cosmetic composition can contain an anti-perspiration active component and/or a deodorant agent.

Examples of the anti-perspiration active component include astringent salts such as aluminum chlorohydrate, aluminum-zirconium tetrachlorohydrex glycine (ZAG), and the like; but aluminum, hafnium, zinc, and zirconium salts (e.g. aluminum halide, aluminum hydroxy halide, zirconium halide, zirconium oxyhalide, zirconium hydroxy halide, zirconyl hydroxide halide, aluminum chloride zirconium, zirconium lactate-aluminum, and basic aluminum halide) can be used. Examples thereof include Al₂(OH)₅Cl, aluminum bromide, buffer aluminum sulphate, alum, dried alum, various aqueous, alcohol, or glycine complexes thereof (e.g. a complex of an aluminum-zirconium chlorohydrate and glycine comprising aluminum, zirconium, and glycine (a ZAG complex), and the like. A single anti-perspiration active component may be used or a combination of two or more may be used. In cases where the anti-perspirant composition according to the present invention is a water-in-oil emulsion-type anti-perspirant composition, these anti-perspiration active components are an aqueous phase component. On the other hand, soybean extracts and isoflavones are known for their anti-perspirant effects; and, because they have low water solubility, are preferably used by dissolving them in the oil phase.

In the present invention, a compounded amount of the anti-perspiration active component is an amount sufficient to reduce perspiration, and suppressing the compounded amount to a small amount can be beneficial in personal care compositions. Specifically, from the standpoints of anti-perspirant effects and feeling to touch, the compounded amount of the anti-perspiration active component in an anti-perspirant composition is preferably from 5 to 25 wt.% (mass%) of the entire cosmetic composition. When using a water soluble anti-perspiration active component, from the standpoint of cost effectiveness, it is preferable to increase the proportion of water in the composition to a maximum limit, while maintaining anti-perspirant effects, but the anti-perspiration active component can also be added to the aqueous phase at amount near the saturation amount.

A deodorant agent can be compounded together with or instead of the anti-perspiration active component in the external use preparation or the cosmetic composition of the present invention, and particularly an antiperspiration composition. Examples of the deodorant agent include deodorizers, perfumes, and substances that prevent or remove odors caused by perspiration. Such deodorant agents are antimicrobial agents (germicides or fungicides), bacteriostatic agents, odor absorbing substances, deodorizers, perfumes, or the like, and are compounded for the purpose of preventing underarm odor, odor from perspiration, foot odor, and other bodily odors. Note that these deodorant agents are useful in cosmetic compositions or external use preparations other than anti-perspirants and it goes without saying that they can be beneficially compounded in the external use preparation or cosmetic composition of the present invention.

Examples of antimicrobial agents include alkyltrimethylammonium bromide, cetylpyridinium chloride, benzethonium chloride, benzalkonium chloride, chlorhexidine hydrochloride, chlorhexidine gluconate, [[(diisobutylphenoxy)ethoxy]ethyl] dimethylbenzylammonium chloride, N-lauroyl sarcosine sodium, N-palmitoyl sarcosine sodium, N-myristoyl glycine, N-lauroyl sarcosine potassium, trimethyl ammonium chloride, aluminum chlorohydroxy sodium lactate, triethyl citrate, tricetyl methyl ammonium chloride, 1,5-pentanediol, 1,6-hexanediol, 2,4,4'-trichloro-2'-hydroxy diphenylether (triclosan), and 3,4,4'-trichlorocarbanilide(triclocarban); L-lysine hexadecylamide and similar diaminoalkylamidos; citric acid, salicylic acid, piroctose, and other heavy metal salts, preferably zinc salts and acids thereof; pyrithione heavy metal salts, preferably pyrithione zinc, phenol zinc sulfate, ethylparaben, butylparaben, hinokitiol, farnesol, phenoxyethanol, isopropyl methylphenol, propolis, lysozyme, lysozyme chloride, combinations of lysozyme and vitamin E or derivatives thereof, combinations of organic acids such as lysozyme and α-hydroxyacid and the like; and the like.

Examples of bacteriostatic agents include 1-heptyl glyceryl ether, 1-(2-ethylhexyl)glyceryl ether, 1-octyl glyceryl ether, 1-decyl glyceryl ether, 1-dodecyl glyceryl ether, and similar glyceryl monoalkyl ethers.

The odor absorbing substance is not particularly limited, provided that it absorbs odor causing substances and reduces odor, is constituted by a portion of the inorganic powders and organic polymers described above, and displays the same characteristics.

Examples of the odor absorbing substance include zinc oxide, magnesium oxide, zeolite, aluminometasilicate, silicic anhydride, colloidal silica, talc, mica, hydroxyapatite, cellulose, corn starch, silk, nylon powder, crosslinking organopolysiloxane powder, organopolysiloxane elastomer spherical powder, and the like. Likewise, carbonates such as alkali metal carbonates, alkali metal bicarbonate salts, and the like and hydrogen carbonates, ammonium salts, tetraalkylammonium salts, and the like can be used. Of these odor absorbing substances, sodium salts and potassium salts are more preferable. Additionally, organic or inorganic porous particles carrying silver, copper, zinc, cerium, or similar metal ions (e.g. silver ion-carrying zeolite, silver ion/zinc ion/ammonium ion-carrying zeolite), or aggregates of needle-like crystals including silver cancrinite can be used. Because these function as antimicrobial agents and odor absorbing substances, they can be used beneficially as the deodorant agent.

Furthermore, hydroxyalkylated cyclodextrin, sake cake extract containing rice fermenting liquid, and various extracts derived from animals, vegetables, microorganisms, fungi, and the like such as brown seaweed extract, cinnamon bark, clove, fennel, ginger, mentha, citron, gentiana lutea, apricot, eucalyptus, Sophora flavescens, mulberry, althea, sage, Anthemis nobilis, Scutellaria root, nutgall, gardenia, hamamelis, herbs, and the like can be used as the deodorant agent. A part of these components overlaps with a bioactive component described above, but selecting these extracts as the deodorant agent for the purpose of the functional effects thereof is both beneficial and preferable from the standpoint of the composition design of the cosmetic composition.

Preferably from 0.001 to 60 wt.% (mass%), more preferably from 0.01 to 30 wt.% (mass%), and yet more preferably from 0.01 to 3 wt.% (mass%) of the odor absorbing substance is included in the entire composition. Provided that the compounded amount of the odor absorbing substance is within this range, there is an advantage that deodorizing performance can be improved while not negatively affecting the strength and feeling to touch of the formulation.

Suitable perfumes include known topical use substances, topical use substances that are effective in masking malodor accompanied by perspiration, and various topical use substances that provide a composition having a desired aroma. Examples thereof include the whole of perfumes and perfume chemicals such as perfume precursors, deodorizing fragrances, and the like that are suitable for topical application to the skin and, as necessary, may be a blended perfume component.

### [TECHNICAL FIELD]

The liquid organopolysiloxane of the present invention or the composition containing the same can be suitably used as a raw material for an external use preparation or a cosmetic composition. In particular, the liquid organopolysiloxane of the present invention produced via an acidizing process and the composition of the present invention containing the liquid organopolysiloxane of the present invention and at least one type of acid have a reduced odor and can therefore be suitably compounded in an external use preparation or a cosmetic composition.

Note that the carbonyl value measurement method described above is capable of accurately and simply assaying a carbonyl compound, so the method can be preferably used for the evaluation of the odor of a product of an external use preparation or a cosmetic composition.

### Practical Examples

The present invention is described below using examples, but the present invention is not limited thereto. In the following composition formulas, an Me₃SiO group (or an Me₃Si group) is represented by "M", an Me₃SiO group is represented by "D", an Me₂HSiO group (or an Me₂HSi group) is represented by "M^{H}", an MeHSiO group is represented by "D^{H}", an Me₂(CH₂=CH)SiO group [or an Me₂(CH₂=CH)Si group] is represented by "M^{Vi}", and an Me(CH₂=CH)SiO group is represented by "D^{Vi}". An Me₂[CH₂=CH-(CH₂)₄]SiO group [or an Me₂[CH₂=CH-(CH₂)₄]Si group] is represented by "M^{Hex}", an Me[CH₂=CH-(CH₂)₄]SiO group is represented by "D^{Hex}", an MeSiO_{3/2} group is represented by "T", and an MeSiO_{4/2} group is represented by "Q". Units in which the methyl groups in M and D are modified by one of the substitutents are represented by M^{R} and D^{R}.

In addition, the xylitol monoallyl ether and xylitol residue described below are a raw material and functional groups as described in this specification. More specifically, the xylitol monoallyl ether is a raw material containing a xylitol monoallyl ether expressed by the structural formula: CH₂=CH-CH₂-OCH₂[CH(OH)]₃CH₂OH and the structural formula: CH₂=CH-CH₂-OCH[CH(OH)CH₂OH]₂ at a composition ratio of approximately 9:1, and a xylitol residue represented by the corresponding formula -C₃H₆-OCH₂[CH(OH)]₃CH₂OH or -C₃H₆-OCH[CH(OH)CH₂OH]₂ is introduced to the comodified silicone of the present invention at the same composition ratio.

Note that in the practical examples and comparative examples below, "Production of Silicone Compound No. X" and the like is included for the sake of convenience, and the obtained products are in the form of mixtures containing a small amount of unreacted raw material and the like in addition to the main components.

### [Practical Example 1] <Production of Silicone Compound No. 1>

First, 137.7 g of a methylhydrogenpolysiloxane expressed by the average composition formula: MD_{42.9}D^{H}_{6.7}M and 14.9 g of a 3-methacryloxypropyl(tris(trimethylsiloxy)silyl ethyl dimethylsiloxy)silane expressed by the following average composition formula: 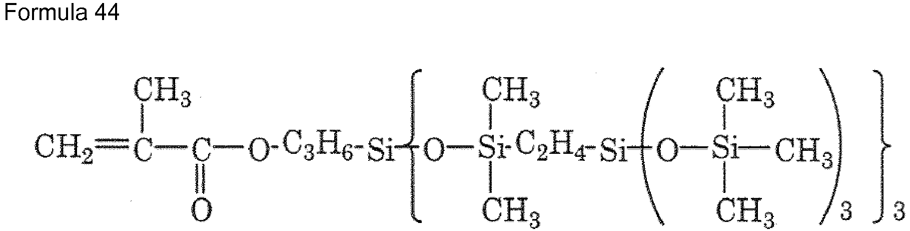 were placed in a reaction vessel and heated to 80°C while agitating under a nitrogen stream. Next, 0.12 mL of an isopropyl alcohol solution of a platinum-1,3-divinyl-1,1,3,3-tetramethyldisiloxane complex (Pt concentration: 0.45 wt.%) was added and the mixture was reacted for 2 hours at 80 to 90°C. A small amount of the reaction liquid was then sampled, and it was confirmed that the target reaction rate was reached by an alkali decomposition gas generation method (the remaining Si-H groups were decomposed using a KOH ethanol/aqueous solution, and the reaction rate was calculated from the volume of the generated hydrogen gas). Next, 36.0 g of hexadecene (α-olefin purity: 91.7%) was added to the reaction mixture, and after the mixture was reacted for one hour at 85 to 100°C, it was confirmed via the same method that the target reaction rate was reached. Next, 10.1 g of xylitol monoallyl ether (purity: 91.2%) and 120 g of isopropyl alcohol (IPA) were added to the reaction mixture, and 0.20 ml of the platinum catalyst described above was added. After reacting for 4 hours at 65 to 80°C, the mixture was sampled. As a result of calculating the reaction rate, it was found that a modified silicone intermediate expressed by the average composition formula: MD_{42.9}D^{R*31}_{0.3}D^{R*21}_{1.1}D^{R*11}_{4.0}D^{H}_{1.3}M had been produced. Here, R^{*11}, R^{*21}, and R^{*31} are as described below.

R^{*11}=-C₁₆H₃₃

R^{*21}=xylitol residue

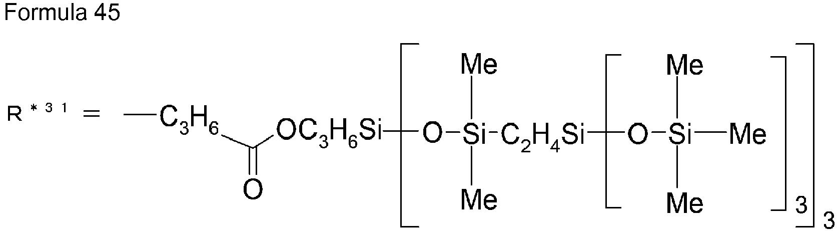

The reaction liquid was cooled to 50°C, and then 2.1 g of 1,5-hexadiene and 0.025 g of natural vitamin E were added and reacted for 4 hours at 50 to 80°C. In this case, the Vi/H molar ratio when crosslinking was 1.0. The mixture was sampled and the reaction rate was calculated. As a result, it was found that the reaction was substantially complete. Next, 3.0 g of a 0.12% phosphoric acid aqueous solution and 3.0 g of purified water were added, and after the mixture was treated for 1 hour at 80°C, the mixture was neutralized by adding 0.1 g of 2.5% ammonia water. Thereafter, low-boiling-point matter was removed by distillation under reduced pressure at 80 to 110°C to obtain a liquid organomodified organopolysilxane having a xylitol group and a crosslinking portion, wherein the organopolysiloxane portion and the organic portion are linked via carbon-silicon bonds of the crosslinking portion. This product was a light yellow to ash-white colored viscous liquid at 25°C, and virtually no odor was perceived.

The average structural formula (schematic illustration) of the liquid organopolysiloxane obtained in Practical Example 1 is shown below. 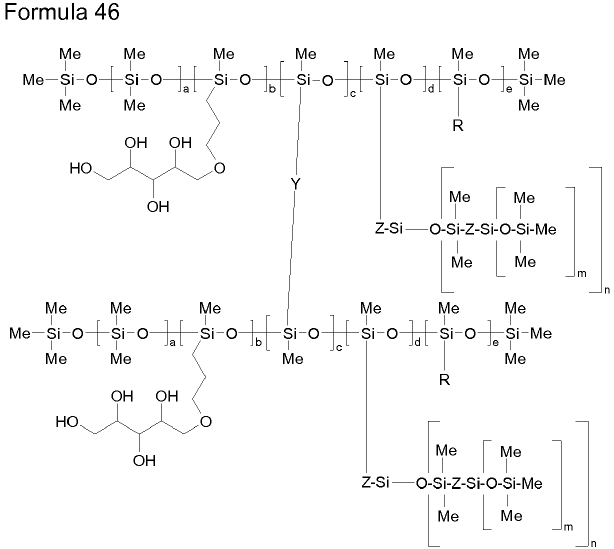 (In this formula, Me is a methyl group, Z in [] n is -CH₂CH₂-, Z outside of []n is -C₃H₆-COO-C₃H₆-, R is -C₁₆H₃₃, Y is -(CH₂)₆-, a is 42.9, b is 1.1, c is 1.3, d is 0.3, e is 4.0, m is 3, and n is 3.)

### [Practical Example 2]<Production of Silicone Compound No. 2]>

First, 136.8 g of a methylhydrogenpolysiloxane expressed by the average composition formula: MD_{43.4}D^{H}_{7.4}M and 43.7 g of hexedecene (α-olefin purity: 91.7%) were placed in a reaction vessel and heated to 70°C while agitating under a nitrogen stream. When 0.15 ml of a hexamethyldisiloxane solution of a platinum-1,3-divinyl-1,1,3,3-tetramethyldisiloxane complex (Pt concentration: 0.45 wt.%) was added, heat was generated immediately, and the mixture heated to 115°C within one minute. A reaction was then performed for 4.5 hours while maintaining the temperature at 90 to 100°C. A small amount of the reaction liquid was then sampled, and it was confirmed that the target reaction rate was reached by an alkali decomposition gas method. Next, 20.1 g of a xylitol monoallyl ether (purity: 91.2%), 131 g of isopropyl alcohol, and 0.02 g of natural vitamin E were added to the reaction mixture, and 0.15 ml of the platinum catalyst described above was then added. After reacting for 4.5 hours at 65 to 80°C, the mixture was sampled. As a result of calculating the reaction rate with the same method as that described above, it was found that a modified silicone intermediate expressed by the average composition formula:
MD_{43.4}D^{R*21}_{5.04}D^{R*11}₂₀D^{H}_{0.36}M had been produced. Here, R^{*11} and R^{*21} are as defined above.

Next, after the mixture was heated under reduced pressure and the isopropyl alcohol was removed by distillation, 124 g of toluene was added as a solvent for a crosslinking reaction. Next, 14.6 g of a methylhexenylpolysiloxane expressed by the average composition formula: ^{Hex}MD_{56.7}D^{Hex}_{3.3}M^{Hex} and 0.48 ml of the platinum catalyst described above were added and reacted for 12 hours at 90 to 115°C. The Vi/H molar ratio at the time of crosslinking was 2.45. The mixture was sampled, and as a result of calculating the reaction rate, it was found that 0.20 of the average of 0.36 remaining Si-H groups in the modified silicone intermediate were consumed and that 0.16 groups remained. Next, 6.0 g of a 0.12% phosphoric acid aqueous solution, 3.0 g of purified water, and 60 g of isopropyl alcohol were added, and after the mixture was treated for 1 hour at 70 to 80°C, the low-boiling-point matter was removed by distillation at 70 to 100°C under reduced pressure to obtain a liquid organomodified organopolysiloxane having a xylitol group and a crosslinking portion, wherein the organopolysiloxane portion and the organic portion are linked via carbon-silicon bonds of the crosslinking portion. This product was an ash-white colored viscous liquid at 25°C, and virtually no odor was perceived.

### [Practical Example 3] <Production of Silicone Compound No. 3>

First, 86.7 g of a methylhydrogenpolysiloxane represented by the average composition formula: MD_{43.4}D^{H}_{7.4}M and 9.4 g of 3-methacryloxypropyl(tris(trimethylsiloxy)silylethyldimethylsiloxy)silane were placed in a reaction vessel, and 0.10 ml of an isopropyl alcohol solution of a platinum-1,3-divinyl-1,1,3,3-tetramethyldisiloxane complex (Pt concentration: 0.45 wt.%) was added while agitating under a nitrogen stream. After reacting for 2.5 hours at 75 to 90°C, 19.2 g of hexedecene (α-olefin purity: 91.7%) was added to the reaction mixture. After reacting for 2 hours at 70 to 90°C, a small amount of the reaction liquid was sampled, and when the reaction rate was confirmed by an alkali decomposition gas generation method, it was found that an intermediate expressed by the average composition formula: MD_{43.4}D^{R*31}_{0.31}D^{R*11}_{3.78}D^{H}_{2.81}D^{OR}_{0.50}M had been produced. Next, 8.6 g of a xylitol monoallyl ether (purity: 91.2%), 120 g of isopropyl alcohol, and 0.03 g of natural vitamin E were added to the reaction mixture, and 0.10 ml of the platinum catalyst described above was then added. After reacting for 5.5 hours at 55 to 80°C, the mixture was sampled. As a result of calculating the reaction rate, it was found that a modified silicone intermediate expressed by the average composition formula: MD₄₃.₄D^{R*31}₀.₃₁D^{R*21}_{1.80}D^{R*11}_{3.78}D^{H}_{1.01}D^{OR}_{0.50}M had been produced. Here, R^{*11}, R^{*21}, and R^{*31} are as described above. In addition, D^{OR} is a structural unit produced by the dehydrogenation of D^{H} and an alcoholic hydroxyl group, the unit being an Me(OR)SiO group containing a Si-O-C bond or a Si-O-H bond. In this example, OR=O-CH(CH₃)₂ or OH.

Next, after the mixture was heated under reduced pressure and the isopropyl alcohol was removed by distillation, 60 g of toluene was added as a solvent for a crosslinking reaction. Next, 65.4 g of a methylhexenylpolysiloxane expressed by the average composition formula: ^{Vi}MD₁₃₆D^{Vi}_{2.25}M^{Vi}: and 0.10 ml of the platinum catalyst described above were added and reacted for 2 hours at 90 to 100°C. The Vi/H molar ratio at the time of crosslinking was 1.15. The mixture was sampled, and as a result of calculating the reaction rate, it was found that 0.83 of the average of 1.01 remaining Si-H groups in the modified silicone intermediate were consumed and that 0.18 groups remained. Next, 6.0 g of a 0.12% phosphoric acid aqueous solution, 3.0 g of purified water, and 60 g of isopropyl alcohol were added, and after the mixture was treated for 1 hour at 75°, 175 g of a dimethylpolysiloxane (6 cst) serving as a diluent was added and mixed. Thereafter, the low-boiling-point matter was removed by distillation under reduced pressure while heating at 80 to 100°C to obtain a 6 cs diluted product (concentration: 50%) of a liquid organomodified organopolysilxane having a xylitol group and a crosslinking portion, wherein the organopolysiloxane portion and the organic portion are linked via Si-C bonds of the crosslinking portion. This product was very viscous at 100°C but was a tan to ash-white colored liquid with fluidity. At 25°C, the product had thread-forming properties with a soft, jelly-like consistency, and it was confirmed to be a liquid from the fact that the product demonstrated fluidity when tilted.

### [Practical Example 4] <Production of Silicone Compound No. 4>

First, 137.6 g of a methylhydrogenpolysiloxane represented by the average composition formula: MD_{43.4}D^{H}_{7.4}M and 14.9 g of 3-methacryloxypropyl(tris(trimethylsiloxy)silylethyldimethylsiloxy)silane were placed in a reaction vessel, and 0.10 ml of a hexamethyldisiloxane solution of a platinum-1,3-divinyl-1,1,3,3-tetramethyldisiloxane complex (Pt concentration: 0.45 wt.%) was added while agitating under a nitrogen stream. After reacting for 2 hours at 80 to 90°C, a small amount of the reaction liquid was sampled, and it was confirmed by an alkali decomposition gas generation method that the target reaction rate was reached. Next, when 31.4 g of hexadecene (α-olefin purity: 91.7%) was added to the reaction mixture, heat was generated immediately, and the mixture heated to 107°C after one minute. After reacting for 3.5 hours while maintaining the temperature at 90°C, the mixture was confirmed with the same method as that described above, and it was found that an intermediate expressed by the average composition formula: MD_{43.4}D^{R*31}_{0.30}D^{R*11}_{3.79}D^{H}_{3.25}D^{OR}_{0.06}M had been produced. Next, 13.0 g of a xylitol monoallyl ether (purity: 91.2%), 123 g of IPA, and 0.02 g of natural vitamin E were added to the reaction mixture, and 0.10 ml of the platinum catalyst described above was then added. After reacting for 4 hours at 60 to 80°C, the mixture was sampled. As a result of calculating the reaction rate, it was found that a modified silicone intermediate expressed by the average composition formula: MD_{43.4}D^{R*31}_{0.30}D^{R*21}_{1.71}D^{R*11}₃₇₉D^{H}_{3.79}D^{OR}_{0.22}M mad been produced. Here, R^{*11}, R^{*21}, and R^{*31} are as described below. In addition, D^{OR} is a structural unit produced by the dehydrogenation of D^{H} and an alcoholic hydroxyl group, the unit being an Me(OR)SiO group containing a Si-O-C bond or a Si-O-H bond. In this practical example, this may be OR=O-CH(CH₃)₂, OH, or a group of a form in which a hydrogen atom is removed from one of the hydroxyl groups of the xylitol portion (in this case, R is a monovalent or divalent organic group). R^{*11}=-C₁₆H₃₃

Most of R^{*21}= is a xylitol residue expressed by -C₃H₆-OCH₂[CH(OH)]₃CH₂OH OR -C₃H₆-OCH[CH(OH)CH₂OH]₂ (monovalent organic group), but a portion of the moiety may be a divalent organic group in which a hydrogen atom is removed from one of the OH groups in these xylitol residues (in a form in which the organic group is bonded to another Si atom via ether oxygen).

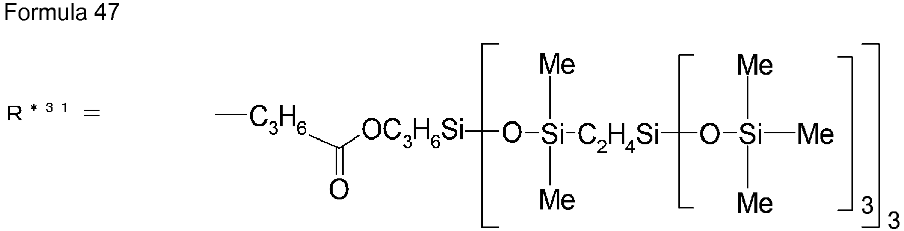

Here, 7.6 g of a vinylcyclohexene monooxide expressed by the following structural formula: 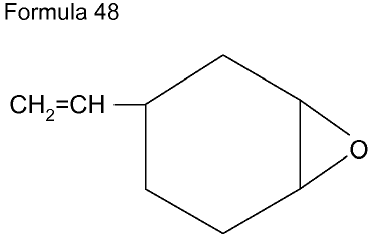 and 0.10 ml of the platinum catalyst described above were added to the mixture and reacted for 4.5 hours at 80°C, and the reaction was roughly completed. Further, after the mixture was heated at 95°C under reduced pressure and the isopropyl alcohol was removed by distillation, the mixture was once again heated for 2 hours at 140°C, and the viscosity of the reaction product increased. After the mixture was cooled to 100°C, the pressure was restored, and 106 g of isopropyl alcohol, 6.1 g of a 0.12% phosphoric acid aqueous solution, and 3.0 g of purified water were added. After the mixture was treated for 1 hour at 70 to 80°C, the mixture was neutralized by adding 0.21 g of 2.5% ammonia water. Thereafter, the low-boiling-point matter was removed by distillation under reduced pressure while heating at 90 to 100°C to obtain a liquid organomodified organopolysilxane having a

xylitol group and a crosslinking portion, wherein the organopolysiloxane portion and the organic portion are linked via Si-C bonds of the crosslinking portion. This product was a dark tan colored viscous liquid at 25°C, and virtually no odor was perceived.

### [Practical Example 5] <Production of Silicone Compound No. 5>

First, 127.8 g of a methylhydrogenpolysiloxane represented by the average composition formula: MD_{43.4}D^{H}_{7.4}M and 13.8 g of 3-methacryloxypropyl(tris(trimethylsiloxy)silylethyldimethylsiloxy)silane were placed in a reaction vessel, and 0.20 ml of an isopropyl alcohol solution of a platinum-1,3-divinyl-1,1,3,3-tetramethyldisiloxane complex (Pt concentration: 0.45 wt.%) was added while agitating under a nitrogen stream. After reacting for 1.5 hours at 80 to 95°C and 30.2 g of hexadecene (α-olefin purity: 91.7%) was added to the reaction mixture, heat was generated immediately, and the mixture heated to 106°C after one minute. After reacting for 4.5 hours while maintaining the temperature at 80 to 95°C, a small amount of the reaction liquid was sampled, and when the reaction rate was confirmed by an alkali decomposition gas generation method, it was found that an intermediate expressed by the average composition formula: MD₄₃. ₄D^{R*31}_{0.30}D^{R*11}_{4.00}D^{H}_{2.77}D^{OR}_{0.33}M had been produced. Next, 12.9 g of a xylitol monoallyl ether (purity: 91.2%), 132 g of isopropyl alcohol, and 0.03 g of natural vitamin E were added to the reaction mixture, and after reacting for 4.5 hours at 60 to 80°C, the mixture was sampled. As a result of calculating the reaction rate, it was found that a modified silicone intermediate expressed by the average composition formula: MD_{43.4}D^{R*31}_{0.30}D^{R*21}_{1.66}D^{R*11}_{4.00}D^{H}_{1.11}D^{OR}_{0.33}M had been produced. Here, R^{*11}, R^{*21}, and R^{*31} are as described above. In addition, D^{OR} is a structural unit produced by the dehydrogenation of D^{H} and an alcoholic hydroxyl group, the unit being an Me(OR)SiO group containing a Si-O-C bond or a Si-O-H bond. In this example, OR=O-CH(CH₃)₂ or OH.

Here, 15.0 g of a bis-allyl polyether expressed by the average composition formula: CH₂=CH-CH₂-(OCH₂CH₂)₁₅-O-CH₂-CH=CH₂ and 0.10 ml of the platinum catalyst described above were added and reacted for 1.5 hours at 80°C. The Vi/H molar ratio at the time of crosslinking was 1.1. When a small amount of the reaction liquid was sampled and the reaction rate was calculated, it was found that 0.91 of the average of 1.11 remaining Si-H groups in the modified silicone intermediate were consumed and that 0.20 groups remained. Therefore, 2.4 g of hexadecene (α-olefin purity: 91.7%) was further added and reacted for 4 hours at 80° to reduce the number of remaining Si-H groups to 0.07 groups, and the reaction was then terminated.

Next, 6.0 g of a 0.12% phosphoric acid aqueous solution and 3.0 g of purified water were added, and after the mixture was treated for 1.5 hours at 80°C, the mixture was neutralized by adding 0.19 g of 2.5% ammonia water. Thereafter, the low-boiling-point matter was removed by distillation under reduced pressure while heating at 95 to 100°C to obtain a liquid organomodified organopolysilxane having a xylitol group and a crosslinking portion, wherein the organopolysiloxane portion and the organic portion are linked via Si-C bonds of the crosslinking portion. This product was a dark brown colored viscous liquid at 25°C, and virtually no odor was perceived.

### [Practical Example 6] <Production of Silicone Compound No. 6>

First, 89.9 g of a methylhydrogenpolysiloxane expressed by the average composition formula: MD_{43.4}D^{H}_{7.4}M and 23.8 g of hexadecene (α-olefin purity: 91.7%) were placed in a reaction vessel, and when 0.10 ml of a hexamethyldisiloxane solution of a platinum-1,3-divinyl-1,1,3,3-tetramethyldisiloxane complex (Pt concentration: 0.45 wt.%) was added at 83°C while agitating under a nitrogen stream, heat was generated immediately, and the mixture heated to 106°C after one minute. After reacting for 1 hour while maintaining the temperature at 80 to 90°C, a small amount of the reaction liquid was sampled, and when the reaction rate was confirmed by an alkali decomposition gas generation method, it was found that an intermediate expressed by the average composition formula: MD_{43.4}D^{R*11}_{4.50}D^{H}_{2.90}M had been produced. Next, 9.4 g of a xylitol monoallyl ether (purity: 91.2%), 120 g of isopropyl alcohol, and 0.03 g of natural vitamin E were added to the reaction mixture, and 0.10 ml of the platinum catalyst described above was then added. After reacting for 2.5 hours at 60 to 80°C, the mixture was sampled. As a result of calculating the reaction rate, it was found that a modified silicone intermediate expressed by the average composition formula: MD₄₃. ₄D^{R*11}_{4.50}D^{R*21}_{1.90}D^{H}_{1.00}M had been produced. Here, R^{*11} and R^{*21} are as described above.

Next, after the mixture was heated under reduced pressure and the isopropyl alcohol was removed by distillation, 111 g of toluene was added as a solvent for a crosslinking reaction. Here, 76.6 g of a methylhexenylpolysiloxane expressed by the average composition formula: ^{Vi}MD₁₃₆D^{Vi}_{2.25}M^{Vi} and 0.15 ml of the platinum catalyst described above were added and reacted for 4.5 hours at 85 to 100°C. The Vi/H molar ratio at the time of crosslinking was 1.33. The mixture was sampled, and as a result of calculating the reaction rate, it was found that 0.89 of the average of 1.00 remaining Si-H groups in the modified silicone intermediate were consumed and that 0.11 groups remained. Therefore, 2.0 g of hexadecene (α-olefin purity: 91.7%) and 0.10 ml of the platinum catalyst described above were added, and as a result of reacting for 1 hour at 100°C, the number of remaining Si-H groups decreased to 0.03 groups. Next, 6.0 g of a 0.12% phosphoric acid aqueous solution, 3.0 g of purified water, and 60 g of isopropyl alcohol were added, and after the mixture was treated for 1 hour at 70 to 80°C, 180 g of a dimethylpolysiloxane (6 cst) serving as a diluent was added and mixed. Thereafter, the low-boiling-point matter was removed by distillation under reduced pressure while heating at 100°C to obtain a 6 cs diluted product (concentration: 50) of a liquid organomodified organopolysilxane having a xylitol group and a crosslinking portion, wherein the organopolysiloxane portion and the organic portion are linked via Si-C bonds of the crosslinking portion. This product was a light brown to tan colored viscous liquid (with good fluidity, in contrast to Practical Example 3) at 100°C. At 25°C, the product is a viscous liquid with thread-forming properties, and virtually no odor was perceived.

### [Practical Example 7] <Production of Silicone Compound No. 7>

First, 23.0 g of a methylhydrogenpolysiloxane expressed by the average composition formula: MD_{43.4}D^{H}_{7.4}M, 9.5 g of a methylhydrogenpolysiloxane expressed by the average composition formula: MD₇D^{H}₁₂T₁M, and 2.1 g of a methylhydrogenpolysiloxane expressed by the average composition formula: M^{H}₄Q were placed in a reaction vessel (the average composition formula of the mixture was M_{1.33}M^{H}_{1.33}D_{1.68}D^{H}_{6.47}T_{0.33}Q_{0.33}M). Next, 2.5 g of 3-methacryloxypropyl(tris(trimethylsiloxy)silylethyldimethylsiloxy)silane was added, and after 0.05 ml of a hexamethyldisiloxane solution of a platinum-1,3-divinyl-1,1,3,3-tetramethyldisiloxane complex (Pt concentration: 0.45 wt.%) was added while agitating under a nitrogen stream, the mixture was reacted for 1.5 hours at 70°C. The mixture was cooled to 55°C, and after 18.0 g of hexadecene (α-olefin purity: 91.7%) was added to the reaction mixture over the course of five additions, 0.05 ml of the platinum catalyst described above was added at an intermediate stage, and the mixture was reacted for 7 hours at 70 to 100°C. A small amount of the reaction liquid was sampled, and when the reaction rate was confirmed by an alkali decomposition gas generation method, it was found that 4.0 of the total of 7.8 Si-H groups of M^{H} and D^{H} in the average molecular composition of the methylhydrogenpolysiloxane were consumed and that the target level was reached. Next, 10.4 g of a xylitol monoallyl ether (purity: 91.2%), 30 g of isopropyl alcohol, and 0.03 g of natural vitamin E were added to the reaction mixture, and 0.10 ml of the platinum catalyst described above was then added. After reacting for 3 hours at 80 to 85°C, a small amount of the reaction liquid was sampled. As a result of calculating the reaction rate, it was found that 3.2 of the 3.8 remaining Si-H groups were consumed (2.7 groups were consumed by hydrosilylation with the allyl group of the allyl xylitol, and 0.5 groups were consumed by dehydrogenation with alcoholic hydroxyl groups).

Next, after the mixture was heated under reduced pressure and the isopropyl alcohol was removed by distillation, 60 g of toluene was added as a solvent for a crosslinking reaction. Here, 73.5 g of a methylvinylpolysiloxane expressed by the average composition formula: ^{Vi}MD₁₄₉M^{Vi} and 0.22 ml of the platinum catalyst described above were added and reacted for 4.5 hours at 95 to 100°C, and the reaction was completed. The Vi/H molar ratio at the time of crosslinking was 1.2. Next, 6.0 g of a 0.12% phosphoric acid aqueous solution, 3.0 g of purified water, and 30 g of isopropyl alcohol were added, and after the mixture was treated for 1 hour at 75°C, the mixture was neutralized by adding 0.16 g of 2.5% ammonia water. Thereafter, the low-boiling-point matter was removed by distillation under reduced pressure while heating at 100°C to obtain a liquid organomodified organopolysilxane having a xylitol group and a crosslinking portion, wherein the organopolysiloxane portion and the organic portion are linked via Si-C bonds of the crosslinking portion. This product was a viscous liquid at 100°C, it was confirmed to be a liquid from the fact that the product had a consistency of a mixture of a tan wax-like substance and an oily substance (demonstrating fluidity when tilted) at 25°C. In addition, virtually no odor was perceived.

### [Comparative Example 1] <Production of Silicone Compound No. RE-1>

First, 224.6 g of a methylhydrogenpolysiloxane expressed by the average composition formula: MD₇₀D^{H}₃M, 30.5 g of a xylitol monoallyl ether (purity: 92.3%), 105 g of isopropyl alcohol (IPA), and 0.24 g of a methanol solution of 2.3% sodium acetate were placed in a reaction vessel and heated to 45°C while agitating under a nitrogen stream. Here, 0.18 ml of a 10% isopropyl alcohol solution of chloroplatinic acid was added to the mixture, and after reacting for 7 hours at 50 to 80°C, a small amount of the reaction liquid was sampled. When the mixture was confirmed by an alkali decomposition gas generation method, the reaction was completed. Low-boiling-point matter was removed by distillation by heating the reaction liquid at 80°C under reduced pressure so as to obtain a xylitol-modified silicone represented by the average composition formula: MD₇₀D^{R*21}₃M as a light yellow colored, opaque homogeneous viscous liquid. Here, R^{*21} is as described above. An aldehyde-like odor was perceived from this product.

### [Comparative Example 2] <Production of Silicone Compound No. RE-2>

First, 195.0 g of an alkyl-monoamino-comodified polysiloxane expressed by the average composition formula: MD₆₇D^{*22}_{1.2}D^{*12}₁₀M, 5.0 g of glucolactone, and 100 g of ethanol were placed in a reaction vessel and reacted for 4 hours while agitating under a nitrogen stream and maintaining the temperature at 75°C. The ethanol was then removed by distillation under reduced pressure to obtain a sugar alcohol-modified silicone expressed by the average composition formula: MD₆₇D^{*23}_{1.2}D^{*12}₁₀M as a viscous, light yellow colored, transparent homogeneous liquid. A sweet ester-like odor was perceived from this product. Here, R^{*12}, R^{*22}, and R^{*23} are as described below.

R^{*12}=-C₁₈H₃₇

R^{*22}=-C₃H₆NH₂

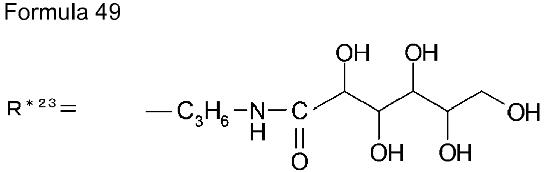

### Practical Examples 8 to 31 and Comparative Examples 3 to 10

Using the silicone compounds obtained in Practical Examples 1 to 6 and Comparative Examples 1 and 2, water-in-oil emulsion compositions having the formulations shown in Tables 2 to 5 were prepared as described below. These compositions were evaluated in terms of the aesthetic appearance of the composition, functionality (tactile sensation and sensation during use), viscosity stability, emulsified particle size stability, and odor over time according to the evaluation criteria below. In addition, the carbonyl values of the silicone compounds were measured in accordance with the method described below. The results are shown in Tables 2 to 5. In the table, "parts" indicates "parts by weight (mass)".

### Preparation method for water-in-oil emulsion composition

1. A silicone compound comprising an oil agent and a surfactant was placed in a 200 mL container.
2. The compound was heated and agitated, and the surfactant was uniformly dispersed or dissolved in the oil agent (oil phase A).
3. Table salt and ion exchanged water were placed in a separate container. The salt was dissolved by mixing using a spatula. Furthermore, 1,3-butylene glycol was mixed and dissolved therein (aqueous phase B).
4. The saw teeth of the homo-disper were immersed in the oil phase A and, after the container was secured, the aqueous phase B was poured into the oil phase A at a constant rate over a period of about 40 seconds while agitating at 1,000 rpm.
5. The speed of the homo-disper was increased to 3,000 rpm, and the aqueous phase B was poured into the oil phase A at a constant rate over a period of about 40 seconds while agitating.
6. Agitation was halted after agitating for two more minutes. The oily component adhering to the inner wall of the container was then scraped off using a spatula and mixed with the produced emulsion.
7. The mixture was agitated for three minutes at a speed of 3,000 rpm using the homo-disper.

### [Evaluation of aesthetic appearance]

After each water-in-oil emulsion composition was prepared, the beauty of the emulsion surface was evaluated in accordance with the following criteria after being left to stand for one day at room temperature.
⊚: A smooth, beautiful appearance on the whole with practically no observable grains due to air bubbles, holes or the like in the emulsion surface.
○: Fine grains are observed to a small degree due to air bubbles, holes or the like in the surface, but the grains are not particularly noticeable. The appearance is smooth and uniform.
Δ: Surface roughness of the emulsion is easily visible, and there are continuous grains due to air bubbles, holes, or the like.
×: Emulsion itself is not complete, and phase separation is observed. The surface is not uniform.

### Functionality evaluation (tactile sensation and sensation during use)

The sensation during use at the time of application, during application, and after application when using each water-in-oil emulsion composition as a cosmetic composition was evaluated according to the following criteria. Note that relative comparisons were carried out within groups that used a common oil agent. Specifically:
1. 0.20 g of the water-in-oil emulsion composition was placed on a finger and spread on the back of the hand.
2. At this time, the spreadability and smoothness at the time of application and during application and the skin compatibility, durability of moisturizing feel, and suppression of oiliness during and after application were evaluated in accordance with the following criteria.

### Spreadability and smoothness: Applying to during application

⊚: Thick, smooth tactile sensation with good spread and high-class feel of use.
○: A thick, smooth tactile sensation is maintained, but the spread is slightly inadequate.
Δ: Initial smoothness was experienced, but spreadability was lacking. Resistance (stickiness and adhesion when dry) with progressive spreading was experienced.
×: Heavy, poor spreadability, noticeable stickiness when initially applied, or good spreadability but noticeably oil tactile sensation. (Cases where the emulsifying itself was not possible are also indicated as "×")

### "Skin compatibility, durability of moisturizing feel, and suppression of oiliness: during and after application"

⊚: Skin compatibility is good, and a favorable moisturizing feel is also achieved. In addition, the oiliness is appropriately controlled, so an extremely natural sensation during use with no discomfort in terms of appearance can be obtained.
○: Skin compatibility is good, and there is a moisturizing feel, while a natural skin sensation is achieved. However, oily shine is somewhat strong, or a slight feeling of dryness is observed.
Δ: Although there is a moisturizing feel, the oil agent is not compatible with the skin when touched, and the skin surface is slippery. Oily shininess is also strong. Alternatively, there is a significant dry sensation, and the moisturizing feel is somewhat inadequate.
×: There is no moisturizing feel, the oil agent is not compatible with the skin when touched, the skin surface yields a slippery sensation, and oily shine is strong. (Cases where the emulsifying itself was not possible are also indicated as "×")

### Evaluation of viscosity stability

First, 28 g of each water-in-oil emulsion composition was measured into a 35 mL glass bottle. The bottles were capped and allowed to sit at rest in a 50°C constant temperature bath for two weeks. The viscosity stability of the emulsions before and after sitting was evaluated according to the following standards.
⊚: Viscosity variation= <±10% and appearance was uniform without change
○: ±10%<viscosity variation=<±20% and appearance was uniform
Δ: ±20%<viscosity variation=<±30%, or slight decrease in uniformity of the surface of the emulsion.
×: ±30%<viscosity variation, or separation of water drops, aqueous phase, oil phase, or the like.

### (Cases where the emulsifying itself was not possible are also indicated as "×")

Measurement of emulsified particle size and evaluation of stability One day after preparing the water-in-oil emulsion compositions, and after allowing the emulsion compositions (the capped 35 mL glass bottles containing 28 g of each water-in-oil emulsion composition, as described above) to sit at rest at 50°C for two weeks, the compositions were observed (1.000X) and photographed using an optical microscope, and the distribution range of the particle sizes was visually determined. Thereby, stability was evaluated by examining the initial emulsified particle size and the emulsified particle size over time.

Note that notes were made in the tables when particle coalescence was observed.
⊚: Change in emulsified particle size was small, and signs of coalescence were absent.
○: The emulsified particle size potentially increased slightly but definite coalescence was not observed. Alternatively, the emulsified particle size increased, but the overall particle size was small and the emulsion system was maintained.
Δ: It is thought that partial coalescence of the particles occurred. Definite increase in the maximum emulsified particle size.
×: Many particles were coalesced and emulsion was in the state of breaking down. (Cases where the emulsifying itself was not possible are also indicated as "×")

### [Evaluation of odor over time]

After allowing the emulsion compositions (the capped 35 mL glass bottles containing 28 g of each water-in-oil emulsion composition, as described above) to sit at rest at 50°C for two weeks, the compositions were removed and returned to room temperature. The bottles were opened on the next day, and the odor produced was evaluated in accordance with the following criteria. Note that relative comparisons were carried out within groups that used a common oil agent.
⊚: Level with practically no noticeable odor.
○: Slight odor (sweet solution-like specific odor) is perceived.
Δ: Moderate odor (sweet solution-like specific odor) is perceived.
×: Strong odor (sweet solution-like specific odor) is perceived.

### [Measurement of carbonyl value]

In accordance with the following procedure, the "carbonyl value COV", which is the cause of odor in the emulsion compositions, was quantitatively evaluated by measuring the carbonyl value of the silicone compounds (samples) obtained in Practical Examples 1 to 6 and Comparative Examples 1 and 2.

### [Preparation Example 1A]

Reagent-grade n-butanol (A) was weighed out into a 100 mL brown glass bottle, and 4.3 g of reagent-grade trichloroacetic acid was added. The bottle was covered and then shaken to homogenize it, thereby preparing an alcohol solution of trichloroacetic acid (acid concentration: 4.3% (wt/vol)). Hereinafter, this solution is called "trichloroacetic acid solution (1A)." Furthermore, this preparation operation was performed within 3 hours before measurement of absorbance.

### [Preparation Example 2A]

Reagent-grade n-butanol (A) was weighed out into a 100 mL brown glass bottle. Then, 50 mg of 2,4-dinitrophenylhydrazine (reagent-grade product containing equal amount of water; abbreviated hereinafter as "2,4-DNPH") was added. After the bottle was covered, the bottle was subjected to an ultrasonic bath for 10 minutes. Thereby, the 2,4-DNPH was completely dissolved by alcohol (A), and a 0.025% (wt/vol) 2,4-DNPH alcohol solution was prepared. Hereinafter, this solution is called "2,4-DNPH solution (2A)." Furthermore, this preparation operation was performed within 3 hours before measurement of absorbance.

### [Preparation Example 3B]

Reagent-grade ethanol (B) was weighed out into a 100 mL brown glass bottle. Then, 4.0 g of potassium hydroxide (pellet shaped reagent-grade product) was directly added. After the bottle was covered, while being occasionally shaken, the bottle was subjected to an ultrasonic bath for 20 minutes. Thereby, the potassium hydroxide was completely dissolved by the alcohol (B), and a 4.0% (wt/vol) potassium hydroxide alcohol solution was prepared. Hereinafter, this solution is called "potassium hydroxide solution (3B)." Furthermore, this preparation operation was performed within 3 hours before measurement of absorbance.

### [Measurement of carbonyl value]

2.00 g of sample and 23.00 g of reagent-grade n-butanol (A) were fed into a 50 mL screw tube with cap and mixed, to prepare 25.00 g of sample solution (Sa) having a sample concentration of 8 mass %.

1.250 g of the obtained sample solution (Sa) and 3.750 g of reagent-grade n-butanol (A) were fed into a 50 mL volumetric flask, and mixed, to prepare 5.000 g of sample solution (Sb) having a sample concentration of 2 mass %.

3 mL of the trichloroacetic acid solution (1A) obtained in Preparation Example 1A and 5 mL of the 2,4-DNPH solution (2A) obtained in Preparation Example 2Awere added to the volumetric flask containing 5.000 g of sample solution (Sb) using a volumetric pipette. Furthermore, 1.050 g of purified water was added and mixed to detect precursors of carbonyl compound such as acetal that might be present in the sample as carbonyl by hydrolyzing the precursors.

Next, the volumetric flask was stoppered and a Teflon (registered trademark) seal was wound around the stopper to keep the volumetric flask airtight. Then, the volumetric flask was placed in a 60°C constant temperature bath and heated for 30 minutes, thereby reacting the 2,4-DNPH and the carbonyls contained in the sample. Next, said volumetric flask was retrieved from the constant temperature bath, and was left standing for 30 minutes at room temperature.

Next, said volumetric flask was opened, and 10 mL of the potassium hydroxide solution (3B) obtained in Preparation Example 3B was added using a volumetric pipette, and then said volumetric flask was shaken to mix the mixture. 8 minutes after the addition of 10 mL of potassium hydroxide solution (3B), reagent-grade n-butanol (A) was added as the diluent solvent. This system was shaken to prepare a total of 50 mL of reaction solution (basic reaction solution). Next, 15 minutes after the addition of 10 mL of potassium hydroxide solution (3B), the reaction solution was placed in an absorption cell (length of liquid layer: 1 cm), and an absorptiometer was used to measure the absorbance at 430 nm (A₁).

On the other hand, 5.000 g of reagent-grade n-butanol (A) was used instead of the sample solution (Sb), for blank measurement. A solution obtained by performing the same operations as above (addition of trichloroacetic acid solution (1A), addition of 2,4-DNPH solution (2A), heating and cooling of the obtained mixed solution, addition of potassium hydroxide solution (3B), and addition of a diluent solvent composed of reagent-grade n-butanol (A)) was placed into an absorption cell (length of liquid layer = 1 cm), and the absorbance (A₂) at 430 nm was measured.

The carbonyl value (COV) was determined by substituting the absorbance (A₁) and the absorbance (A₂) determined as described above, into the formula CV = (A₁ - A₂)/0.1.
Table 2

Table 2: Formulations and evaluation results of the water-in-oil emulsion compositions

### (Practical Examples 8 to 13 and Comparative Examples 3 and 4)

| Name of raw material | Practical Examples | | | | | | Comparative Examples | |
|---|---|---|---|---|---|---|---|---|
| | 8 | 9 | 10 | 11 | 12 | 13 | 3 | 4 |
| Silicone compound No. 1 | 2 | - | - | - | - | - | - | - |
| Silicone compound No. 2 | - | 2 | - | - | - | - | - | - |
| Silicone compound No. 3 (50%, 6 cs dilution) | - | - | 4 | - | - | - | - | - |
| Silicone compound No. 4 | - | - | - | 2 | - | - | - | - |
| Silicone compound No. 5 | - | - | - | - | 2 | - | - | - |
| Silicone compound No. 6 (50%, 6 cs dilution) | - | - | - | - | - | 4 | - | - |
| Silicone compound No. RE-1 | - | - | - | - | - | - | 2 | - |
| Silicone compound No. RE-2 | - | - | - | - | - | - | - | 2 |
| Dimethylpolysiloxane (6 cSt) | 23 | 23 | 21 | 23 | 23 | 21 | 23 | 23 |
| Sodium chloride | 0.5 | 0.5 | 0.5 | 0.5 | 0.5 | 0.5 | 0.5 | 0.5 |
| Purified water | 68.5 | 68.5 | 68.5 | 68.5 | 68.5 | 68.5 | 68.5 | 68.5 |
| 1,3-butylene glycol | 6 | 6 | 6 | 6 | 6 | 6 | 6 | 6 |
| Aesthetic appearance | ○ | ⊚ | ○ | ⊚ | ⊚ | ⊚ | ○ | ○ |
| Sensation during use (at the time of application and during application) | ⊚ | ⊚ | ⊚ | ⊚ | ○ | ⊚ | Δ | × |
| Sensation during use (during and after application) | ⊚ | ⊚ | ⊚ | ○ | ○ | ⊚ | Δ | ○ |
| Viscosity stability of emulsion | ⊚ | ⊚ | ⊚ | ⊚ | ⊚ | ⊚ | × | Δ |
| Initial particle size distribution (µm) | 2-7.7 | 2-5.0 | 2-8.1 | 2-7.0 | 2-5.3 | 2-6.3 | 1.5-5.2 | 2-7.0 |
| Particle size distribution over time (µm) | 2-7.3 | 2-4.7 | 2-8.0 | 2-7.3 | 2-5.7 | 2-6.5 | 1.5-5.5 | 3-10 |
| Stability of emulsified particles | ⊚ | ⊚ | ⊚ | ⊚ | ⊚ | ⊚ | ⊚ | Δ |
| Odor over time | ⊚ | ⊚ | ⊚ | ⊚ | ○ | ⊚ | × | ○ |
| Carbonyl value | 1.2 | 0.6 | 0.3 | 1.0 | 1.5 | 0.5 | 9.8 | 2.5 |

[Table 3]

**Table 3: Formulations and evaluation results of the water-in-oil emulsion compositions (Practical Examples 14 to 19 and Comparative Examples 5 and 6)**

| Name of raw material | Practical Examples | | | | | | Comparative Examples | |
|---|---|---|---|---|---|---|---|---|
| | 14 | 15 | 16 | 17 | 18 | 19 | 5 | 6 |
| Silicone compound No. 1 | 2 | - | - | - | - | - | - | - |
| Silicone compound No. 2 | - | 2 | - | - | - | - | - | - |
| Silicone compound No. 3 (50%, 6 cs dilution) | - | - | 4 | - | - | - | - | - |
| Silicone compound No. 4 | - | - | - | 2 | - | - | - | - |
| Silicone compound No. 5 | - | - | - | - | 2 | - | - | - |
| Silicone compound No. 6 (50%, 6 cs dilution) | - | - | - | - | - | 4 | - | - |
| Silicone compound No. RE-1 | - | - | - | - | - | - | 2 | - |
| Silicone compound No. RE-2 | - | - | - | - | - | - | - | 2 |
| Mineral oil 50SUS (37.8°C) | 23 | 23 | 21 | 23 | 23 | 21 | 23 | 23 |
| Sodium chloride | 0.5 | 0.5 | 0.5 | 0.5 | 0.5 | 0.5 | 0.5 | 0.5 |
| Purified water | 68.5 | 68.5 | 68.5 | 68.5 | 68.5 | 68.5 | 68.5 | 68.5 |
| 1,3-butylene glycol | 6 | 6 | 6 | 6 | 6 | 6 | 6 | 6 |
| Aesthetic appearance | ○ | ⊚ | ○ | ⊚ | ⊚ | ⊚ | × | ○ |
| Sensation during use (at the time of application and during application) | ⊚ | ⊚ | ⊚ | ⊚ | ○ | ⊚ | × | × |
| Sensation during use (during and after application) | ⊚ | ⊚ | ⊚ | ○ | ○ | ⊚ | × | Δ |
| Viscosity stability of emulsion | ⊚ | ⊚ | ⊚ | ⊚ | ⊚ | ⊚ | × | × |
| Initial particle size distribution (µm) | 2-5.3 | 1.5-4.0 | 2-7.3 | 2-5.5 | 1.5-3.5 | 2-6.5 | - | 2-20 Pass |
| Particle size distribution over time (µm) | 2-4.4 | 1.5-4.1 | 2-7.5 | 2-5.3 | 1.5-3.3 | 2-6.5 | - | Breakd own |
| Stability of emulsified particles | ⊚ | ⊚ | ⊚ | ⊚ | ⊚ | ⊚ | × | × |
| Odor of emulsion over time | ⊚ | ⊚ | ⊚ | ⊚ | ○ | ⊚ | × | ○ |
| Carbonyl value | 1.2 | 0.6 | 0.3 | 1.0 | 1.5 | 0.5 | 9.8 | 2.5 |

Table 4

**Table 4: Formulations and evaluation results of the water-in-oil emulsion compositions (Practical Examples 20 to 25 and Comparative Examples 7 and 8)**

| Name of raw material | Practical Examples | | | | | | Comparative Examples | |
|---|---|---|---|---|---|---|---|---|
| | 20 | 21 | 22 | 23 | 24 | 25 | 7 | 8 |
| Silicone compound No. 1 | 2 | - | - | - | - | - | - | - |
| Silicone compound No. 2 | - | 2 | - | - | - | - | - | - |
| Silicone compound No. 3 (50%, 6 cs dilution) | - | - | 4 | - | - | - | - | - |
| Silicone compound No. 4 | - | - | - | 2 | - | - | - | - |
| Silicone compound No. 5 | - | - | - | - | 2 | - | - | - |
| Silicone compound No. 6 (50%, 6 cs dilution) | - | - | - | - | - | 4 | - | - |
| Silicone compound No. RE-1 | - | - | - | - | - | - | 2 | - |
| Silicone compound No. RE-2 | - | - | - | - | - | - | - | 2 |
| Trioctanoin | 23 | 23 | 21 | 23 | 23 | 21 | 23 | 23 |
| Sodium chloride | 0.5 | 0.5 | 0.5 | 0.5 | 0.5 | 0.5 | 0.5 | 0.5 |
| Purified water | 68.5 | 68.5 | 68.5 | 68.5 | 68.5 | 68.5 | 68.5 | 68.5 |
| 1,3-butylene glycol | 6 | 6 | 6 | 6 | 6 | 6 | 6 | 6 |
| Aesthetic appearance | ○ | ○ | ○ | ○ | ○ | ○ | × | ○ |
| Sensation during use (at the time of application and during application) | ○ | ○ | ○ | ○ | ○ | ○ | × | × |
| Sensation during use (during and after application) | ○ | ○ | ○ | ○ | ○ | ○ | × | ○ |
| Viscosity stability of emulsion | ○ | ⊚ | ⊚ | ⊚ | ○ | ⊚ | × | Δ |
| Initial particle size distribution (µm) | 0.5-1.5 | 0.5-1.0 | 0.5-3.0 | 0.5-2.0 | 0.5-1.0 | 0.5-2.5 | - | 0.5-3.0 |
| Particle size distribution over time (µm) | 0.5-1.5 | 0.5-1.0 | 0.5-3.0 | 0.5-2.0 | 0.5-1.0 | 0.5-2.5 | - | 0.5-4.0 |
| Stability of emulsified particles | ⊚ | ⊚ | ⊚ | ⊚ | ⊚ | ⊚ | × | ○ |
| Odor of emulsion over time | ○ | ⊚ | ⊚ | ⊚ | ○ | ⊚ | - | Δ |
| Carbonyl value | 1.2 | 0.6 | 0.3 | 1.0 | 1.5 | 0.5 | 9.8 | 2.5 |

Table 5

**Table 5: Formulations and evaluation results of the water-in-oil emulsion compositions (Practical Examples 26 to 31 and Comparative Examples 9 and 10)**

| Name of raw material | Practical Examples | | | | | | Comparative Examples | |
|---|---|---|---|---|---|---|---|---|
| | 26 | 27 | 28 | 29 | 30 | 31 | 9 | 10 |
| Silicone compound No. 1 | 2 | - | - | - | - | - | - | - |
| Silicone compound No. 2 | - | 2 | - | - | - | - | - | - |
| Silicone compound No. 3 (50%, 6 cs dilution) | - | - | 4 | - | - | - | - | - |
| Silicone compound No. 4 | - | - | - | 2 | - | - | - | - |
| Silicone compound No. 5 | - | - | - | - | 2 | - | - | - |
| Silicone compound No. 6 (50%, 6 cs dilution) | - | - | - | - | - | 4 | - | - |
| Silicone compound No. RE-1 | - | - | - | - | - | - | 2 | - |
| Silicone compound No. RE-2 | - | - | - | - | - | - | - | 2 |
| Isododecane | 23 | 23 | 21 | 23 | 23 | 21 | 23 | 23 |
| Sodium chloride | 0.5 | 0.5 | 0.5 | 0.5 | 0.5 | 0.5 | 0.5 | 0.5 |
| Purified water | 68.5 | 68.5 | 68.5 | 68.5 | 68.5 | 68.5 | 68.5 | 68.5 |
| 1,3-butylene glycol | 6 | 6 | 6 | 6 | 6 | 6 | 6 | 6 |
| Aesthetic appearance | ⊚ | ⊚ | ⊚ | ⊚ | ⊚ | ⊚ | ○ | × |
| Sensation during use (at the time of application and during application) | ⊚ | ⊚ | ⊚ | ⊚ | ⊚ | ⊚ | Δ | × |
| Sensation during use (during and after application) | ○ | ○ | ⊚ | ○ | ○ | ⊚ | Δ | × |
| Viscosity stability of emulsion | ⊚ | ⊚ | ⊚ | ⊚ | ⊚ | ⊚ | × | × |
| Initial particle size distribution (µm) | 3-10 | 3-9 | 3-8 | 3-10 | 3-8 | 3-8 | 4-12 | 5-30 Pass |
| Particle size distribution over time (µm) | 3-10 | 3-9 | 3-8 | 3-10 | 3-8 | 3-8 | 4-15 Pass | Breakdown |
| Stability of emulsified particles | ⊚ | ⊚ | ⊚ | ⊚ | ⊚ | ⊚ | Δ | × |
| Odor of emulsion over time | ⊚ | ⊚ | ⊚ | ⊚ | ⊚ | ⊚ | × | - |
| Carbonyl value | 1.2 | 0.6 | 0.3 | 1.0 | 1.5 | 0.5 | 9.8 | 2.5 |

It was verified from the above results that when the organomodified organopolysiloxane of the present invention having a xylitol group and a crosslinking portion, wherein the organopolysiloxane portion and the organic portion are linked by the Si-C bonds of the crosslinking portion, is used as an emulsifier for a water-in-oil composition, it is possible to stably emulsify/disperse an aqueous sphase in not only silicone oils, but also various oil agents having various characteristics such as polar ester oils (trioctanoin), mineral oils with relatively high molecular weights, and indodecane which is non-polar and has a low molecular weight, which enables much more multifaceted use than the conventional sugar alcohol-modified silicones used in the comparative examples.

In addition, it became clear that even if the emulsified particle size is somewhat large at approximately 2 to 10 µm, the stability with respect to the temperature of the emulsified particles and the passage of time is excellent, and the viscosity stability of the emulsion is also excellent. Moreover, with regard to tactile sensation, it was shown that the effects of water are utilized to suppress oiliness and to provide an excellent sensation during use as indicated by a thick, smooth feeling when applied, a natural feeling with good skin compatibility, lasting moisturizing effects, and a lack of stickiness.

Further, the emulsion obtained here has the excellent feature that there is very little odor generation due to the passage of time or temperature, which verifies that the emulsion is extremely useful as a raw material for an external use preparation or a cosmetic composition.

### Practical Examples 32 and 31 and Comparative Examples 11 and 12

In accordance with the following procedure, mixtures of the silicone compounds obtained in Practical Examples 1 to 6 and Comparative Examples 1 and 2 and oil agents were prepared as prescribed by the compositions in Table 6. In addition, the viscosity adjusting effects of the oil agents were evaluated in accordance with the following evaluation criteria for the obtained compositions. The results are shown in Table 6. In the table, "parts" indicates "parts by weight (mass)".

### [Preparation method for mixture with oil agent]

1. A dimethylpolysiloxane (6 cst) serving as an oil agent and a silicone compound serving as a viscosity adjusting agent were placed in a container at a prescribed compounding ratio, heated at 60°C, mixed well, and uniformly dissolved or dispersed to form a mixture.

### [Viscosity measurement and evaluation criteria]

1. After the mixture was returned to room temperature, the viscosity was measured under conditions at 25°C.
2. It was calculated by how many fold the viscosity of the mixture increased with respect to dimethylpolysiloxane (6 cst) (factor indicating how much the viscosity is changed at an added amount of 1/10).

Table 6

**Table 6: Formulations and evaluation results of mixtures (compositions) with oil agents (Practical Examples 32 to 37 and Comparative Examples 11 and 12)**

| Name of raw material | Practical Examples | | | | | | Comparative Examples | |
|---|---|---|---|---|---|---|---|---|
| | 32 | 33 | 34 | 35 | 36 | 37 | 11 | 12 |
| Silicone compound No. 1 | 10 | - | - | - | - | - | - | - |
| Silicone compound No. 2 | - | 10 | - | - | - | - | - | - |
| Silicone compound No. 3 (50%, 6 cs dilution) | - | - | 20 | - | - | - | - | - |
| Silicone compound No. 4 | - | - | - | 10 | - | - | - | - |
| Silicone compound No. 5 | - | - | - | - | 10 | - | - | - |
| Silicone compound No. 6 (50%, 6 cs dilution) | - | - | - | - | - | 20 | - | - |
| Silicone compound No. RE-1 | - | - | - | - | - | - | 10 | - |
| Silicone compound No. RE-2 | - | - | - | - | - | - | - | 10 |
| Dimethylpolysiloxane (6 cSt) | 90 | 90 | 80 | 90 | 90 | 80 | 90 | 90 |
| Factor of change in viscosity | 2.4 | 1.7 | 694 | 1.8 | 1.8 | 23.2 | 2.9 | 1.8 |

### Practical Examples 38 to 43 and Comparative Examples 13 and 14

In accordance with the following procedure, mixtures of the silicone compounds obtained in Practical Examples 1 to 6 and Comparative Examples 1 and 2 and volatile oil agents were prepared as prescribed by the compositions in Table 7, and the viscosity was measured. In addition, the viscosity adjusting effects were evaluated. Next, the film effect was evaluated in accordance with the following evaluation criteria for a film obtained by coating a slide glass surface with the resulting mixture and volatilizing the oil agent. The results are shown in Table 7. In the table, "parts" indicates "parts by weight (mass)".

### [Preparation method for mixture with oil agent]

1. Indodecane serving as a volatile oil agent and a silicone compound serving as a film agent were placed in a container at a prescribed compounding ratio, heated at 60°C, mixed well, and uniformly dissolved or dispersed to form a mixture.

### [Viscosity measurement and evaluation criteria]

1. After the mixture was returned to room temperature, the viscosity was measured under conditions at 25°C.
2. It was calculated by how many fold the viscosity of the mixture increased with respect to indodecane (1.7 cst) (factor indicating how much the viscosity is changed at an added amount of 1/10).

### [Test method for film effect]

1. After a clean slide glass was immersed in the mixture described above, the slide glass was pulled out, suspended in a constant-temperature bath at 50°C in a vertical state, and left to dry for two hours. Three of these samples were prepared for each mixture.
2. Two drops of physiological saline were placed on one slide glass coated with a film agent as described in 1 above.
3. Two drops of squalane were placed on a different slide glass coated with a film agent as described in 1 above.
4. The difficulty to remove the film was evaluated by using the index finger to lightly rub the wet surfaces of the slide glasses prepared in 2 and 3 and the dry surfaces of the slide glasses coated with a film agent in 1.

### [Film effect evaluation criteria]

⊚ The viscosity of the film is high, and the film moves negligibly when rubbed with the finger.
○: Although part of the film moves when rubbed with the finger, the viscosity is maintained on the whole, and the film appears to be settled on the glass.
Δ: Intermediate state between ○ above and × below.
×: The peeling of the film is observed, or the film dissolved and demonstrates decrease in viscosity on the whole.

Table 7

**Table 7: Formulations and evaluation results of mixtures (compositions) with oil agents (Practical Examples 38 to 43 and Comparative Examples 13 and 14)**

| | Practical Examples | | | | | | Comparative Examples | |
|---|---|---|---|---|---|---|---|---|
| Name of raw material | 38 | 39 | 40 | 41 | 42 | 43 | 13 | 14 |
| Silicone compound No. 1 | 10 | - | - | - | - | - | - | - |
| Silicone compound No. 2 | - | 10 | - | - | - | - | - | - |
| Silicone compound No. 3 (50%, 6 cs dilution) | - | - | 20 | - | - | - | - | - |
| Silicone compound No. 4 | - | - | - | 10 | - | - | - | - |
| Silicone compound No. 5 | - | - | - | - | 10 | - | - | - |
| Silicone compound No. 6 (50%, 6 cs dilution) | - | - | - | - | - | 20 | - | - |
| Silicone compound No. RE-1 | - | - | - | - | - | - | 10 | - |
| Silicone compound No. RE-2 | - | - | - | - | - | - | - | 10 |
| Isododecane | 90 | 90 | 80 | 90 | 90 | 80 | 90 | 90 |
| Factor of change in viscosity | 2.1 | 1.5 | 941 | 1.4 | 1.9 | 22.1 | 2.0 | 1.8 |
| Film effect (after coating) | ○ | ○ | ⊚ | ○ | ○ | ⊚ | Δ | ○ |
| Film effect (after immersion in saline) | ○ | ○ | ⊚ | ⊚ | ⊚ | ⊚ | Δ | Δ |
| Film effect (after immersion in squalane) | Δ | Δ | ⊚ | Δ | Δ | ○ | Δ | × |

When obvious gelation or hardening is unnecessary but it is desirable to moderately increase the viscosity of the compounding system, or when it is desirable to control the viscosity of the compounding system to a prescribed range, a wide range of viscosity increasing effects from a slight increase to a substantial increase was demonstrated as a result of adding the organomodified organopolysiloxane of the present invention having a xylitol group and a crosslinking portion to an oil phase, wherein the organopolysiloxane portion and the organic portion are linked by the Si-C bonds of the crosslinking portion. That is, the organomodified organopolysiloxane of the present invention is a viscosity adjuster with excellent convenience when formulating and designing an external use preparation or a cosmetic composition. Further, taking into consideration the fact that one of the factors governing the form or viscosity of an emulsion is the viscosity of the oil phase portion in an emulsion system in which an outer phase portion such as a water-in-oil or polyol-in-oil portion is an oil, it is clear that the organomodified organopolysiloxane of the present invention functions as a useful viscosity adjuster in this system.

Further, it was found that the organomodified organopolysiloxane of the present invention having a xylitol group and a crosslinking portion, wherein the organopolysiloxane portion and the organic portion are linked by the Si-C bonds of the crosslinking portion, is not only able to form a film that has high viscosity and is resistant to movement, but is also able to repel water, and favorably maintain the film state. Further, it was also found that the silicone compound Nos. 3 and 6 of Practical Examples 3 and 6 exhibit excellent film maintaining effects, even in a state in which squalane is present. It was determined from the above results that the organomodified organopolysiloxane of the present invention is useful as a film agent or a protective film oil agent.

### Practical Examples 44 to 49 and Comparative Examples 15 and 16

In accordance with the following procedure, oil-based foundations containing the silicone compounds obtained in Practical Examples 1 to 6 and Comparative Examples 1 and 2 were prepared as prescribed by the compositions in Table 8. In addition, an artificial leather surface was coated with each obtained foundation using a Meyer bar, and the binder effect with respect to powders was evaluated in accordance with the following evaluation criteria for films obtained after the foundations dried. The results are shown in Table 8. In the table, "parts" indicates "parts by weight".

### [Oil-based foundation preparation method]

1. Components other than an inorganic powder were dissolved or uniformly dispersed while agitating at 70 to 80°C.
2. An inorganic powder (silicone-treated kaolin, silicone-treated titanium dioxide, and silicone-treated red iron oxide) was added to this, mixed well, and uniformly dispersed.
3. After being degassed, a container was filled with the mixture, and the mixture was returned to room temperature.

### [Test method for binder effect]

1. After an artificial leather surface was coated with an oil-based foundation using a Meyer bar, the foundation was left to stand overnight and dried in a constant-temperature bath at 40°C. Three of these samples were prepared for each foundation.
2. A filter paper soaked with water, a filter paper soaked with squalane, and a filter paper not soaked with anything were prepared.
3. The artificial leather surfaces coated with the foundation in 1 were pressed into the filter papers of 2 and moved forward and backward ten times.
4. The amount of transfer of the sample from the artificial leather to the filter paper after the completion of the forward and backward movements was determined visually based on the darkness of the color.

### [Binder effect evaluation criteria]

⊚: No transfer whatsoever.
○: Very little transfer.
Δ: Some transfer.
×: Severe transfer.

Table 8

**Table 8: Formulations and evaluation results of oil-based foundations (Practical Examples 44 to 49 and Comparative Examples 15 and 16)**

| Name of raw material | Practical Examples | | | | | | Comparative Examples | |
|---|---|---|---|---|---|---|---|---|
| | 44 | 45 | 46 | 47 | 48 | 49 | 15 | 16 |
| Silicone compound No. 1 | 8 | - | - | - | - | - | - | - |
| Silicone compound No. 2 | - | 8 | - | - | - | - | - | - |
| Silicone compound No. 3 (50%, 6 cs dilution) | - | - | 8 | - | - | - | - | - |
| Silicone compound No. 4 | - | - | - | 8 | - | - | - | - |
| Silicone compound No. 5 | - | - | - | - | 8 | - | - | - |
| Silicone compound No. 6 (50%, 6 cs dilution) | - | - | - | - | - | 8 | - | - |
| Silicone compound No. RE-1 | - | - | - | - | - | - | 8 | - |
| Silicone compound No. RE-2 | - | - | - | - | - | - | - | 8 |
| Microcrystalline wax | 4 | 4 | 4 | 4 | 4 | 4 | 4 | 4 |
| Mineral oil | 3 | 3 | 3 | 3 | 3 | 3 | 3 | 3 |
| Sorbitan sesquioleate | 1 | 1 | 1 | 1 | 1 | 1 | 1 | 1 |
| Decamethyl cyclopentasiloxane | 39 | 39 | 39 | 39 | 39 | 39 | 39 | 39 |
| Isopropyl myristate | 2 | 2 | 2 | 2 | 2 | 2 | 2 | 2 |
| Silicone-treated kaolin | 25 | 25 | 25 | 25 | 25 | 25 | 25 | 25 |
| Silicone-treated titanium dioxide | 15 | 15 | 15 | 15 | 15 | 15 | 15 | 15 |
| Silicone-treated red Iron oxide | 3 | 3 | 3 | 3 | 3 | 3 | 3 | 3 |
| Binder effect (uncoated) | ⊚ | ⊚ | ⊚ | ⊚ | ○ | ⊚ | Δ | ○ |
| Binder effect (water-coated) | ⊚ | ⊚ | ⊚ | ⊚ | ○ | ⊚ | ○ | Δ |
| Binder effect (squalane-coated) | ○ | ○ | ⊚ | ○ | ○ | ⊚ | Δ | × |

As described above, it was verified that when the organomodified organopolysiloxane of the present invention having a xylitol group and a crosslinking portion, wherein the organopolysiloxane portion and the organic portion are linked via Si-C bonds of the crosslinking portion, is used, the moisture resistance and sebum resistance of a cosmetic composition containing a powder is clearly improved in comparison to cases in which the conventional sugar alcohol-modified silicones used in the comparative examples are used, and there is little secondary adhesion. This result demonstrates that the organomodified organopolysiloxane of the present invention is an excellent binder.

Further, the liquid organopolysiloxane of the present invention (silicone compounds Nos. 1 to 7) can also be used in formulations 1 to 62 of the specific cosmetic compositions shown in the table below.

Table 9

**Table 9**

| | |
|---|---|
| Formulation Example 1: Emulsion foundation | Formulation Example 32: Rouge |
| Formulation Example 2: Liquid foundation | Formulation Example 33: Lip gloss |
| Formulation Example 3: Foundation | Formulation Example 34: Mascara |
| Formulation Example 4: Pressed powder cosmetic | Formulation Example 35: Mascara |
| Formulation Example 5: Powder foundation | Formulation Example 36: Mascara |
| Formulation Example 6: Pressed foundation | Formulation Example 37: Eye shadow |
| Formulation Example 7: Foundation | Formulation Example 38: Solid powder eye shadow |
| Formulation Example 8: Foundation | Formulation Example 39: Anti-perspirant aerosolized cosmetic composition |
| Formulation Example 9: Solid foundation | Formulation Example 40: Nonaqueous pressurized anti-perspirant product |
| Formulation Example 10: Oil-based foundation | Formulation Example 41: Anti-perspirant lotion composition |
| Formulation Example 11: Water-in-oil cream | Formulation Example 42: Anti-perspirant composition |
| Formulation Example 12: Water-in-oil emulsion composition | Formulation Example 43: Aerosol type anti-perspirant composition |
| Formulation Example 13: Water-in-oil emulsion rouge (liquid) | Formulation Example 44: W/O solid anti-perspirant stick composition |
| Formulation Example 14: Emulsion | Formulation Example 45: W/O emulsion type anti-perspirant cream composition |
| Formulation Example 15: Cream | Formulation Example 46: Hair conditioner |
| Formulation Example 16: Aftershave cream | Formulation Example 47: Hair conditioner |
| Formulation Example 17: Foundation | Formulation Example 48: Hair treatment rinse-type |
| Formulation Example 18: W/O emulsion-type skin external use preparation | Formulation Example 49: Hair treatment leave-on type |
| Formulation Example 19: Polyol/O-type nonaqueous emulsion skin external use preparation | Formulation Example 50: Shampoo |
| Formulation Example 20: Polyol/O-type nonaqueous emulsion skin external use preparation | Formulation Example 51: Shampoo |
| Formulation Example 21: O/W cream | Formulation Example 52: Hair cream (set type) |
| Formulation Example 22: Sunscreen emulsion | Formulation Example 53: Hair mist |
| Formulation Example 23: UV blocking cream | Formulation Example 54: Hair foam |
| Formulation Example 24: UV blocking water-in-oil emulsion | Formulation Example 55: Hair spray |
| Formulation Example 25: Sunscreen agent | Formulation Example 56: Hair wax |
| Formulation Example 26: Water-in-oil emulsion sunscreen | Formulation Example 57: Hair cream |
| Formulation Example 27: Water-in-oil emulsion-type sunscreen | Formulation Example 58: Hair lotion |
| Formulation Example 28: Sun tanning cream | Formulation Example 59: Hair oil |
| Formulation Example 29: Liquid rouge | Formulation Example 60: Oxidation type hair color |
| Formulation Example 30: Rouge | Formulation Example 61: Hair manicure |
| Formulation Example 31: Lipstick | Formulation Example 62: Perm |

The specific content of these formulation examples has been disclosed in detail and in entirety in the corresponding patent application (Japanese Patent Application No. 2011-121095) filed in Japan by the present patent applicant, and the content thereof is incorporated herein by reference.

## Claims

1. A liquid organopolysiloxane, having fluidity at a temperature of at least 100°C, having a silicon-bonded sugar alcohol-modified group, and having a crosslinked structure comprising a carbon-silicon bond in the crosslinking portion.

2. The liquid organopolysiloxane according to claim 1, wherein the sugar alcohol-modified group is expressed by the following general formula (4-1): 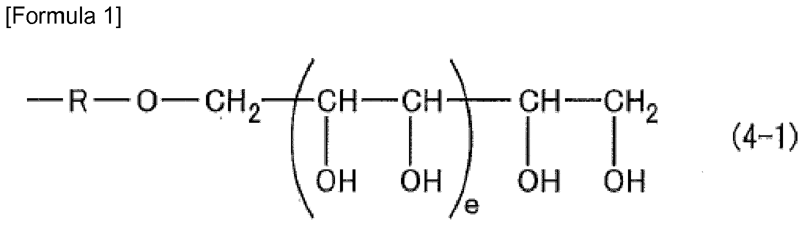 (wherein
R is a divalent organic group, and
e is 1 or 2) or the following general formula (4-2): 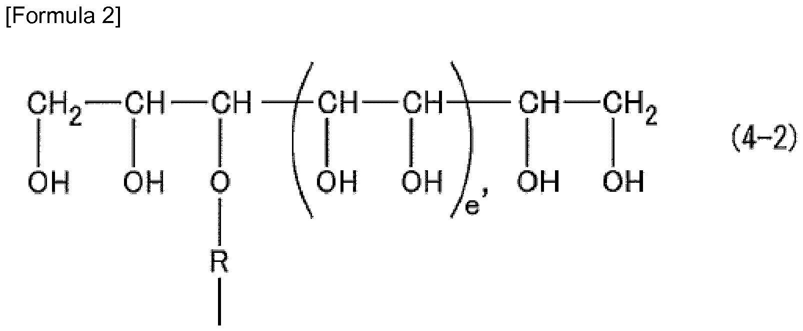
(wherein
R is as defined above, and
e' is 0 or 1).

3. The liquid organopolysiloxane according to claim 1 or 2, wherein in the general formula (4-1) or (4-2), the divalent organic group represented by R is a substituted or unsubstituted, straight-chain or branched divalent hydrocarbon group having from 3 to 5 carbon atoms.

4. The liquid organopolysiloxane according to any one of claims 1 to 3 obtained by reacting:
(A) an organohydrogenpolysiloxane;
(B) a sugar alcohol-group containing organic compound having an unsaturated bond; and
(C) at least one type of organic compound selected from the group consisting of (C1) an organic compound having an average number of unsaturated bonds in the molecule that is greater than 1 and (C2) an organic compound having at least one unsaturated bond and at least one epoxy group in the molecule.

5. The liquid organopolysiloxane according to claim 4, wherein an average value of a number of silicon-bonded hydrogen atoms per molecule of the component (A) reacting with the unsaturated bonds of the component (C) constituting the crosslinking portion is greater than 0.1 and less than 2.0.

6. The liquid organopolysiloxane according to claim 4 or 5, wherein the component (A) is expressed by the average composition formula (1):
[Formula 3] R¹ₐH_{b}SiO_{(4-a-b)/2} (1)
(wherein R¹ moieties are each independently monovalent organic groups, 1.0≤a≤3.0, and 0.001≤b≤1.5).

7. The liquid organopolysiloxane according to any one of claims 4 to 6, wherein the component (C) is at least one type of organic compound selected from the following formulas (C1-1) to (C1-5) and (C2-1) to (C2-2):
(C1-1) an α,ω-diene expressed by the general formula (2-1):
[Formula 4] CH₂=CH(CH₂)ₓCH=CH₂ (2-1)
(wherein 1≤**x**≤20);
(C1-2) an α,ω-diyne expressed by the general formula (2-2):
[Formula 5] CH≡C(CH₂)ₓC≡CH (2-2)
(wherein 1≤**x**≤20);
(C1-3) an α,ω-ene-yne expressed by the general formula (2-3):
[Formula 6] CH₂=CH(CH₂)ₓC≡CH (2-3)
(wherein 1≤**x**≤20);
(C1-4) a bisalkenyl polyether compound expressed by the general formula (2-4):
[Formula 7] CₘH₂ₘ₋₁O(CₙH₂ₙO)_{y}CₘH₂ₘ₋₁ (2-4)
(wherein 2≤m≤20, 2≤n≤4, y is the total value of the number of repetitions of the oxyethylene unit, the oxypropylene unit, and the oxybutylene unit, and 1≤y≤180);
(C1-5) an unsaturated group-containing silicone-compound expressed by the average composition formula (2-5):
[Formula 8] R²ₚR³_{q}SiO_{(4-p-q)/2} (2-5)
(wherein R² moieties may each be independent from one another but are monovalent organic groups that are different from R³;
R³ moieties are each independently monovalent unsaturated aliphatic hydrocarbon groups having from 2 to 30 carbon atoms, 1.0≤p≤2.5, and 0.001≤q≤1.5);
(C2-1) an unsaturated epoxy compound expressed by the general formula (2-6): 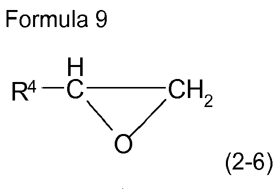 (wherein R⁴ is a substituted or unsubstituted, straight-chain or branched monovalent hydrocarbon group having one unsaturated bond and from 2 to 20 carbon atoms); and
(C2-2) an unsaturated group-containing cycloaliphatic epoxy compound expressed by the general formula (2-7): 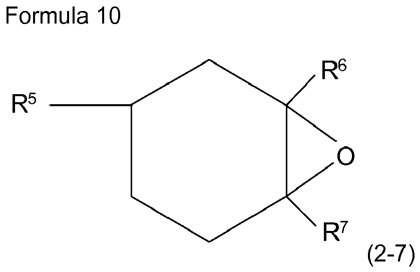
(wherein R⁵ is a substituted or unsubstituted, straight-chain or branched monovalent hydrocarbon group having one unsaturated bond and from 2 to 20 carbon atoms;
R⁶ is a hydrogen atom or a methyl group; and
R⁷ is a hydrogen atom or a methyl group).

8. The liquid organopolysiloxane according to any one of claims 1 to 7, wherein in the average composition formula (1), the monovalent organic group represented by R¹ is selected from the following (D1) to (D10);
(D1) a substituted or unsubstituted, straight-chain or branched monovalent hydrocarbon group having from 1 to 60 carbon atoms;
(D2) a polyoxyalkylene group expressed by -R⁸O(AO)_{z}R⁹ (wherein AO is an oxyalkylene group having from 2 to 4 carbon atoms; R⁸ is a substituted or unsubstituted, straight-chain or branched divalent hydrocarbon group having from 3 to 5 carbon atoms; R⁹ is a hydrogen atom, a substituted or unsubstituted, straight-chain or branched monovalent hydrocarbon group having from 1 to 24 carbon atoms, or a substituted or unsubstituted, straight-chain or branched acyl group having from 2 to 24 carbon atoms; and z=1 to 100);
(D3) a substituted or unsubstituted, straight-chain or branched alkoxy group having from 1 to 30 carbon atoms;
(D4) a hydroxyl group;
(D5) an ester group expressed by -R¹⁰-COOR¹¹ (wherein R¹⁰ is a substituted or unsubstituted, straight-chain or branched divalent hydrocarbon group having from 2 to 20 carbon atoms, and R¹¹ is a substituted or unsubstituted, straight-chain or branched monovalent hydrocarbon group having from 1 to 30 carbon atoms);
(D6) an ester group expressed by -R¹⁷-OCOR¹⁸ (wherein R¹⁷ is a substituted or unsubstituted, straight-chain or branched divalent hydrocarbon group having from 2 to 20 carbon atoms, and R¹⁸ is a substituted or unsubstituted, straight-chain or branched monovalent hydrocarbon group having from 1 to 30 carbon atoms);
(D7) L¹
here, L¹ is a silylalkyl group having a siloxane dendron structure and, when i=1, is expressed by the following general formula (3): 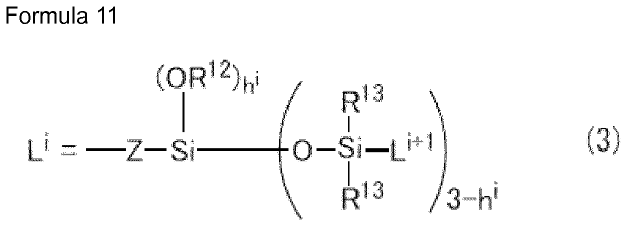 (wherein
R¹² is a substituted or unsubstituted, straight-chain or branched monovalent hydrocarbon group having from 1 to 30 carbon atoms;
R¹³ moieties each independently represents an alkyl group or phenyl group having from 1 to 6 carbon atoms;
Z represents a divalent organic group;
i represents a generation of the aforementioned silylalkyl group represented by Lⁱ and is an integer of 1 to k when k is the number of generations, which is the number of repetitions of the silylalkyl group; the number of generations k is an integer from 1 to 10; Lⁱ⁺¹ is the silylalkyl group when i is less than k, and R¹³ when i=k; and hⁱ is a number in a range from 0 to 3); (D8) an alkyl group substituted by a chain polysiloxane structure expressed by the following general formula (4): 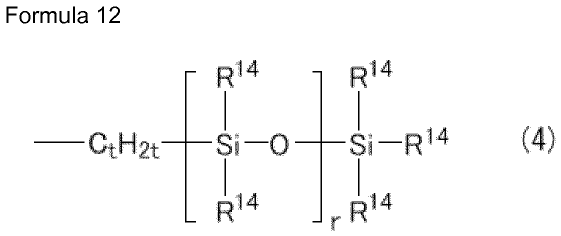 (wherein R¹⁴ moieties are each independently substituted or unsubstituted, straight-chain or branched monovalent hydrocarbon groups having from 1 to 30 carbon atoms, hydroxyl groups, or hydrogen atoms, and at least one of the R¹⁴ moieties is the monovalent hydrocarbon group; t is a number in a range from 2 to 10; and r is a number in a range from 1 to 100);
(D9) an epoxy group expressed by the following general formula (5): 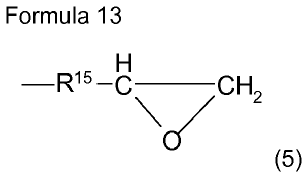 (wherein R¹⁵ is a substituted or unsubstituted, straight-chain or branched divalent hydrocarbon group having from 2 to 20 carbon atoms); and
(D10) a cycloaliphatic epoxy group expressed by the following general formula (6): 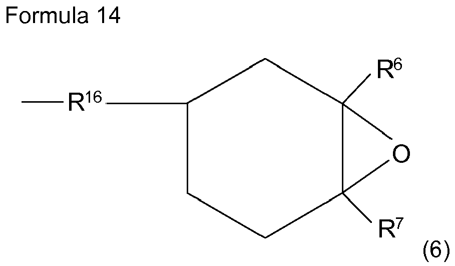
(wherein R¹⁶ is a substituted or unsubstituted, straight-chain or branched divalent hydrocarbon group having from 2 to 20 carbon atoms, and R⁶ and R⁷ are synonymous with those described above).

9. A composition, containing the liquid organopolysiloxane according to any one of claims 1 to 8 and at least one type of oil agent.

10. A liquid organopolysiloxane or a composition of the liquid organopolysiloxane, formed by treating the liquid organopolysiloxane according to any one of claims 1 to 8 or the composition according to claim 9 with at least one type of acidic substance and removing volatile components by heating or decompression.

11. A composition, containing the liquid organopolysiloxane according to any one of claims 1 to 8 and at least one type of acidic substance.

12. An emulsion composition, containing the liquid organopolysiloxane according to any one of claims 1 to 8 or the composition according to claims 9 to 11.

13. A raw material for an external use preparation or a cosmetic composition, containing the liquid organopolysiloxane according to any one of claims 1 to 8 or the composition according to any one of claims 9 to 12.

14. A raw material for the external use preparation or a cosmetic composition according to claim 13, the raw material being a tactile sensation improver, a film-forming agent, a binder, a viscosity adjuster, a surfactant, an emulsifier, or a powder dispersing agent.

15. An external use preparation or a cosmetic composition, containing the liquid organopolysiloxane according to any one of claims 1 to 8 or the composition according to any one of claims 9 to 12.

16. A production method for a liquid organopolysiloxane, wherein the liquid organopolysiloxane according to any one of claims 1 to 8 contains (A) an organohydrogenpolysiloxane, (B) a sugar alcohol-group containing organic compound having an unsaturated bond, and (C) at least one type of organic compound selected from the group consisting of (C1) an organic compound having an average number of unsaturated bonds in the molecule that is greater than 1 and (C2) an organic compound having at least one unsaturated bond and at least one epoxy group in the molecule as essential components, and other components are reacted with the component (A) successively in the presence of a hydrosilylation reaction catalyst.

17. The production method for a liquid organopolysiloxane according to claim 16, wherein a crosslinking reaction is performed by adding the component (C) after first reacting the component (A) and the component (B); and an optional component (Q) that is a compound having one unsaturated group in the molecule (excluding the compound of (C2)) may be reacted with the component (A) prior to the reaction between the component (A) and the component (B), further reacted after the reaction between the component (A) and the component (B), or further reacted after crosslinking by the component (C).

18. The production method for a liquid organopolysiloxane according to claim 16, wherein a reaction between the component (A) and the component (C) is first performed to derive a crosslinked portion, and the component (B) is then added and reacted thereafter; and an optional component (Q) that is a compound having one unsaturated group in the molecule (excluding the compound of (C2)) may be reacted with the component (A) prior to the reaction between the component (A) and the component (C), further reacted after the reaction between the component (A) and the component (C), or further reacted after the reaction with the component (B).

19. A production method for a liquid organopolysiloxane or a composition of the liquid organopolysiloxane, wherein after a liquid organopolysiloxane obtained by a reaction according to any one of claims 16 or 18 or a composition containing the same is treated with at least one type of an acidic substance, volatile components are removed by heating or decompression.

20. The production method for a liquid organopolysiloxane or a composition of the liquid organopolysiloxane according to claim 19, wherein after the liquid organopolysiloxane or the composition containing the same is treated with the acidic substance, the liquid organopolysiloxane or the composition containing the same is neutralized by adding at least one type of a basic substance.
